# EUROPEAN PATENT SPECIFICATION

(11) **EP 3 774 836 B1**
(45) Date of publication and mention of the grant of the patent: **04.10.2023**
(21) Application number: 19719698.3
(22) Date of filing: 05.04.2019
(51) Int. Cl.: C07J 71/00, A61K 31/58, A61P 29/00

(54) **HEXADECAHYDRO-1H-CYCLOPENTA[A]PHENANTHRENE DERIVATIVES USEFUL IN TREATING PAIN AND INFLAMMATION**
ZUR BEHANDLUNG VON SCHMERZEN UND ENTZÜNDUNGEN NÜTZLICHE HEXADECAHYDRO-1H-CYCLOPENTA[A]PHENANTHREN-DERIVATE
DÉRIVÉS D'HEXADÉCAHYDRO-1 H-CYCLOPENTA [A]PHÉNANTHRÈNE UTILES DANS LE TRAITEMENT DE LA DOULEUR ET DE L'INFLAMMATION

(30) Priority: 06.04.2018 US 201862654186 P
(43) Date of publication of application: 17.02.2021
(73) Proprietor: Taro Pharmaceuticals Inc., Brampton, ON L6T 1C1 (CA)
(72) Inventor: HARWIG, Curtis, Vancouver, British Columbia V5N 2V1 (CA); PETTIGREW, Jeremy D., Burnably, British Columbia V5B 3Y1 (CA); CROSS, Jennifer, Pitt Meadows, British Columbia V3Y 1B2 (CA); SEENISAMY, Jeyaprakashnarayanan, Vancouver, British Columbia V5T 4T5 (CA); KEREGADDE, Mahesh Narayan, Uttara Kannada District Karnataka 581402 (IN); KHER, Samir, Satish, Vancouver, British Columbia V5T 4T5 (CA); IYANAR, Karthikeyan, Vancouver, British Columbia V5T 4T5 (CA)
(74) Representative: Harrison IP Limited
(86) International application number: PCT/US2019/026009
(87) International publication number: WO 2019/195692

(56) References cited:
- WO-A1-01/83512
- WO-A1-2004/092100
- WO-A1-2014/158654
- WO-A2-98/02450

## Description

### FIELD OF THE INVENTION

The present invention is generally directed to hexadecahydro-1*H-*cyclopenta[a]phenanthrene derivatives which are useful in treating pain and inflammation, as well as to compositions and methods related to the same.

### BACKGROUND OF THE INVENTION

Tissue injury results in the initiation of a complex cascade of cellular events which result in redness, swelling, heat and pain at the site of insult. This inflammatory response is the normal mechanism by which the body contains and removes pathogens and repairs tissue damage. These classic and acute signs of inflammation are in large part attributable to the influx and accumulation of activated leukocytes and the subsequent release of mediators such as histamine, leukotrienes, substance P, prostaglandins, cytokines, reactive oxygen species and proteases. Activated immune cells and their proinflammatory products can also induce sensitization of peripheral nociceptors, contributing to the development of both acute and chronic inflammatory pain.

Normal inflammation is a tightly controlled, temporary, process, involving many different cell types, and is ultimately followed by a resolution phase featuring the expression of anti-inflammatory mediators and involving cell subsets that coordinate the tissue repair process. Inflammatory disease occurs when this normal cycle of activation/repair goes awry, resulting in a chronic state of immune cell activation and misdirection of the immune response towards host tissue. Inflammatory pain is also a protective response, designed to shield the injured tissue from further damage, but under conditions of uncontrolled inflammation, the multi-faceted interplay between the immune and nervous systems can drive the establishment of chronic pain and create a host of pathologies which are difficult to manage, creating a large personal and economic burden on society.

One of the key signaling pathways involved in the initiation and propagation of immune cell activation is the phosphoinositide-3-kinase (PI3K) pathway. In response to extracellular signals, phosphoinositide 3-kinase (PI3K) becomes activated and phosphorylates phosphatidylinositol-4,5-bisphosphate (PI-4,5-P2) within the plasma membrane to generate phosphatidylinositol-3,4,5-trisphosphate (PIP3). PIP3 then initiates a cascade of downstream signaling pathways by interacting with pleckstrin homology (PH) domain-containing proteins, such as protein kinase B (PKB, also known as Akt), that regulate cellular activation, function, proliferation and/or survival, depending on the cell type and stimulus (Deane et al., Annu Rev Immunol 22, 563-598, 2004). Cellular levels of PIP3 are normally tightly regulated by PI3K, the inositol 5-phosphatases SHIP1 (SH2 domain-containing inositol phosphatase), SHIP2, and by the inositol 3-phosphatase PTEN.

To date, a number of small molecule SHIP1 modulators have been disclosed, including sesquiterpene compounds such as pelorol. Other reported SHIP1 modulators include the compounds described in U.S. Patent Nos. 8,765,994, 7,601,874, 9,000,050, 9,540,353, and 9,765,085 U.S. Published Patent Application No. 2017/0253596.

Pain is another critical component of a body's defense system. In general, pain is a basic bodily sensation induced by a noxious stimulus, received by nerve endings (nociceptive receptors) and characterized by physical discomfort (such as pricking, throbbing, or aching), and typically leads to evasive action (*i.e*., removing oneself from the source of the stimulus). Pain is typically classified into two main categories: acute and chronic pain.

Acute or nociceptive pain is part of a rapid warning relay instructing the motor neurons of the central nervous system to minimize detected physical harm. It is mediated by nociceptors, which are free nerve endings that terminate just below the skin, in tendons, joints, and in body organs. They serve to detect cutaneous pain, somatic pain and visceral pain. Nociception can be associated with nerve damage caused by trauma, diseases such as diabetes, shingles, irritable bowel syndrome, late stage cancer or the toxic effects of chemotherapy.

Chronic pain is typically classified into two types: inflammatory nociceptive pain and neuropathic pain. Inflammatory nociceptive pain is associated with tissue damage and the resulting inflammatory process.

Neuropathic pain is produced by damage to and/or inflammation of the neurons in the peripheral and central nervous systems and involves sensitization of these systems.

One of the challenges for researchers is that chronic pain may involve a mix of both inflammatory and neuropathic components. In inflammatory nociceptive pain, inflammation may cause damage to the neurons and produce neuropathic pain.
Likewise, neuronal injury may cause an inflammatory reaction (neurogenic inflammation) that contributes to inflammatory pain.

WO-A-01/83512 discloses 17-alkylene-6,7-dihydroxy steroids condensed in positions 2 and 3 with a heteroaryl ring. These are indicated as having anti-inflammatory activity.

While significant strides have been made in the field of anti-inflammatory agents and analgesics, there remains a need for effective small molecule for the treatment of inflammation and/or pain. There is also a need for pharmaceutical compositions containing such compounds, as well as for methods relating to the use thereof to treat diseases, disorders or conditions that would benefit from such treatment. The present invention fulfills these needs, and provides other related advantages.

### SUMMARY OF THE INVENTION

The present invention is generally directed to compounds which are useful in treating inflammation and/or pain, pharmaceutical compositions comprising the compounds and methods of using the compounds and the pharmaceutical compositions of the invention for the treatment of inflammation and/or pain.

In the present specification, any reference to the use of the compounds of the invention in methods of treatment by therapy practiced on the human or animal body as being part of the present invention, is to be interpreted as a reference to the compounds of the invention for use in those same therapeutic methods.

Accordingly, in one aspect, this invention is directed to compounds of formula (I): 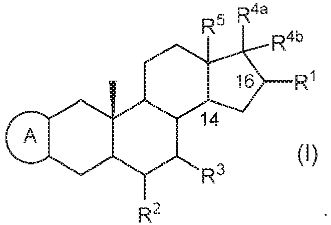 wherein: 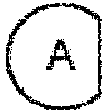 is an optionally substituted fused pyrazolyl; R¹ is hydrogen or -OR⁶; R² is -OR⁶ or -N(R⁷)₂;
R³ is -OR⁶ or -N(R⁷)₂;
R^{4a} is hydrogen, alkyl, alkenyl, -R⁸-OR⁶,
R^{4b} is hydrogen, alkyl, -OR⁶ or a direct bond to the carbon at C16;
R⁵ is alkyl or a direct bond to the carbon at C14;
each R⁶ is independently selected from hydrogen or alkyl;
each R⁷ is independently selected from hydrogen or alkyl; and
R⁸ is direct bond or a straight or branched alkylene chain; or tautomer thereof or mixtures thereof, or a pharmaceutically acceptable salt or solvate thereof.

In another aspect, this invention is directed to compositions comprising a pharmaceutically acceptable excipient, carrier and/or diluent and a compound of formula (I), or mixtures thereof; or a pharmaceutically acceptable salt or solvate thereof.

In another aspect, this invention is directed to methods for treating inflammation and/or pain in a mammal comprising administering an effective amount of a compound of formula (I), or mixtures thereof, or a pharmaceutically acceptable salt or solvate thereof, as set forth above, to the mammal in need thereof.

In another aspect, this invention is directed to methods for treating inflammation and/or pain in a mammal comprising administering a composition comprising an effective amount of a compound of formula (I), or mixtures thereof; or a pharmaceutically acceptable salt or solvate thereof, as set forth above, to the mammal in need thereof.

In another aspect, this invention is directed to methods of preparing compounds of formula (I), or mixtures thereof; or pharmaceutically acceptable salts or solvates thereof.

In another aspect, this invention is directed to the use of the compounds of the invention, as set forth above, or mixtures thereof, or a pharmaceutically acceptable salt or solvate thereof, or the use of a pharmaceutical composition comprising a pharmaceutically acceptable excipient and a compound of the invention, as set forth above, or mixtures thereof, or a pharmaceutically acceptable salt or solvate thereof, in the preparation of a medicament for the treatment of inflammation and/or pain.

These aspects and embodiments thereof are described in more detail below.

### DETAILED DESCRIPTION OF THE INVENTION

### Definitions

As used in the specification and appended claims, unless specified to the contrary, the following terms have the meaning indicated:
"Oxo" refers to =O.

"Cyano" refers to -CN.

"Nitro" refers to -NO₂.

"Hydroxy" or "hydroxyl" refers to -OH.

"Alkyl" refers to a straight or branched hydrocarbon chain radical consisting solely of carbon and hydrogen atoms, containing no unsaturation, having from one to twelve carbon atoms, preferably one to ten carbon atoms, more preferably one to eight carbon atoms, most preferably one to six carbon atoms and which is attached to the rest of the molecule by a single bond, e.g., methyl, ethyl, *n*-propyl, 1-methylethyl (*iso*-propyl), *n*-butyl, *n*-pentyl, 1,1-dimethylethyl (*t*-butyl), 3-methylhexyl, 2-methylhexyl, 6-methylheptan-2-yl, 5-ethyl-6-methylheptan-2-yl and the like. When specifically stated in the specification, an alkyl group may be optionally substituted by one of the following groups: alkyl, halo, haloalkyl, cyano, nitro, aryl, cycloalkyl, heterocyclyl, heteroaryl, oxo, trimethylsilanyl, -OR²⁰, -OC(O)-R²⁰, -N(R²⁰)₂, -C(O)R²⁰, -C(O)OR²⁰, -C(O)N(R²⁰)₂, -N(R²⁰)C(O)OR²², -N(R²⁰)C(O)R²², -N(R²⁰)S(O)ₚR²² (where p is 1 to 2), -S(O)ₚOR²² (where p is 1 to 2), -S(O)ₜR²² (where t is 0 to 2), and -S(O)ₚN(R²⁰)₂ (where p is 1 to 2) where each R²⁰ is independently hydrogen, alkyl, haloalkyl, cycloalkyl, cycloalkylalkyl, aryl, aralkyl, heterocyclyl, heterocyclylalkyl, heteroaryl or heteroarylalkyl; and each R²² is alkyl, haloalkyl, cycloalkyl, cycloalkylalkyl, aryl, aralkyl, heterocyclyl, heterocyclylalkyl, heteroaryl or heteroarylalkyl. For purposes of this invention, the alkyl group for R^{4a} and R^{4b} is defined as having one to ten carbons and the alkyl group for R⁵ is defined as having one to three carbons.

"Alkenyl" refers to a straight or branched hydrocarbon chain radical group consisting solely of carbon and hydrogen atoms, containing at least one double bond, having from two to twelve carbon atoms, preferably one to eight carbon atoms and which is attached to the rest of the molecule by a single bond, *e.g*., ethenyl, prop-1-enyl, but-1-enyl, pent-1-enyl, penta-1,4-dienyl, and the like. When specifically stated in the specification, an alkenyl group may be optionally substituted by one of the following groups: alkyl, halo, haloalkyl, cyano, nitro, aryl, cycloalkyl, heterocyclyl, heteroaryl, oxo, trimethylsilanyl, -OR²⁰, -OC(O)-R²⁰, -N(R²⁰)₂, -C(O)R²⁰, -C(O)OR²⁰, -C(O)N(R²⁰)₂, -N(R²⁰)C(O)OR²², -N(R²)C(O)R²², -N(R²⁰)S(O)ₚR²² (where p is 1 to 2), -S(O)ₚOR²² (where p is 1 to 2), -S(O)ₜR²² (where t is 0 to 2), and -S(O)ₚN(R²⁰)₂ (where p is 1 to 2) where each R²⁰ is independently hydrogen, alkyl, haloalkyl, cycloalkyl, cycloalkylalkyl, aryl, aralkyl, heterocyclyl, heterocyclylalkyl, heteroaryl or heteroarylalkyl; and each R²² is alkyl, haloalkyl, cycloalkyl, cycloalkylalkyl, aryl, aralkyl, heterocyclyl, heterocyclylalkyl, heteroaryl or heteroarylalkyl.

"Alkylene" or "alkylene chain" refers to a straight or branched divalent hydrocarbon chain linking the rest of the molecule to a radical group, consisting solely of carbon and hydrogen, containing no unsaturation and having from one to twelve carbon atoms, e.g., -CH₂, -CH₂-CH₂-, -CH₂-CH₂-CH₂-, and the like. The alkylene chain is attached to the rest of the molecule through a single bond and to the radical group through a single bond. The radical group can be attached to any carbon in the alkylene chain. When specifically stated in the specification, an alkylene chain may be optionally substituted by one of the following groups: alkyl, alkenyl, halo, cyano, nitro, aryl, cycloalkyl, heterocyclyl, heteroaryl, oxo, trimethylsilanyl, -OR²⁰, -OC(O)-R²⁰, -N(R²)₂, -C(O)R²⁰, -C(O)OR²⁰, -C(O)N(R²⁰)₂, -N(R²⁰)C(O)OR²², -N(R²⁰)C(O)R²², -N(R²⁰)S(O)ₚR²² (where p is 1 to 2), -S(O)ₚOR²² (where p is 1 to 2), -S(O)ₜR²² (where t is 0 to 2), and -S(O)ₚN(R²⁰)₂ (where p is 1 to 2) where each R²⁰ is independently hydrogen, alkyl, haloalkyl, cycloalkyl, cycloalkylalkyl, aryl, aralkyl, heterocyclyl, heterocyclylalkyl, heteroaryl or heteroarylalkyl; and each R²² is alkyl, haloalkyl, cycloalkyl, cycloalkylalkyl, aryl, aralkyl, heterocyclyl, heterocyclylalkyl, heteroaryl or heteroarylalkyl.

"Alkylidene" refers to a straight or branched hydrocarbon radical group consisting solely of carbon and hydrogen, containing at least one double bond, having from one to seven carbon atoms, and that is attached to the rest of the molecule through a double bond, *e.g*., methylene, ethylidene, propylidene, and the like. When specifically stated in the specification, an alkylidene radical may be optionally substituted by one of the following groups: alkyl, halo, haloalkyl, cyano, nitro, aryl, cycloalkyl, heterocyclyl, heteroaryl, oxo, trimethylsilanyl, -OR²⁰, -OC(O)-R²⁰, -N(R²⁰)₂, -C(O)R²⁰, -C(O)OR²⁰, -C(O)N(R²⁰)₂, -N(R²⁰)C(O)OR²², -N(R²⁰)C(O)R²², -N(R²⁰)S(O)ₚR²² (where p is 1 to 2), -S(O)ₚOR²² (where p is 1 to 2), -S(O)ₜR²² (where t is 0 to 2), and -S(O)ₚN(R²⁰)₂ (where p is 1 to 2) where each R²⁰ is independently hydrogen, alkyl, haloalkyl, cycloalkyl, cycloalkylalkyl, aryl, aralkyl, heterocyclyl, heterocyclylalkyl, heteroaryl or heteroarylalkyl; and each R²² is alkyl, haloalkyl, cycloalkyl, cycloalkylalkyl, aryl, aralkyl, heterocyclyl, heterocyclylalkyl, heteroaryl or heteroarylalkyl.

"Aryl" refers to a hydrocarbon ring system radical comprising hydrogen, 6 to 18 carbon atoms and at least one aromatic ring. For purposes of this invention, the aryl radical may be a monocyclic, bicyclic, tricyclic or tetracyclic ring system, which may include fused or bridged ring systems. Aryl radicals include, but are not limited to, aryl radicals derived from aceanthrylene, acenaphthylene, acephenanthrylene, anthracene, azulene, benzene, chrysene, fluoranthene, fluorene, as-indacene, s-indacene, indane, indene, naphthalene, phenalene, phenanthrene, pleiadene, pyrene, and triphenylene. When specifically stated in the specification, an aryl group may be optionally substituted by one or more substituents independently selected from the group consisting of alkyl, alkenyl, halo, haloalkyl, cyano, nitro, aryl, aralkyl, cycloalkyl, cycloalkylalkyl, heterocyclyl, heterocyclylalkyl, heteroaryl, heteroarylalkyl, -R²¹-OR²⁰, -R²¹-OC(O)-R²⁰, -R²¹-N(R²)₂, -R²¹-C(O)R²⁰, -R²¹-C(O)OR²⁰, -R²¹-C(O)N(R²⁰)₂, -R²¹-N(R²⁰)C(O)OR²², -R²¹-N(R²⁰)C(O)R²², -R²¹-N(R²⁰)S(O)ₚR²² (where p is 1 to 2), -R²¹-N=C(OR²⁰)R²⁰, -R²¹-S(O)ₚOR²² (where p is 1 to 2), -R²¹-S(O)ₜR²² (where t is 0 to 2), and -R²¹-S(O)ₚN(R²⁰)₂ (where p is 1 to 2) where each R²⁰ is independently hydrogen, alkyl, haloalkyl, cycloalkyl, cycloalkylalkyl, aryl, aralkyl, heterocyclyl, heterocyclylalkyl, heteroaryl or heteroarylalkyl; each R²¹ is independently a direct bond or a straight or branched alkylene chain; and each R²² is alkyl, haloalkyl, cycloalkyl, cycloalkylalkyl, aryl, aralkyl, heterocyclyl, heterocyclylalkyl, heteroaryl or heteroarylalkyl.

"Aralkyl" refers to a radical of the formula -R_{b}-R_{c} where R_{b} is an alkylene chain as defined above and R_{c} is one or more aryl radicals as defined above, for example, benzyl, diphenylmethyl and the like. When specifically stated in the specification, the alkylene chain part of the aralkyl radical may be optionally substituted as described above for an optionally substituted alkylene chain. When specifically stated in the specification, the aryl part of the aralkyl radical may be optionally substituted as described above for an optionally substituted aryl group.

"Cycloalkyl" refers to a stable non-aromatic monocyclic or polycyclic hydrocarbon radical consisting solely of carbon and hydrogen atoms, which may include fused or bridged ring systems, having from three to fifteen carbon atoms, preferably having from three to ten carbon atoms, and which is saturated or unsaturated and attached to the rest of the molecule by a single bond. Monocyclic radicals include, for example, cyclopropyl, cyclobutyl, cyclopentyl, cyclohexyl, cycloheptly, and cyclooctyl. Polycyclic radicals include, for example, adamantyl, norbornyl, decalinyl, and the like. When specifically stated in the specification, a cycloalkyl group may be optionally substituted by one or more substituents independently selected from the group consisting of alkyl, halo, haloalkyl, cyano, nitro, oxo, aryl, aralkyl, cycloalkyl, cycloalkylalkyl, heterocyclyl, heterocyclylalkyl, heteroaryl, heteroarylalkyl, -R²¹-OR²⁰, -R²¹-OC(O)-R²⁰, -R²¹-N(R²⁰)₂, -R²¹-C(O)R²⁰, -R²¹-C(O)OR²⁰, -R²¹-C(O)N(R²⁰)₂, -R²¹-N(R²)C(O)OR²², -R²¹-N(R²⁰)C(O)R²², -R²¹-N(R²⁰)S(O)ₚR²² (where p is 1 to 2), -R²¹-N=C(OR²⁰)R²⁰, -R²¹-S(O)ₚOR²² (where p is 1 to 2), -R²¹-S(O)ₜR²² (where t is 0 to 2), and -R²¹-S(O)ₚN(R²⁰)₂ (where p is 1 to 2) where each R²⁰ is independently hydrogen, alkyl, haloalkyl, cycloalkyl, cycloalkylalkyl, aryl, aralkyl, heterocyclyl, heterocyclylalkyl, heteroaryl or heteroarylalkyl; each R²¹ is independently a direct bond or a straight or branched alkylene chain; and each R²² is alkyl, haloalkyl, cycloalkyl, cycloalkylalkyl, aryl, aralkyl, heterocyclyl, heterocyclylalkyl, heteroaryl or heteroarylalkyl.

"Cycloalkylalkyl" refers to a radical of the formula -R_{b}R_{g} where R_{b} is an alkylene chain as defined above and R_{g} is a cycloalkyl radical as defined above. When specifically stated in the specification, the alkylene chain and/or the cycloalkyl radical may be optionally substituted as defined above for optionally substituted alkylene chain and optionally substituted cycloalkyl.

"Halo" refers to bromo, chloro, fluoro or iodo.

"Haloalkyl" refers to an alkyl radical, as defined above, that is substituted by one or more halo radicals, as defined above, *e.g*., trifluoromethyl, difluoromethyl, trichloromethyl, 2,2,2-trifluoroethyl, 1-fluoromethyl-2-fluoroethyl, 3-bromo-2-fluoropropyl, 1-bromomethyl-2-bromoethyl, and the like. The alkyl part of the haloalkyl radical may be optionally substituted as defined above for an alkyl group.

"Heterocyclyl" refers to a stable 3- to 18-membered non-aromatic ring radical which consists of two to twelve carbon atoms and from one to six heteroatoms selected from the group consisting of nitrogen, oxygen and sulfur. Unless stated otherwise specifically in the specification, the heterocyclyl radical may be a monocyclic, bicyclic, tricyclic or tetracyclic ring system, which may include spiro, fused or bridged ring systems; and the nitrogen, carbon or sulfur atoms in the heterocyclyl radical may be optionally oxidized; the nitrogen atom may be optionally quaternized; and the heterocyclyl radical may be partially or fully saturated. Examples of such heterocyclyl radicals include, but are not limited to, dioxolanyl, dioxinyl, thienyl[1,3]dithianyl, decahydroisoquinolyl, imidazolinyl, imidazolidinyl, isothiazolidinyl, isoxazolidinyl, morpholinyl, octahydroindolyl, octahydroisoindolyl, 2-oxopiperazinyl, 2-oxopiperidinyl, 2-oxopyrrolidinyl, oxazolidinyl, piperidinyl, piperazinyl, 4-piperidonyl, pyrrolidinyl, pyrazolidinyl, quinuclidinyl, thiazolidinyl, 1,2,4-thiadiazol-5(4*H*)-ylidene, tetrahydrofuryl, trioxanyl, trithianyl, triazinanyl, tetrahydropyranyl, thiomorpholinyl, thiamorpholinyl, 1-oxo-thiomorpholinyl, and 1,1-dioxo-thiomorpholinyl. When specifically stated in the specification, a heterocyclyl group may be optionally substituted by one or more substituents selected from the group consisting of alkyl, alkenyl, halo, haloalkyl, cyano, oxo, thioxo, nitro, aryl, aralkyl, cycloalkyl, cycloalkylalkyl, optionally substituted heterocyclyl, optionally substituted heterocyclylalkyl, optionally substituted heteroaryl, optionally substituted heteroarylalkyl, -R²¹-OR²⁰, -R²¹-OC(O)-R²⁰, -R²¹-N(R²⁰)₂, -R²¹-C(O)R²⁰, -R²¹-C(O)OR²⁰, -R²¹-C(O)N(R²⁰)₂, -R²¹-N(R²⁰)C(O)OR²², -R²¹-N(R²⁰)C(O)R²², -R²¹-N(R²⁰)S(O)ₚR²² (where p is 1 to 2), -R²¹-N=C(OR²⁰)R²⁰, -R²¹-S(O)ₚOR²² (where p is 1 to 2), -R²¹-S(O)ₜR²² (where t is 0 to 2), and -R²¹-S(O)ₚN(R²⁰)₂ (where p is 1 to 2) where each R²⁰ is independently hydrogen, alkyl, alkenyl, haloalkyl, cycloalkyl, cycloalkylalkyl, aryl, aralkyl, heterocyclyl, heterocyclylalkyl, heteroaryl or heteroarylalkyl; each R²¹ is independently a direct bond or a straight or branched alkylene chain; and each R²² is alkyl, alkenyl, haloalkyl, cycloalkyl, cycloalkylalkyl, aryl, aralkyl, heterocyclyl, heterocyclylalkyl, heteroaryl or heteroarylalkyl, and where the optional substituents on the heterocyclyl, heterocyclylalkyl, heteroaryl and heteroarylalkyl substitutents are selected from alkyl, halo or haloalkyl.

"Heterocyclylalkyl" refers to a radical of the formula -R_{b}Rₕ where R_{b} is an alkylene chain as defined above and Rₕ is a heterocyclyl radical as defined above, and if the heterocyclyl is a nitrogen-containing heterocyclyl, the heterocyclyl may be attached to the alkyl radical at the nitrogen atom. When specifically stated in the specification, the alkylene chain of the heterocyclylalkyl radical may be optionally substituted as defined above for an optionally substituted alkylene chain. When specifically stated in the specification, the heterocyclyl part of the heterocyclylalkyl radical may be optionally substituted as defined above for an optionally substituted heterocyclyl group.

"Heteroaryl" refers to a 5- to 14-membered ring system radical comprising hydrogen atoms, one to thirteen carbon atoms, one to six heteroatoms selected from the group consisting of nitrogen, oxygen and sulfur, and at least one aromatic ring. For purposes of this invention, the heteroaryl radical may be a monocyclic, bicyclic, tricyclic or tetracyclic ring system, which may include fused or bridged ring systems; and the nitrogen, carbon or sulfur atoms in the heteroaryl radical may be optionally oxidized; and the nitrogen atom may be optionally quatemized. Examples include, but are not limited to, azepinyl, acridinyl, benzimidazolyl, benzo[*d*]imidazolyl, benzimidazopyrimidinyl, benzo[4,5]imidazo[1,2-a]pyrimidinyl, benzthiazolyl, benzindolyl, benzodioxolyl, benzofuranyl, benzooxazolyl, benzothiazolyl, benzo[*d*]isoxazolyl, benzothiadiazolyl, benzo[b][1,4]dioxepinyl, 1,4-benzodioxanyl, benzonaphthofuranyl, benzoxazolyl, benzodioxolyl, benzodioxinyl, benzopyranyl, benzopyranonyl, benzofuranyl, benzofuranonyl, benzothienyl (benzothiophenyl), benzotriazolyl, benzo[4,6]imidazo[1,2-a]pyridinyl, benzoxazolinonyl, benzimidazolthionyl, carbazolyl, cinnolinyl, dibenzofuranyl, dibenzothiophenyl, furanyl, furanonyl, isothiazolyl, imidazo[1,2-*a*]pyridinyl, imidazo[1,2-*a*]pyrimidinyl, imidazo[1,2-a]pyrazinyl, imidazo[1,5-*a*]pyrazinyl, imidazolyl, indolyl, indazolyl, isoindolyl, indolinyl, isoindolinyl, isoquinolyl, indolizinyl, isoxazolyl, naphthyridinyl, oxadiazolyl, 2-oxoazepinyl, oxazolyl, oxiranyl, 1-oxidopyridinyl, 1-oxidopyrimidinyl, 1-oxidopyrazinyl, 1-oxidopyridazinyl, 1-phenyl-1*H*-pyrrolyl, phenazinyl, phenothiazinyl, phenoxazinyl, phthalazinyl, pteridinyl, pteridinonyl, purinyl, pyrrolyl, pyrazolyl, pyridinyl, pyridinonyl, pyrazinyl, pyrimidinyl, pryrimidinonyl, pyridazinyl, pyrido[2,3-*d*]pyrimidinonyl,pyrazolo[1,5-*a*]pyrimidinyl, quinazolinyl, quinazolinonyl, quinoxalinyl, quinoxalinonyl, quinolinyl, isoquinolinyl, tetrahydroquinolinyl, thiazolyl, thiadiazolyl, thieno[3,2-*d*]pyrimidin-4-onyl, thieno[2,3-*d*]pyrimidin-4-onyl, triazolyl, tetrazolyl, triazinyl, and thiophenyl (i.e. thienyl). When specifically stated in the specification, a heteroaryl group may be optionally substituted by one or more substituents selected from the group consisting of alkyl, alkenyl, halo, haloalkyl, cyano, oxo, thioxo, nitro, thioxo, aryl, aralkyl, cycloalkyl, cycloalkylalkyl, heterocyclyl, heterocyclylalkyl, heteroaryl, heteroarylalkyl, -R²¹-OR²⁰, -R²¹-OC(O)-R²⁰, -R²¹-N(R²⁰)₂, -R²¹-C(O)R²⁰, -R²¹-C(O)OR²⁰, -R²¹-C(O)N(R²⁰)₂, -R²¹-N(R²⁰)C(O)OR²², -R²¹-N(R²⁰)C(O)R²², -R²¹-N(R²⁰)S(O)ₚR²² (where p is 1 to 2), -R²¹-N=C(OR²⁰)R²⁰, -R²¹-S(O)ₚOR²² (where p is 1 to 2), -R²¹-S(O)ₜR²² (where t is 0 to 2), and -R²¹-S(O)ₚN(R²⁰)₂ (where p is 1 to 2) where each R²⁰ is independently hydrogen, alkyl, alkenyl, haloalkyl, cycloalkyl, cycloalkylalkyl, aryl, aralkyl, heterocyclyl, heterocyclylalkyl, heteroaryl or heteroarylalkyl; each R²¹ is independently a direct bond or a straight or branched alkylene chain; and each R²² is alkyl, alkenyl, haloalkyl, cycloalkyl, cycloalkylalkyl, aryl, aralkyl, heterocyclyl, heterocyclylalkyl, heteroaryl or heteroarylalkyl.

"*N*-heteroaryl" refers to a heteroaryl radical as defined above containing at least one nitrogen. The point of attachment of the *N*-heteroaryl to the rest of the molecule can be through a nitrogen atom or a carbon atom in the *N*-heteroaryl. When specifically stated in the specification, an *N*-heteroaryl radical may be optionally substituted as described above for an optionally substituted heteroaryl radical.

"Heteroarylalkyl" refers to a radical of the formula -R_{b}Rᵢ where R_{b} is an alkylene chain as defined above and Rᵢ is a heteroaryl radical as defined above. When specifically stated in the specification, the heteroaryl part of the heteroarylalkyl radical may be optionally substituted as defined above for an optionally substituted heteroaryl group. When specifically stated in the specification, the alkylene chain part of the heteroarylalkyl radical may be optionally substituted as defined above for an optionally substituted alkylene chain.

"Fused" refers to any ring structure described herein which is fused to an existing ring structure in the compounds of the invention. When the fused ring is a heterocyclyl ring or a heteroaryl ring, any carbon atom on the existing ring structure which becomes part of the fused heterocyclyl ring or the fused heteroaryl ring may be replaced with a nitrogen atom.

"Stable compound" and "stable structure" are meant to indicate a compound that is sufficiently robust to survive isolation to a useful degree of purity from a reaction mixture, and formulation into an efficacious therapeutic agent.

"Mammal" includes humans and both domestic animals such as laboratory animals and household pets, (*e.g*., cats, dogs, swine, cattle, sheep, goats, horses, rabbits), and non-domestic animals such as wildlife and the like.

"Optional" or "optionally" means that the subsequently described event of circumstances may or may not occur, and that the description includes instances where said event or circumstance occurs and instances in which it does not. For example, "optionally substituted aryl" means that the aryl radical may or may not be substituted and that the description includes both substituted aryl radicals and aryl radicals having no substitution ("unsubstituted"). When a functional group is described as "optionally substituted," and in turn, substitutents on the functional group are also "optionally substituted" and so on, for the purposes of this invention, such iterations are limited to five, preferably such iterations are limited to two.

"Pharmaceutically acceptable carrier, diluent or excipient" includes without limitation any adjuvant, carrier, excipient, glidant, sweetening agent, diluent, preservative, dye/colorant, flavor enhancer, surfactant, wetting agent, dispersing agent, suspending agent, stabilizer, isotonic agent, solvent, or emulsifier which has been approved by the United States Food and Drug Administration as being acceptable for use in humans or domestic animals.

"Pharmaceutically acceptable salt" includes both acid and base addition salts.

"Pharmaceutically acceptable acid addition salt" refers to those salts which retain the biological effectiveness and properties of the free bases, which are not biologically or otherwise undesirable, and which are formed with inorganic acids such as, but are not limited to, hydrochloric acid, hydrobromic acid, sulfuric acid, nitric acid, phosphoric acid and the like, and organic acids such as, but not limited to, acetic acid, 2,2-dichloroacetic acid, adipic acid, alginic acid, ascorbic acid, aspartic acid, benzenesulfonic acid, benzoic acid, 4-acetamidobenzoic acid, camphoric acid, camphor-10-sulfonic acid, capric acid, caproic acid, caprylic acid, carbonic acid, cinnamic acid, citric acid, cyclamic acid, dodecylsulfuric acid, ethane-1,2-disulfonic acid, ethanesulfonic acid, 2-hydroxyethanesulfonic acid, formic acid, fumaric acid, galactaric acid, gentisic acid, glucoheptonic acid, gluconic acid, glucuronic acid, glutamic acid, glutaric acid, 2-oxo-glutaric acid, glycerophosphoric acid, glycolic acid, hippuric acid, isobutyric acid, lactic acid, lactobionic acid, lauric acid, maleic acid, malic acid, malonic acid, mandelic acid, methanesulfonic acid, mucic acid, naphthalene-1,5-disulfonic acid, naphthalene-2-sulfonic acid, 1-hydroxy-2-naphthoic acid, nicotinic acid, oleic acid, orotic acid, oxalic acid, palmitic acid, pamoic acid, propionic acid, pyroglutamic acid, pyruvic acid, salicylic acid, 4-aminosalicylic acid, sebacic acid, stearic acid, succinic acid, tartaric acid, thiocyanic acid, p-toluenesulfonic acid, trifluoroacetic acid, undecylenic acid, and the like.

"Pharmaceutically acceptable base addition salt" refers to those salts which retain the biological effectiveness and properties of the free acids, which are not biologically or otherwise undesirable. These salts are prepared from addition of an inorganic base or an organic base to the free acid. Salts derived from inorganic bases include, but are not limited to, the sodium, potassium, lithium, ammonium, calcium, magnesium, iron, zinc, copper, manganese, aluminum salts and the like. Preferred inorganic salts are the ammonium, sodium, potassium, calcium, and magnesium salts. Salts derived from organic bases include, but are not limited to, salts of primary, secondary, and tertiary amines, substituted amines including naturally occurring substituted amines, cyclic amines and basic ion exchange resins, such as ammonia, isopropylamine, trimethylamine, diethylamine, triethylamine, tripropylamine, diethanolamine, ethanolamine, deanol, 2-dimethylaminoethanol, 2-diethylaminoethanol, dicyclohexylamine, lysine, arginine, histidine, caffeine, procaine, hydrabamine, choline, betaine, benethamine, benzathine, ethylenediamine, glucosamine, methylglucamine, theobromine, triethanolamine, tromethamine, purines, piperazine, piperidine, *N*-ethylpiperidine, polyamine resins and the like. Particularly preferred organic bases are isopropylamine, diethylamine, ethanolamine, trimethylamine, dicyclohexylamine, choline and caffeine.

Often crystallizations produce a solvate of the compound of the invention. As used herein, the term "solvate" refers to an aggregate that comprises one or more molecules of a compound of the invention with one or more molecules of solvent. The solvent may be water, in which case the solvate may be a hydrate. Alternatively, the solvent may be an organic solvent. Thus, the compounds of the present invention may exist as a hydrate, including a monohydrate, dihydrate, hemihydrate, sesquihydrate, trihydrate, tetrahydrate and the like, as well as the corresponding solvated forms. The compound of the invention may be true solvates, while in other cases, the compound of the invention may merely retain adventitious water or be a mixture of water plus some adventitious solvent. Furthermore, some of the crystalline forms of the compounds of the invention may exist as polymorphs, which are included in the present invention.

A "pharmaceutical composition" refers to a formulation of a compound of the invention and a medium generally accepted in the art for the delivery of the biologically active compound to mammals, *e.g*., humans. Such a medium includes all pharmaceutically acceptable carriers, diluents or excipients therefor.

"Therapeutically effective amount" refers to that amount of a compound of the invention which, when administered to a mammal, preferably a human, is sufficient to effect treatment, as defined below, of inflammation and/or pain in the mammal, preferably a human. The amount of a compound of the invention which constitutes a "therapeutically effective amount" will vary depending on the compound, the inflammation and/or pain and its severity, the manner of administration, and the age of the mammal to be treated, but can be determined routinely by one of ordinary skill in the art having regard to his own knowledge and to this disclosure.

"Treating" or "treatment" as used herein covers the treatment of inflammation and/or pain in a mammal, preferably a human, having the inflammation and/or pain, and includes:
(a) preventing the inflammation and/or pain from occurring in a mammal, in particular, when such mammal is predisposed to the condition but has not yet been diagnosed as having it;
(b) inhibiting the inflammation and/or pain, *i.e.*, arresting its development;
(c) relieving (or ameliorating) the inflammation and/or pain, *i.e.,* causing regression of the inflammation and/or pain; or
(d) relieving (or ameliorating) the symptoms resulting from the inflammation and/or pain, *e.g*., relieving inflammation and/or pain without addressing the underlying disease or condition.

As used herein, the terms "disease" and "condition" may be used interchangeably or may be different in that the particular malady or condition may not have a known causative agent (so that etiology has not yet been worked out) and it is therefore not yet recognized as a disease but only as an undesirable condition or syndrome, wherein a more or less specific set of symptoms have been identified by clinicians.

The compounds of the invention, or their pharmaceutically acceptable salts or solvates thereof, may contain one or more asymmetric centres and may thus give rise to enantiomers, diastereomers, and other stereoisomeric forms that may be defined, in terms of absolute stereochemistry, as (*R*)- or (*S*)- or, as (D)- or (L)- for amino acids. Compounds of the invention may also possess axial chirality which may result in atropisomers. Optically active (+) and (-), (*R*)- and (*S*)-, or (D)- and (L)- isomers may be prepared using chiral synthons or chiral reagents, or resolved using conventional techniques, for example, chromatography and fractional crystallisation. Conventional techniques for the preparation/isolation of individual enantiomers include chiral synthesis from a suitable optically pure precursor or resolution of the racemate (or the racemate of a salt or derivative) using, for example, chiral high-pressure liquid chromatography (HPLC). When the compounds described herein contain olefinic double bonds or other centres of geometric asymmetry, and unless specified otherwise, it is intended that the compounds include both E and Z geometric isomers.

A "stereoisomer" refers to a compound made up of the same atoms bonded by the same bonds but having different three-dimensional structures, which are not interchangeable. See, for example, Smith, M.B. and J. March, March's Advanced Organic Chemistry: Reactions, Mechanisms, and Structure, 6th edition (Wiley, 2007), for a detailed description of the structure and properties of enantiomers and stereoisomers.

A "tautomer" refers to a proton shift from one atom of a molecule to another atom of the same molecule.

Certain carbons are identified by numerals in the formulae of the compounds of the invention. For purposes herein, the carbon at numeral 14 in formula (I) is indicated herein as C14 and the carbon at numeral 16 is indicated herein as C16, and so forth. These numerals may or may not be the same as the locants in the compound names given herein.

When a substituent is indicated as being substituted, such as -R⁸-OR⁶, it is understood that the substituent may be substituted by the indicated substituent at any carbon in the substituent. Thus, for example, when the R⁸ in the -R⁸-OR⁶ substituent is an alkylene chain, the -OR⁶ group in the -R⁸-OR⁶ group can be on any carbon in the R⁸ alkylene chain.

The chemical naming protocol and structure diagrams used herein are a modified form of the I.U.P.A.C. nomenclature system, using ChemDraw 17.0 software program, wherein the compounds of the invention are named herein as derivatives of a central core structure. For complex chemical names employed herein, a substituent group is named before the group to which it attaches. For example, cyclopropylethyl comprises an ethyl backbone with cyclopropyl substituent. In chemical structure diagrams, all bonds are identified, except for some carbon atoms, which are assumed to be bonded to sufficient hydrogen atoms to complete the valency.

Thus, for example, a compound of formula (I) wherein 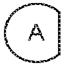 is a fused pyrazolyl, R¹ is hydrogen, R² is hydroxy, R³ is hydroxy, R^{4a} is (2S,5R)-5-ethyl-3-hydroxy-6-methylheptan-2-yl, R^{4b} is hydrogen and R⁵ is methyl, i.e., a compound of the following structure: 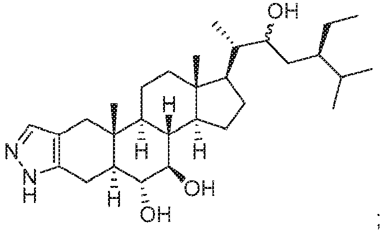 is named herein as (1*R*,3a*S*,3b*S*,4*R*,5*R*,5a*S*,10a*R*,10b*S*,12a*R*)-1-((2*S*,5*R*)-5-ethyl-3-hydroxy-6-methylheptan-2-yl)-10a,12a-dimethyl-1,2,3,3a,3b,4,5,5a,6,7,10,10a,10b,11,12,12a-hexadecahydrocyclopenta[5,6]naphtho[1,2-*f*]indazole-4,5-diol.

### Embodiments of the invention

Of the various aspects of the invention set forth above in the Summary of the Invention, certain embodiments are preferred.

Of the compounds of formula (I), or mixtures thereof, or a pharmaceutically acceptable salt or solvate thereof, as described above in the Summary of the Invention, a first embodiment are compounds of formula (I) wherein: 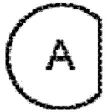 is an optionally substituted fused pyrazolyl;
R¹ is hydrogen or -OR⁶;
R² is -OR⁶;
R³ is -OR⁶;
R^{4a} is hydrogen, alkyl, alkenyl, -R⁸-OR⁶,
R^{4b} is hydrogen, alkyl, -OR⁶ or a direct bond to the carbon at C16;
R⁵ is alkyl or a direct bond to the carbon at C14;
each R⁶ is independently selected from hydrogen or alkyl;
each R⁷ is independently selected from hydrogen or alkyl; and
R⁸ is direct bond or a straight or branched alkylene chain.

One embodiment of this embodiment are compounds of formula (I) wherein: 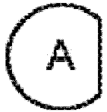 is a fused pyrazolyl optionally substituted by one or more substitutents selected from alkyl, haloalkyl, -C(O)OR⁷, -N(R⁷)₂, -C(O)N(R⁷)₂ or optionally substituted aryl;
R¹ is hydrogen or -OR⁶;
R² is -OR⁶;
R³ is -OR⁶;
R^{4a} is hydrogen, alkyl, alkenyl or -R⁸-OR⁶;
R^{4b} is hydrogen, alkyl, -OR⁶ or a direct bond to the carbon at C16;
R⁵ is alkyl or a direct bond to the carbon at C14;
each R⁶ is independently selected from hydrogen or alkyl;
each R⁷ is independently selected from hydrogen or alkyl; and
R⁸ is direct bond or a straight or branched alkylene chain.

Of this embodiment, an embodiment are compounds of formula (I) wherein: 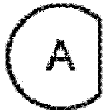 is pyrazolyl optionally substituted by one or more substitutents selected from alkyl, haloalkyl, -C(O)OR⁷, -N(R⁷)₂, -C(O)N(R⁷)₂ or optionally substituted aryl;
R¹ is hydrogen or -OR⁶;
R² is -OR⁶;
R³ is -OR⁶;
R^{4a} is hydrogen, alkyl, alkenyl or -R⁸-OR⁶;
R^{4b} is hydrogen, alkyl, -OR⁶ or a direct bond to the carbon at C16;
R⁵ is alkyl or a direct bond to the carbon at C14;
each R⁶ is independently selected from hydrogen or alkyl;
each R⁷ is independently selected from hydrogen or alkyl; and
R⁸ is direct bond or a straight or branched alkylene chain.

Of this embodiment, an embodiment are compounds of formula (I) selected from:
(1*S*,3a*S*,3b*R*,4*R*,5*R*,5a*S*,10a*R*,10b*S*,12a*S*)-1,10a,12a-trimethyl-1,2,3,3a,3b,4,5,5a,6,7,10,10a, 10b, 11,12,12a-hexadecahydrocyclopenta[5,6]naphtho[1,2-*f*]indazole-1,4,5-triol;
(3a*S*,3b*R*,4*R*,5*R*,5a*S*,10a*R*,10b*S*,12a*R*)-10a,12a-dimethyl-3,3a,3b,4,5,5a,6,7,10,10a,10b,11,12,12a-tetradecahydrocyclopenta[5,6]naphtho[1,2-*f*]indazole-4,5-diol;
(2*R*,4*R*,5*R*,5a*S*,10a*R*,10b*S*)-1,1,10a-trimethy)-1,2,3,3b,4,5,5a,6,7,10,10a,10b,11,12-tetradecahydrocyclopenta[5,6]naphtho[1,2-*f*]indazole-2,4,5-triol;
(1*R*,3a*S*,3b*S*,4*R*,5*R*,5a*S*,10a*R*,10b*S*,12a*R*)-1-((2*S*,5*R*)-5-ethy)-3-hydroxy-6-methylheptan-2-yl)-10a,12a-dimethyl-1,2,3,3a,3b,4,5,5a,6,7,10,10a,10b,11,12,12a-hexadecahydrocyclopenta[5,6]naphtho[1,2-*f*]indazole-4,5-diol;
(1*R*,3a*S*,3b*S*,4*R*,5*R*,5a*S*,10a*R*,12a*R*)-10a,12a-dimethy)-1-((*R*)-6-methy)heptan-2-y))-1,2,3,3a,3b,4,5,5a,6,7,10,10a,10b,11,12,12a-hexadecahydrocyclopenta[5,6]naphtho[1,2-*f*]indazole-4,5-diol; or
(1*R*,3a*S*,3b*S*,4*R*,5*R*,5a*S*,10a*R*,10b*S*,12a*S*)-10a,12a-dimethyl-1-(prop-1-en-2-yl)-1,2,3,3a,3b,4,5,5a,6,7,10,10a, 10b, 11,12,12a-hexadecahydrocyclopenta[5,6]naphtho[1,2-*f*]indazole-4,5-diol.

Another embodiment of the invention are methods for treating inflammation in the presence or absence of pain in a mammal in need thereof is where the inflammation is an autoimmune disease, disorder or condition, an inflammatory disease, disorder or condition, or a neoplastic or cell proliferative disease, disorder or condition.

Another embodiment of the methods for treating inflammation in the presence or absence of pain in a mammal in need thereof is where the inflammation is an autoimmune disease, disorder or condition selected from idiopathic pulmonary fibrosis, an inflammatory bowel disease, rheumatoid arthritis, osteoarthritis, Still's Disease, Sjögren's Syndrome, systemic lupus erythematosus, multiple sclerosis, psoriasis and systemic sclerosis.

Another embodiment of the methods for treating inflammation in the presence or absence of pain in a mammal in need thereof is where the inflammation is an inflammatory bowel disease selected from Crohn's Disease and ulcerative colitis.

Another embodiment of the methods for treating inflammation in the presence or absence of pain in a mammal in need thereof is where the inflammation is an inflammatory disease, disorder or condition selected from acute respiratory distress syndrome, allergic rhinitis, Alzheimer's Disease, asthma, an ocular inflammatory disease, atopic dermatitis, bladder pain syndrome/ interstitial cystitis, chronic prostatitis/chronic pelvic pain syndrome (CP/CPPS), chronic obstructive pulmonary disease (COPD) including emphysematous, bronchitic, and alpa 1 anti-trypsin deficiency related COPD; dermal contact hypersensitivy, eczema, eosiniphilic gastrointestinal disorder, fibromyalgia, gout, hepatic fibrosis, irritable bowl syndrome, ischemic reperfusion disease, kidney fibrosis, pancreatitis, Parkisons Disease, post operative inflammation, a seronegative spondyloarthropathy, and vasculitis.

Another embodiment of the methods for treating inflammation in the presence or absence of pain in a mammal in need thereof is where the inflammation is an ocular inflammatory disease selected from allergic conjunctivitis, dry eye, and uveitis.

Another embodiment of the methods for treating inflammation in the presence or absence of pain in a mammal in need thereof is where the inflammation is a seronegative spondyloarthropathy selected from anklyosing spondylitis, psoriatic arthritis, and Reiter's Syndrome.

Another embodiment of the methods for treating inflammation in the presence or absence of pain in a mammal in need thereof is where the inflammation is vasculitis selected from Wegener's Granulomatosis, polyarteritis nodosa, leucocytoclastic vasculitis, Churg-Strauss Syndrome, cryoglobulinemic vasculitis, and giant cell arteritis.

Another embodiment of the methods for treating inflammation in the presence or absence of pain in a mammal in need thereof is where the inflammation is a neoplastic or cell proliferative disease, disorder or condition selected from acute myelogenous leukemia, chronic myelogenous leukemia, acute lymphocytic leukemia, chronic lymphocytic leukemia, basophilic leukemia, cutaneous T-cell lymphoma, Sezary Syndrome, Hodgkin's Disease, Non-Hodgkin's Lymphoma, multiple myeloma, hypereosinophilic syndromes, mastocytosis and thrombocythemia.

Another embodiment of the methods for treating pain in a mammal in need thereof is where the pain is acute pain, chronic pain, inflammatory pain, nociceptive pain, inflammatory nociceptive pain, neuropathic pain and any combinations thereof.

Another embodiment of the methods for treating pain in a mammal in need thereof is where the pain is in the absence or presence of inflammation.

Another embodiment of the invention is a method of using the compounds of the invention as standards or controls in *in vitro* or *in vivo* assays in determining the efficacy of test compounds in treating inflammation and/or pain.

In another embodiment of the invention, the compounds of the invention are isotopically-labeled by having one or more atoms therein replaced by an atom having a different atomic mass or mass number. Such isotopically-labeled (*i.e*., radiolabelled) compounds of the invention are considered to be within the scope of this invention. Examples of isotopes that can be incorporated into the compounds of the invention include isotopes of hydrogen, carbon, nitrogen, oxygen, phosphorous, sulfur, fluorine, chlorine, and iodine, such as, but not limited to, ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ³⁶Cl, ¹²³I, and ¹²⁵I, respectively. These isotopically-labeled compounds would be useful to help determine or measure the effectiveness of the compounds, by characterizing, for example, the site or mode of action for the modulation, or binding affinity to pharmacologically important site of action for the modulation. Certain isotopically-labeled compounds of the invention, for example, those incorporating a radioactive isotope, are useful in drug and/or substrate tissue distribution studies. The radioactive isotopes tritium, i.e. ³H, and carbon-14, i.e., ¹⁴C, are particularly useful for this purpose in view of their ease of incorporation and ready means of detection.

Substitution with heavier isotopes such as deuterium, i.e. ²H, may afford certain therapeutic advantages resulting from greater metabolic stability, for example, increased *in vivo* half-life or reduced dosage requirements, and hence may be preferred in some circumstances.

Substitution with positron emitting isotopes, such as ¹¹C, ¹⁸F, ¹⁵O and ¹³N, can be useful in Positron Emission Topography (PET) studies for examining substrate receptor occupancy. Isotopically-labeled compounds of the invention can generally be prepared by conventional techniques known to those skilled in the art or by processes analogous to those described in the Synthetic Examples as set out below using an appropriate isotopically-labeled reagent in place of the non-labeled reagent previously employed.

In other embodiments, preferred stereochemistry of the compounds of formula (I) is shown below:

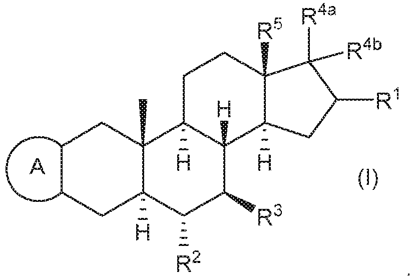

Specific embodiments of the compounds of the invention are described in more detail below in the Preparation of the Compounds of the Invention.

### Utility and Testing of the Compounds of the Invention

Compounds and compositions of the invention are useful in treating inflammation and/or pain. In particular, the compounds and compositions of the invention may be used to treat inflammation in the absence of pain, inflammation in the presence of pain or pain in the absence of inflammation, preferably pain in the absence of visible inflammation.

Without being bound to any theory, and for the sole purpose of this invention, the term "inflammation" is intended to include, but not limited to, an autoimmune disease, disorder or condition, an inflammatory disease, disorder or condition, or a neoplastic or cell proliferative disease, disorder or condition; idiopathic pulmonary fibrosis, an inflammatory bowel disease, rheumatoid arthritis, osteoarthritis, Still's Disease, Sjögren's Syndrome, systemic lupus erythematosus, multiple sclerosis, psoriasis and systemic sclerosis; Crohn's Disease or ulcerative colitis; acute respiratory distress syndrome, allergic rhinitis, Alzheimer's Disease, asthma, an ocular inflammatory disease, atopic dermatitis, bladder pain syndrome/ interstitial cystitis, chronic prostatitis/chronic pelvic pain syndrome (CP/CPPS), chronic obstructive pulmonary disease (COPD) including emphysematous, bronchitic, and alpa 1 anti-trypsin deficiency related COPD; dermal contact hypersensitivity, eczema, eosiniphilic gastrointestinal disorder, fibromyalgia, gout, hepatic fibrosis, irritable bowel syndrome, ischemic reperfusion disease, kidney fibrosis, pancreatitis, Parkinson's Disease, postoperative inflammation, a seronegative spondyloarthropathy or vasculitis; allergic conjunctivitis, dry eye or uveitis; ankylosing spondylitis, psoriatic arthritis or Reiter's Syndrome; vasculitis selected from Wegener's Granulomatosis, polyarteritis nodosa, leucocytoclastic vasculitis, Churg-Strauss Syndrome, cryoglobulinemic vasculitis or giant cell arteritis; a neoplastic or cell proliferative disease, disorder or condition selected from acute myelogenous leukemia, chronic myelogenous leukemia, acute lymphocytic leukemia, chronic lymphocytic leukemia, basophilic leukemia, cutaneous T-cell lymphoma, Sezary Syndrome, Hodgkin's Disease, Non-Hodgkin's Lymphoma, multiple myeloma, hypereosinophilic syndromes, mastocytosis and thrombocythemia.

For the sole purpose of this invention, the term "pain" is intended to include acute pain, chronic pain, inflammatory pain, nociceptive pain, inflammatory nociceptive pain, neuropathic pain and any combinations thereof. The types of pain intended to be treated by the compounds of the invention include, but are not limited to, pain associated with any of the above disclosed inflammatory diseases, disorders and conditions, burn pain, chronic bone pain, low back pain, neck pain, abdominal pain, somatic pain, visceral pain, myofascial pain, dental pain, cancer pain, chemotherapy pain, temporomandibular joint pain, trauma pain, surgical pain, post-surgical pain, labor pain, bladder pain, musculoskeletal pain, peripherally mediated pain, centrally mediated pain, headache pain, migraine pain, phantom limb pain, peripheral nerve injury pain, post-herpetic pain, non-cardiac chest pain, irritable bowel syndrome pain, fibromyalgia and combinations thereof.

The effectiveness of the compounds of the invention in treating inflammation and/or pain may be determined by any number of known *in vitro* and *in vivo* assays, including the assays set forth below in Biological Examples 1-15.

For example, the compounds of the invention may be tested in the following *in vitro* assays:
A. Rat or human dorsal root ganglion excitability assay (see, *e.g*., Young, G.T., et al., Mol. Ther. 22, 1530-43 (2014), and Tams, D., et al., Nature Methods 14 (2017)):
   This assay measures the effect of electrical field stimulation on the excitability of rat or human dorsal root ganglionic cells. Compounds which demonstrate the ability to decrease the excitability response of a cell when tested in this assay may be useful in treating neuropathic pain.
B. T-cell proliferation and cytokine release assay:
   This assay measures the inflammatory response.
C. Metabolism (microsomal stability) (see, *e.g.*, Chiba, M, et al., AAPS J. 2009 11(2) 262):
   This assay measures a compound's stability against microsomal metabolism, which is a primary metabolic pathway: Compounds tested in this assay which are relatively more stable than others may be more effective in treating inflammation or pain.

Furthermore, the general value of the compounds of the of the invention in treating inflammation and pain may be established in industry standard animal models for demonstrating the efficacy of compounds in treating inflammation and pain. Examples of these animal models are as follows:
A. Mouse LPS challenge (see, *e.g*., Kabir, K. et al., Shock, 2002, 17(4), 300-3): This is a well-known animal model for inflammation.
B. Mouse formalin pain (see, *e.g.,* Le Bars, D. et al., Pharmacol. Rev. 2001, 53(4), 597-652):
   This is a well-known model of inflammatory and neuropathic pain.
C. Rat TNBS Colitis (see, *e.g.,* Antoniou, E., et al., Ann Med Surg (Lond), 2016, 11, 9-15):
   This is a model for inflammatory colitis and measures inflammatory response.
D. Rat CYP Cystitis (see, *e.g.,* Golubeva, A.V., et al., Physiol. Rev., 2014, 2(3), e00260 and Keay, S., et al., BMC Urol, 2012, 12, 17):
   This is a cyclophosphamide-induced cystitis model which measures the effect on visceral/abdominal/pelvic pain. This is directly supportive of use of the compounds for treating nociceptive pain.
E. Rat Ketamine cystitis (see, *e.g.,* Jang, M.-Y., et al., Urological Science, 28(3), 123-7, 2017):
   This is a urogenital (upper and lower) model for pain.
F. Rat Chronic Prostatitis / Chronic Pelvic Pain (see, *e.g*., Radhakrishnan, R. and Nallu, R.S., 2009, Inflammopharmacology, 17: 23-28):
   This is a model for assessing a compounds ability to treat prostatitis and prostate inflammatory pain.
G. Rat MIA-induced osteoarthritis (see, *e.g*., Guingamp, C., et al., Arthritis and Rheumatism, 40(9), 1997, 1670-9):
   This is a model for chronic nociceptive joint pain, which has both an inflammatory component and a nociceptive component.
H. Rat Carrageenan-induced hyperalgesia and paw edema (see, *e.g*., Morris, C.J., Methods Mol. Biol., 2003, 225, 115-21): This is an inflammatory response model. It measures both inflammatory and pain responses.
I. Rat Complete Freund's Adjuvant model of inflammatory pain (see, *e.g*., Fehrenbacher, J.C., et al., Curr. Protoc. Pharmacol., 2012 Mar, Chapter 5, Unit 5.4):
   This is well-known model for inflammatory pain, particularly in the joints.
J. Rat Spinal nerve ligation model of neuropathic pain (see, *e.g*., Chung, J.M., et al., Methods in Molecular Medicine, 2004, 99, 35-45):
   This is a model for neuropathic pain.
K. Mouse Bleomycin Lung Fibrosis Model (see, *e.g.,* Moore, B.B., et al., Am J Respir Cell Mol Biol, 2013, 49(2), 167-79):
   This is an inflammatory response model.

### Pharmaceutical Compositions of the Invention and Administration

For the purposes of administration, the compounds of the present invention may be formulated as pharmaceutical compositions. Pharmaceutical compositions comprise one or more compounds of this invention in combination with a pharmaceutically acceptable carrier and/or diluent. The compound is present in the composition in an amount which is effective to treat inflammation and/or pain, and preferably with acceptable toxicity to the patient. Typically, the pharmaceutical compositions of the present invention may include a compound in an amount from 0.1 mg to 250 mg per dosage depending upon the route of administration, and more typically from 1 mg to 60 mg. Appropriate concentrations and dosages can be readily determined by one skilled in the art.

Pharmaceutically acceptable carrier and/or diluents are familiar to those skilled in the art. For compositions formulated as liquid solutions, acceptable carriers and/or diluents include saline and sterile water, and may optionally include antioxidants, buffers, bacteriostats and other common additives. The compositions can also be formulated as pills, capsules, granules, or tablets which contain, in addition to a compound of this invention, diluents, dispersing and surface active agents, binders, and lubricants. One skilled in this art may further formulate the compounds in an appropriate manner, and in accordance with accepted practices, such as those disclosed in Remington's Pharmaceutical Sciences (Mack Pub. Co., N.J. current edition).

In another embodiment, the present invention provides a method for treating inflammation and/or pain generally and, more specifically, to treating the diseases, disorders and conditions as discussed above. Such methods include administering of a compound of the present invention to a mammal, preferably a human, in an amount sufficient to treat the inflammation and/or pain. In this context, "treat" includes prophylactic administration. Such methods include systemic administration of a compound of the invention, preferably in the form of a pharmaceutical composition as discussed above. As used herein, systemic administration includes oral and parenteral methods of administration. For oral administration, suitable pharmaceutical compositions include powders, granules, pills, tablets, and capsules as well as liquids, syrups, suspensions, and emulsions. These compositions may also include flavorants, preservatives, suspending, thickening and emulsifying agents, and other pharmaceutically acceptable additives. For parenteral administration, the compounds of the present invention can be prepared in aqueous injection solutions which may contain buffers, antioxidants, bacteriostats, and other additives commonly employed in such solutions.

### Preparation of the Compounds of the Invention

The General Reaction Schemes below illustrate methods to make intermediates and compounds of the present invention, *i.e*., compounds of formula (I), as set forth above in the Summary of the Invention.

The compounds of the present invention may be prepared by known organic synthesis techniques, including the methods described in more detail in the Synthetic Examples. In general, the compounds of formula (I) may be made by the following General Reaction Schemes, wherein all substituents are as defined above in the Summary of the Invention unless indicated otherwise. Although not generally depicted in the following schemes, one skilled in the art will understand that appropriate protecting group strategies may be useful in preparing compounds of formula (I). Protecting group methodology is well known to those skilled in the art (*see*, *for example,* Greene, T.W. and Wuts, P.G.M. Greene's Protective Groups in Organic Synthesis (latest edition). In particular, suitable protecting groups for an oxygen atom ("oxygen protecting groups") include, but are not limited to, acetyl, trialkylsilyl or diarylalkylsilyl (*e.g.*, *t*-butyldimethylsilyl, *t*-butyldiphenylsilyl or trimethylsilyl), tetrahydropyranyl, benzyl, and the like. Suitable protecting groups for a nitrogen atom ("nitrogen protecting groups") include, but are not limited to, benzhydryl (diphenylmethyl), *t*-butoxycarbonyl, benzyloxycarbonyl, trifluoroacetate, and the like.

It is also understood that one skilled in the art would be able to make the compounds of the invention by similar methods, by methods known to one skilled in the art, or by methods similar to the methods disclosed in U.S. Patent Nos. 6,635,629, 7,601,874, and 9,765,085 and U.S. Published Patent Application No. 2017/0253596. It is also understood that one skilled in the art would be able to make in a similar manner as described below other compounds of the invention not specifically illustrated below by using the appropriate starting components and modifying the parameters of the synthesis as needed. In general, starting components may be obtained from sources such as Sigma Aldrich, Lancaster Synthesis, Inc., Maybridge, Matrix Scientific, TCI, and Fluorochem USA, etc. or synthesized according to sources known to those skilled in the art (see, *e.g.,* Smith, M.B. and J. March, March's Advanced Organic Chemistry: Reactions, Mechanisms, and Structure, 6th edition (Wiley, 2007)) or prepared as described herein. Certain starting materials, or their salts thereof, may be prepared according to the methods disclosed in U.S. Patent Nos. 6,635,629, 7,601,874 and 9,765,085 and U.S. Published Patent Application No. 2017/0253596, or by methods known to one skilled in the art.

It is also understood that in the following description, combinations of substituents and/or variables of the depicted formulae are permissible only if such contributions result in stable compounds.

It will also be appreciated by those skilled in the art, although protected derivatives of compounds of this invention may not possess pharmacological activity as such, they may be administered to a mammal and thereafter metabolized in the body to form compounds of the invention which are pharmacologically active.

When a compound of the invention is depicted in the General Reaction Schemes below without stereochemistry, it is understood that one skilled in the art would readily recognize that such compounds could also be prepared in an optically pure form by utilizing methods known to one skilled in the art, such as the use of stereoselective reagents, chiral starting materials and phase transfer catalysts.

### Abbreviations

The following abbreviations may be used herein in the following General Reaction Schemes and in the Synthetic Examples. If an abbreviation is not included below, it is understood to have its accepted meaning in the field to which it pertains:
Ac for acetyl;
Ac₂O for acetic anhydride;
ACN for acetonitrile
AcOH for acetic acid;
BALF for Bronchoalveolar lavage fluid;
BH₃·THF for borane tetrahydrofuran complex;
*n*-BuLi for *n*-butyl lithium;
*t*-BuOOH or TBHP for *tert*-butyl hydroperoxide;
CFA for Complete Freund's Adjuvant;
CP/CPPS for chronic prostatitis/chronic pelvic pain syndrome;
CSA for camphor sulfonic acid;
DCM for dichloromethane;
DMAP for 4-*N,N*-dimethylaminopyridine;
DMF for *N,N-*dimethylformamide;
DMSO for dimethyl sulfoxide;
DMP for Dess-Martin periodinane;
DRG for Dorsal root ganglion;
EFS for Electrical field stimulation;
ELISA for enzyme-linked immunosorbent assay;
ELSD for Evaporative Light Scattering Detection;
EtOAc for ethyl acetate;
EtOH for ethanol;
Eq for equivalents;
Fluo 8-AM for *Bis*(acetoxymethyl) 2,2'-((4-(6-(acetoxymethoxy)-3-oxo-3*H-*xanthen-9-yl)-2-(2-(*bis*(2-acetoxymethoxy)-2-oxoethyl)amino)phenoxy)ethoxy)phenyl)azanediyl)diacetate;
h for hours;
HCO₂Et for ethyl formate;
HPLC for high pressure liquid chromatography;
IP for intraperitoneal;
KO^{t}Bu for potassium *tert-*butoxide;
LCMS for Liquid Chromatography / Mass Spectrometry;
LC-MS/MS for Liquid Chromatography / Mass Spectrometry / Mass Spectrometry
LiHMDS for lithium *bis*(trimethylsilyl)amide;
LPS for lipopolysaccharide;
*m*-CPBA or MCPBA for *meta*-chloroperoxybenzoic acid;
m or min for minutes;
*m*CPBA for *meta-*chloroperoxybenzoic acid;
MeLi for methyl lithium;
MeOH for methanol;
MIA for monosodium iodoacetate;
MnOAc₃ for manganese (III) acetate;
MnOAc₃.2H₂O for manganese (III) acetate dihydrate;
MRM for multiple reaction monitoring;
MS for Mass spectrometry;
NBS for *N*-bromosuccinimide;
NADPH for nicotinamide adenine dinucleotide phosphate;
NaOAc for sodium acetate;
NaOMe for sodium methoxide;
NH₄OAc for ammonium acetate;
NMO for *N*-methylmorpholine *N*-oxide;
NMR for nuclear magnetic resonance;
*p*TsNHNH₂ for para-toluenesulfonyl hydrazide;
NNHTs for tosylhydrazide;
Pet ether for petroleum ether;
Ph for phenyl;
Ph₃PMeBr for methyltriphenylphosphonium bromide;
PhMe or PhCH₃ for toluene;
PhN(Tf)₂ for *N*-phenyl-*bis*(trifluoromethanesulfionimide);
PhB(OH)₂ for phenyl boronic acid;
Pd(PPh₃)₄ for tetrakis(triphenylphosphine)palladium(0);
POCl₃ for phosphoryl chloride;
*p*TsNHNH₂ for *para*-toluenesulfonyl hydrazide;
Pyr for pyridine;
RB for round bottomed;
RT for room temperature;
s for second;
Tf for triflyl;
TBAF for tetrabutylammonium fluoride;
TBDPS for *tert*-butyldiphenylsilyl;
TBHP for *tert-*butyl hydroperoxide
TBS or TBDMS for *tert*-butyldimethylsilyl;
TBSCI or TBDMSCI for *tert*-butyldimethylsilyl chloride;
TFA for trifluoroacetic acid;
THF for tetrahydrofuran;
TLC for thin layer chromatography;
TNBS for 2,4,6-Trinitrobenzenesulfonic acid;
TPAP for tetrapropylammonium perruthenate;
TTX for tetrodotoxin;
UHPLC for ultra high-pressure liquid chromatography; and
UV for ultraviolet.

The following General Methods and Procedures were used to prepare, seperate or characterize individual compounds of the invention. It will be appreciated that in the following general methods, reagent levels and relative amounts or reagents/intermediates can be changed to suit particular compounds to be synthesized, up or down by up to 50 % without significant change in expected results.

### 1. General LC/MS Analytical Methods

| Method # | Column Details | A | B | Flow rate (ml/min) | | T1 | T2 | T3 | T4 | T5 |
|---|---|---|---|---|---|---|---|---|---|---|
| Ia | A | 0.1 % TFA in water | 0.1 % TFA in ACN | 1.5 | Time | 0 | 2.5 | 4.5 | 4.6 | 6 |
| | | | | | %B | 10 | 95 | 95 | 10 | 10 |
| Ib | B | | | 2 | Time | 0 | 8 | 8.1 | 8.5 | 10 |
| | | | | | %B | 5 | 100 | 100 | 5 | 5 |
| Ic | B | 0.1 % TFA in water:ACN (95:5) | 0.1 % TFA in ACN | 1.5 | Time | 0 | 2.5 | 4 | 4.5 | 6 |
| | | | | | %B | 5 | 95 | 95 | 5 | 5 |
| Id | C | 0.1% formic in water:ACN (95:5) | ACN | 1.5 | Time | 0 | 2.5 | 4 | 4.5 | 6 |
| | | | | | %B | 5 | 95 | 95 | 5 | 5 |
| Ie | B | 10mM NH₄HCO₃ in water | ACN | 2 | Time | 0 | 4 | 5 | 5.5 | 6.5 |
| | | | | | %B | 10 | 95 | 95 | 10 | 10 |
| 1f | A | 0.1% formic acid in water | ACN | 1.5 | Time | 0 | 3 | 5 | 5.5 | 6 |
| | | | | | %B | 50 | 95 | 95 | 50 | 50 |
| 1g | B | 10mM NH₄HCO₃ in water | ACN | 1 | Time | 0 | 8 | 8.1 | 8.5 | 10 |
| | | | | | %B | 5 | 100 | 100 | 5 | 5 |
| 1h | B | 10mM NH₄HCO₃ in water | ACN | 1.2 | Time | 0 | 2.5 | 5.0 | 5.5 | 7 |
| | | | | | %B | 50 | 95 | 95 | 50 | 50 |
| 1i | D | ACN | 0.1 % formic in water | 1.5 | Time | 0 | 2.5 | 4.5 | 4.6 | 6 |
| | | | | | %B | 10 | 95 | 95 | 10 | 10 |
| 1j | E | 10mM NH₄HCO₃ in water | ACN | 1.2 | Time | 0 | 2.5 | 5.0 | 5.5 | 7 |
| | | | | | %B | 50 | 95 | 95 | 50 | 50 |

| | | | | | | | | | | |
|---|---|---|---|---|---|---|---|---|---|---|
| Columm details: A: Atlantis dC18 (50x4.6 mm, 5 µm), B: XBridge C8 (50x4.8 mm, 3.5 µm), C: Zorbax XDB C18 (50x4.6 mm, 3.5 µm), D: Zorbax C18 (50x4.8 mm, 5 µm), E: Zorbax Extend C18 (50x4.6 mm, 5 µm), | | | | | | | | | | |

### 2. General HPLC Analytical Methods

| Method # | Column Details | A | B | Flow rate (ml/min) | | T1 | T2 | T3 | T4 | T5 |
|---|---|---|---|---|---|---|---|---|---|---|
| 2a | B | 0.1 % TFA in water | 0.1 % TFA in ACN | 2 | Time | 0 | 8 | 8.1 | 8.5 | 10 |
| | | | | | %B | 5 | 100 | 100 | 5 | 5 |
| 2b | F | 10mM NH₄HCO₃ in water | ACN | 1 | Time | 0 | 15 | 20 | 26 | 30 |
| | | | | | %B | 10 | 100 | 100 | 10 | 10 |
| 2c | A | 0.1 % TFA in water | 0.1% TFA in ACN | 1.5 | Time | 0 | 8 | 8.1 | 8.5 | 10 |
| | | | | | %B | 5 | 100 | 100 | 5 | 5 |
| 2d | G | 0.1 % TFA in water | ACN | 1 | Time | 0 | 15 | 20 | 26 | 30 |
| | | | | | %B | 10 | 100 | 100 | 10 | 10 |
| 2e | B | 10mM NH₄HCO₃ in water | ACN | 1 | Time | 0 | 8 | 8.1 | 8.5 | 10 |
| | | | | | %B | 5 | 100 | 100 | 5 | 5 |

| | | | | | | | | | | |
|---|---|---|---|---|---|---|---|---|---|---|
| Columm details: A: Atlantis dC18 (50x4.6 mm, 5 µm), B: XBridge C8 (50x4.8 mm, 3.5 µm), C: Zorbax XDB C18 (50x4.6 mm, 3.5 µm), F: Phenomenex Gemini C18 (150x4.6 mm, 3.0 µm), G: Atlantis dC18 (250x4.6 mm, 5 µm) | | | | | | | | | | |

### 5 3. General Preparative HPLC Methods

| Method # | Column Details | A | B | Flow rate (ml/min) |
|---|---|---|---|---|
| 3a | H | 0.1 % TFA in water | 0.1 % TFA in ACN | 15 |
| 3b | H | 0.1% formic acid in water:ACN (95:5) | ACN | 15 |
| 3c | I | 10mM NH₄OAc in water | ACN | 22 |
| 3d | I | Water | ACN | 22 |
| 3e | H | 0.1 % TFA in water | ACN | 15 |

| | | | | |
|---|---|---|---|---|
| Columm details: H: Sunfire C18 (19x150 mm, 5 µm), I: YMC-triart C18 (30x250 mm, 5 µm) | | | | |

### GENERAL PROCEDURE A

### Acetylation with Ac₂O

To a stirred solution of the alcohol (1 equivalent) in pyridine at 0 °C were added DMAP (0.05 equivalent) and Ac₂O (1 equivalent) and the resultant solution was stirred at ambient temperature. In a standard workup, the mixture was concentrated under reduced pressure, diluted with EtOAc and washed consecutively with water and brine. The combined organic extracts were dried (Na₂SO₄ or MgSO₄), filtered and concentrated under reduced pressure. The crude material was purified by flash column chromatography on silica gel.

### GENERAL PROCEDURE B

### TBS protection of alcohols

To a stirred solution of the alcohol (1 equivalent) in DMF at ambient temperature was added imidazole (2 equivalents) followed by TBSCI (1 equivalent) at 0 °C. The resulting mixture was stirred at ambienbt temperature. In a standard workup, the mixture was concentrated under reduced pressure, diluted with CH₂Cl₂ and washed consecutively with water and brine. The combined organic extracts were dried (Na₂SO₄ or MgSO₄), filtered and concentrated under reduced pressure. The crude material was purified by flash column chromatography on silica gel.

### GENERAL PROCEDURE C

### Allylic oxidation with TBHP/cat

### 1. Option using Cooper(I)iodide (CuI)

To a stirred solution of the alkene (1 equivalent) in CH₂Cl₂:ACN (1:1) at 0 °C were added TBHP in decane (5 equivalents) and copper(I)iodide (Cul, 0.1 equivalent) and the resultant mixture was stirred at ambient temperature for 12 hours. In a standard workup, the mixture was concentrated under reduced pressure, diluted with EtOAc and washed consecutively with water and brine. The combined organic extracts were dried (Na₂SO₄ or MgSO₄), filtered and concentrated under reduced pressure. The crude material was purified by flash column chromatography on silica gel.

### 2. Option using Manuanese(III)acetate dihydrate:

To a stirred solution of the alkene (1 equivalent) in CH₂Cl₂:ACN:EtOAc (1:1:1) at room temperature were added TBHP in decane (5.2 equivalents) and 4 Å molecular sieves, the resultant mixture was stirred at room temperature for 0.5 hours. At this point, was added Mn(OAc)₃2H₂O (0.1 equivalent) and stirring was continued overnight at ambient temperature. In a standard workup, the mixture was filtered through a bed of CELITE^{®} and the filtrate was concentrated under reduced pressure. The crude material was purified by flash column chromatography on silica gel.

### 3. Option using Selenium dioxide (SeO₂):

To a stirred solution of the alkene (1 equivalent) in CH₂Cl₂ at 0 °C were added TBHP in decane (5 equivalents) and selenium dioxide (0.5 equivalent) and the resultant mixture was stirred at ambient temperature for 12 hours. In a standard workup, the mixture was diluted with CH₂Cl₂ and washed consecutively with water and brine. The combined organic extracts were dried (Na₂SO₄ or MgSO₄), filtered and concentrated under reduced pressure. The crude material was purified by flash column chromatography on silica gel.

### GENERAL PROCEDURE D

### Hydroboration sequence

To a stirred solution of the ketone (1 equivalent) in tetrahydrofuran (THF) at 0 °C was added Borane in THF (1 M, 2.23 equivalents). The resulting mixture was stirred at same temperature for 12 hours. The mixture was quenched with dropwise addition of purified chilled water and stirred for 15 minutes. To the resulting mixture was added sodium perborate tetrahydrate at ambient temperature and stirred for 3 hours. In a standard workup, the mixture was diluted with EtOAc and washed consecutively with water and brine. The combined organic extracts were dried (Na₂SO₄ or MgSO₄), filtered and concentrated under reduced pressure. The crude material was purified by flash column chromatography on silica gel.

### GENERAL PROCEDURE E

### Ketal! Acetal deprotection with AcOH

The Ketal/Acetal protected moiety (1 equivalent) was dissolved in 80 % aqueous AcOH. The resulting mixture was heated to 65 °C and stirred for 1 hour. In a standard workup, the mixture was concentrated under reduced pressure and diluted with saturated aqueous NaHCO₃. The aqueous was extracted with CH₂Cl₂. The combined organic extracts were washed with brine, dried (Na₂SO₄ or MgSO₄), filtered and concentrated under reduced pressure. The crude material was purified by flash column chromatography on silica gel.

### GENERAL PROCEDURE F

### Acetonide formation with 2,2-dimethoxypropane

To a stirred solution of the diol (1 equivalent) in 2,2-dimethoxypropane at 0 °C was added camphorsulphonic acid (0.1 equivalent). The resulting mixture was stirred at ambient temperature 2-4 hours. In a standard workup, the mixture was concentrated under reduced pressure and diluted with EtOAc and washed consecutively with saturated aqueous NaHCO₃ and brine. The combined organic extracts were dried (Na₂SO₄ or MgSO₄), filtered and concentrated under reduced pressure. The crude material was purified by flash column chromatography on silica gel.

### GENERAL PROCEDURE G

### Wittig reaction

To a stirred solution of methyltriphenylphosphonium bromide (Wittig reagent, 3 equivalents) in THF at 0 °C was added potassium tert-butoxide (2.9 equivalents) and the mixture was stirred at ambient temperature for 2 hours. To the resultant solution was added ketone (1 equivalent) in tetrahydrofuran (THF) at room temperature and stirred overnight. In a standard workup, the mixture was diluted with EtOAc and washed consecutively with saturated aqueous NaHCO₃ and brine. The combined organic extracts were dried (Na₂SO₄ or MgSO₄), filtered and concentrated under reduced pressure. The crude material was purified by flash column chromatography on silica gel.

### GENERAL PROCEDURE H

### TPAP/NMO oxidation

To a stirred solution of the alcohol (1 equivalent) in CH₂Cl₂ at 0 °C were added N-methylmorpholine *N*-oxide (NMO·H₂O, 2 equivalents), 4 Å molecular sieves and tetrapropylammonium perruthenate (TPAP, 0.1 equivalents). The resulting mixture was stirred at ambient temperature for 2 hours. In a standard workup, the mixture was diluted with CH₂Cl₂ and filtered through a bed of CELITE^{®} and the filtrate was concentrated under reduced pressure. The crude material was purified by flash column chromatography on silica gel.

### GENERAL PROCEDURE I

### Dess-Martin periodinane oxidation

To a stirred solution of the alcohol (1 equivalent) in CH₂Cl₂ at 0 °C was added Dess-Martin periodinane (2 equivalents). The resulting mixture was stirred at ambient temperature for 2 hours. In a standard workup, the mixture was diluted with CH₂Cl₂ and washed consecutively with saturated aqueous NaHCO₃ and brine. The combined organic extracts were dried (Na₂SO₄ or MgSO₄), filtered and concentrated under reduced pressure. The crude material was purified by flash column chromatography on silica gel.

### GENERAL PROCEDURE J

### Knoevenagel condensation

To a stirred solution of the sodium hydride (60 % dispersion in mineral oil) (4 equivalents) in THF at 0 °C was added the ketone in THF (1 equivalent) dropwise. The resulting mixture was stirred at same temperature for 2 hours. To the resulting mixture was added ethyl formate (6 equivalents) at 0 °C and stirred at ambient temperature for 8 hours. In a standard workup, the mixture was quenched with a saturated aqueous solution of NH₄Cl and the aqueous layer extracted with EtOAc. The combined organic extracts were washed with brine, dried (Na₂SO₄ or MgSO₄), filtered and concentrated under reduced pressure. The crude material was purified by flash column chromatography on silica gel. Alternative conditions for the Knoevenagel condensation include the following: To a stirred solution of the ketone in toluene at 0 °C were added sodium methoxide solution (25 % wt. in MeOH, 1.5 to 3 equivalents) and ethyl formate (5-6 equivalents) dropwise and the resultant solution stirred at ambient temperature for 16 hours. In a standard workup, the mixture was concentrated under reduced pressure and diluted with chilled water. The aqueous layer was extracted with EtOAc, washed with brine and the organic layer dried (Na₂SO₄ or MgSO₄), filtered, concentrated and purified by silica gel chromatography, if required.

### GENERAL PROCEDURE K

### Hydrazine condensation to form pyrazoles

To a stirred solution of the ketone (1 equivalent) in EtOH at ambient temperature was added hydrazine hydrate (2 equivalents) dropwise. The resulting mixture was heated to 70 °C and stirred for 2 hours. In a standard workup, the mixture was concentrated under reduced pressure and diluted with EtOAc and washed consecutively with water and brine. The combined organic extracts were dried (Na₂SO₄ or MgSO₄), filtered and concentrated under reduced pressure. The crude material was purified by flash column chromatography on silica gel.

### GENERAL PROCEDURE L

### TBS deprotection with TBAF

The TBS silyl ether (1 equivalent) was dissolved in THF and TBAF, (1 M in THF, 2 equivalents) was added at ambient temperature. The mixture was heated to 65 °C and stirred for 2 hours. In a standard workup, the mixture was diluted with EtOAc and washed consecutively with water and brine. The combined organic extracts were dried (Na₂SO₄ or MgSO₄), filtered and concentrated under reduced pressure. The crude material was purified by flash column chromatography on silica gel.

### GENERAL REACTION SCHEMES

The following General Reaction Schemes illustrate methods to make compounds of formula (I), or mixtures thereof, or pharmaceutically acceptable salts or solvates thereof, as set forth above in the Summary of the Invention. Specifically General Reaction Schemes 1-4, 6 and 7 relate to the preparation of compounds of the present invention. The other General Reaction Schemes relate to the preparation of compounds not forming part of the present invention, some of which may serve as intermediates for the preparation of compounds of the present invention of formula (I).

### General Reaction Scheme 1

Compounds of formula (I-1) are compounds of formula (I), as described above in the Summary of the Invention, and may be prepared according the following General Reaction Scheme 1 wherein R¹ and R⁵ are as described above in the Summary of the Invention, Pg¹ is an oxygen protecting group, such as *tert-*butydimethylsilyl or *tert-*butyldiphenylsilyl, and X is halo, preferably chloro:

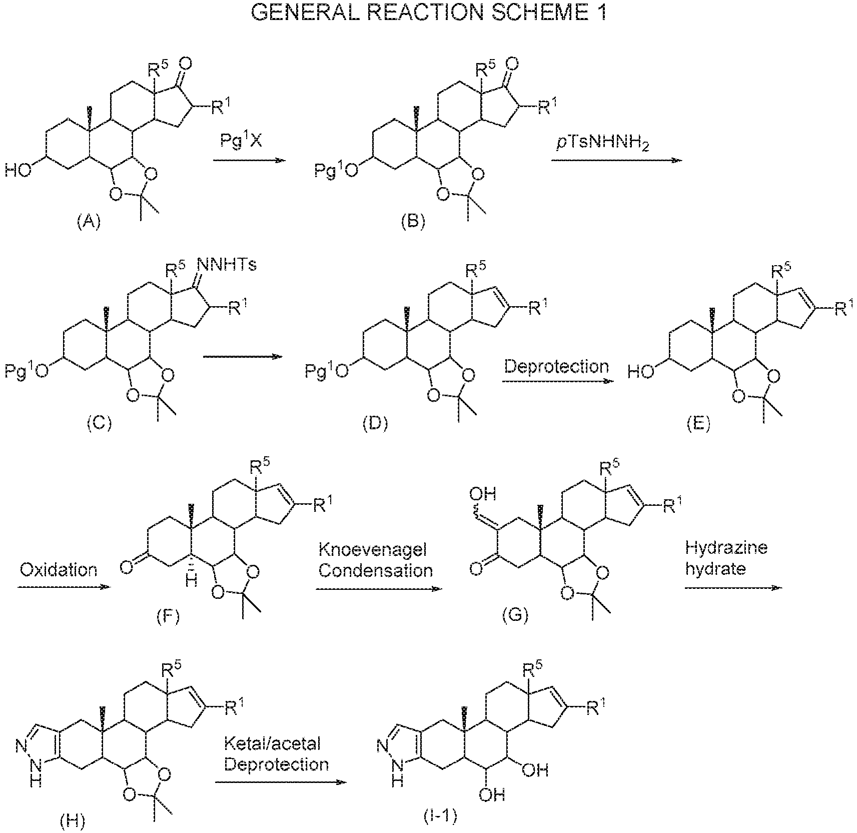

Compounds of formula (A) may be prepared according to the methods disclosed in U.S. Patent No. 6,046,185, or by methods known to one skilled in the art.

In general, compounds of formula (I-1) are prepared by first treating a compound of formula (A) under the appropriate General Procedure B conditions to yield a compound of formula (B), which is then treated with *p*-toluenesulfonyl hydrazide under the appropriate conditions to yield a compound of formula (C), which is then treated under the appropriate basic conditions (*e.g*., organolithium reagent such as *n-*butyllithium) conditions to yield a compound of formula (D), which is then treated under the appropriate General Procedure L conditions to yield a compound of formula (E), which is then treated under the appropriate General Procedure I conditions to yield a compound of formula (F), which is then treated under the appropriate General Procedure J conditions to yield a compound of formula (G), which is then treated under the appropriate General Procedure K conditions to yield a compound of formula (H), which is then treated under the appropriate General Procedure E conditions to yield a compound of formula (I-1).

An embodiment of General Reaction Scheme 1 is described in more detail below in Synthetic Example 1.

### General Reaction Scheme 2

Compounds of formula (I-2) are compounds of formula (I), as described above in the Summary of the Invention, and may be prepared according the following General Reaction Scheme 2 wherein R¹ and R⁵ are as described above in the Summary of the Invention, R^{4a} is hydrogen, alkyl, alkenyl, optionally substituted aryl or optionally substituted heteroaryl, R^{4b} is hydrogen or alkyl, Pg¹ is an oxygen protecting group, such as *tert*-butydimethylsilyl or *tert*-butyldiphenylsilyl, and X is halo, preferably chloro:

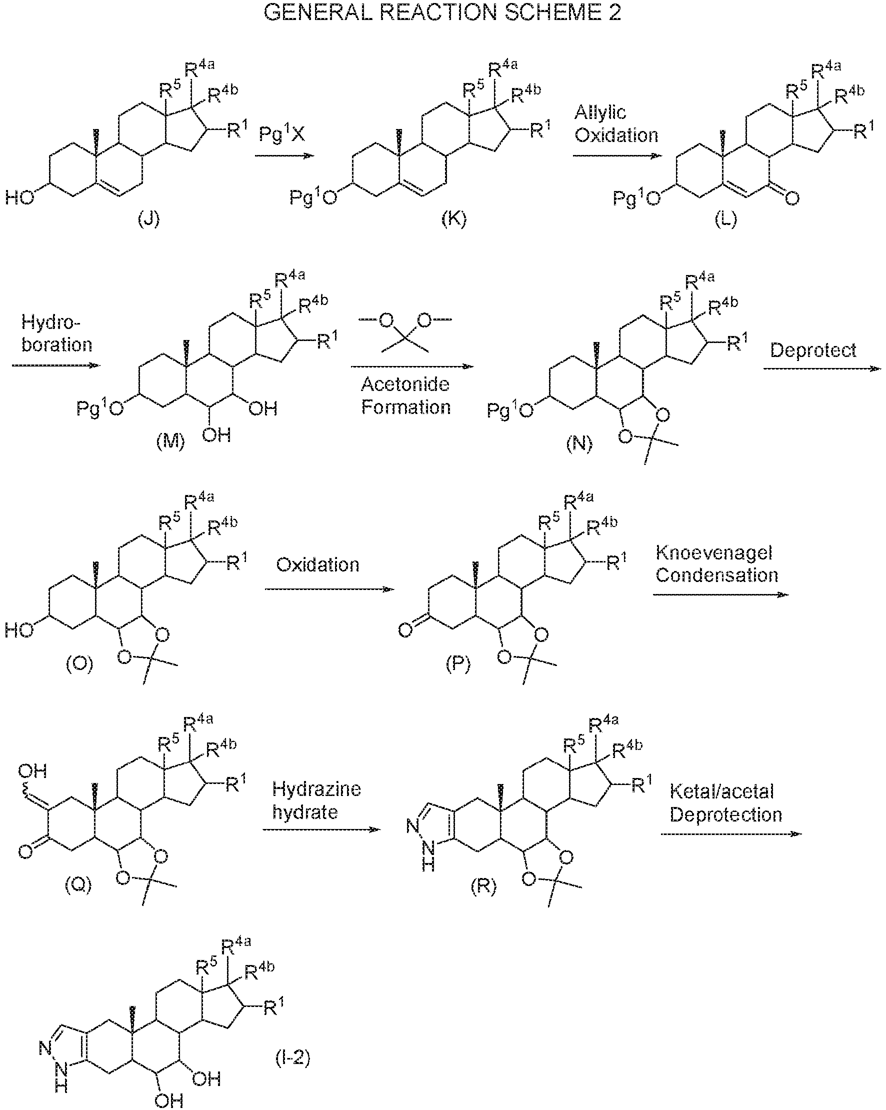

Compounds of formula (J) are commercially available, such as cholesterol, or can be prepared accordingly to methods known to one skilled in the art.

In general, compounds of formula (I-2) are prepared by first treating a compound of formula (J) under appropriate General Procedure B conditions to yield a compound of formula (K), which is then treated under appropriate General Procedure C conditions to yield a compound of formula (L), which is then treated under appropriate General Procedure D conditions to yield a compound of formula (M), when is then treated with 2,2-dimethoxypropane under appropriate General Procedure F conditions to yield a compound of formula (N), which is then treated under appropriate General Procedure L conditions to yield a compound of formula (O), which is then oxidized under appropriate General Procedure H conditions to yield a compound of formula (P), which is then treated under appropriate General Procedure J conditions to yield a compound of formula (Q), when is then treated with hydrazine hydrate under appropriate General Procedure (K) conditions to yield a compound of formula (R), when is then treated under appropriate General Procedure E conditions to yield a compound of formula (I-2).

An embodiment of General Reaction Scheme 2 is described in more detail below in Synthetic Example 2.

### General Reaction Scheme 3

Compounds of formula (I-3) are compounds of formula (I), as described above in the Summary of the Invention, and may be prepared according the following General Reaction Scheme 3 wherein R¹ is hydrogen, R⁵ is alkyl, R^{4a} is alkyl, alkenyl, optionally substituted aryl or optionally substituted heteroaryl, R^{4b} is alkyl and Pg¹ is an oxygen protecting group, such as *tert*-butydimethylsilyl or *tert-*butyldiphenylsilyl:

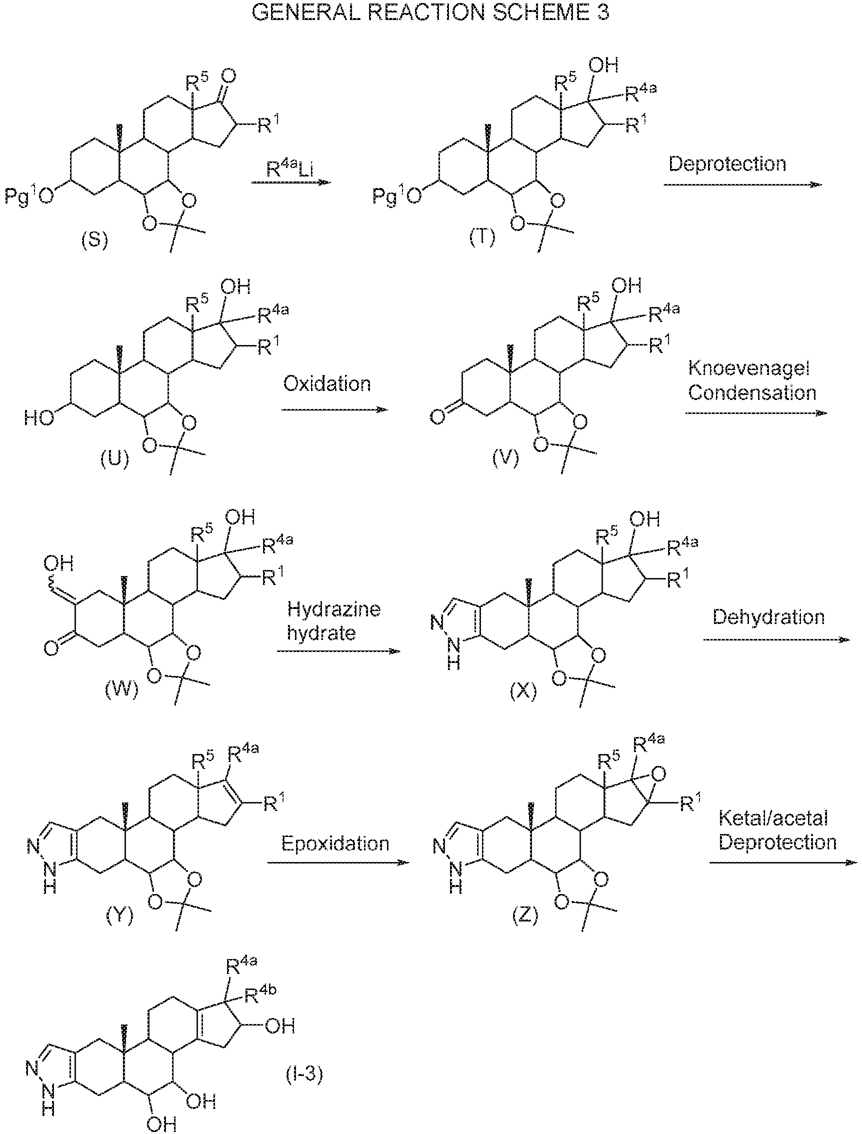

Compounds of formula (S) may be prepared accordingly to methods described herein or by methods known to one skilled in the art.

In general, compounds of formula (I-3) are prepared by nucleophilic addition to a compound of formula (S) with R^{4a}Li under standard reduction conditions to yield a compound of formula (T), which is then treated under appropriate General Procedure L conditions to yield a compound of formula (U), which is then treated under appropriate General Procedure I conditions to yield a compound of formula (V), which is then treated under appropriate General Procedure J conditions to yield a compound of formula (W), which is then treated under appropriate General Procedure K conditions to yield a compound of formula (X), which is then treated under appropriate dehydration conditions to yield a compound of formula (Y), which is then treated under appropriate epoxidation conditions to yield a compound of formula (Z), which is then treated under appropriate General Procedure E conditions to yield a compound of formula (I-3).

An embodiment of General Reaction Scheme 3 is described in more detail below in Synthetic Example 3.

Alternatively, compounds of formula (X) prepared above can be treated under appropriate General Procedure E conditions to yield compounds of formula (I-3a): 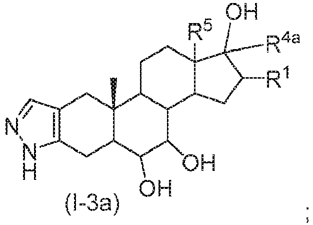 where R¹, R^{4a} and R⁵ are as described above for compounds of formula (I-3). Embodiments of preparing compounds of formula (I-3a) are described in more detail below in Synthetic Example 7 and Synthetic Examples 9 and 9.1.

### General Reaction Scheme 4

Compounds of formula (I-4) are compounds of formula (I), as described above in the Summary of the Invention, and may be prepared according the following General Reaction Scheme 4 wherein R¹ is hydrogen, R⁵ is methyl, and Pg¹ is an oxygen protecting group, such as *tert*-butydimethylsilyl or *tert-*butyldiphenylsilyl:

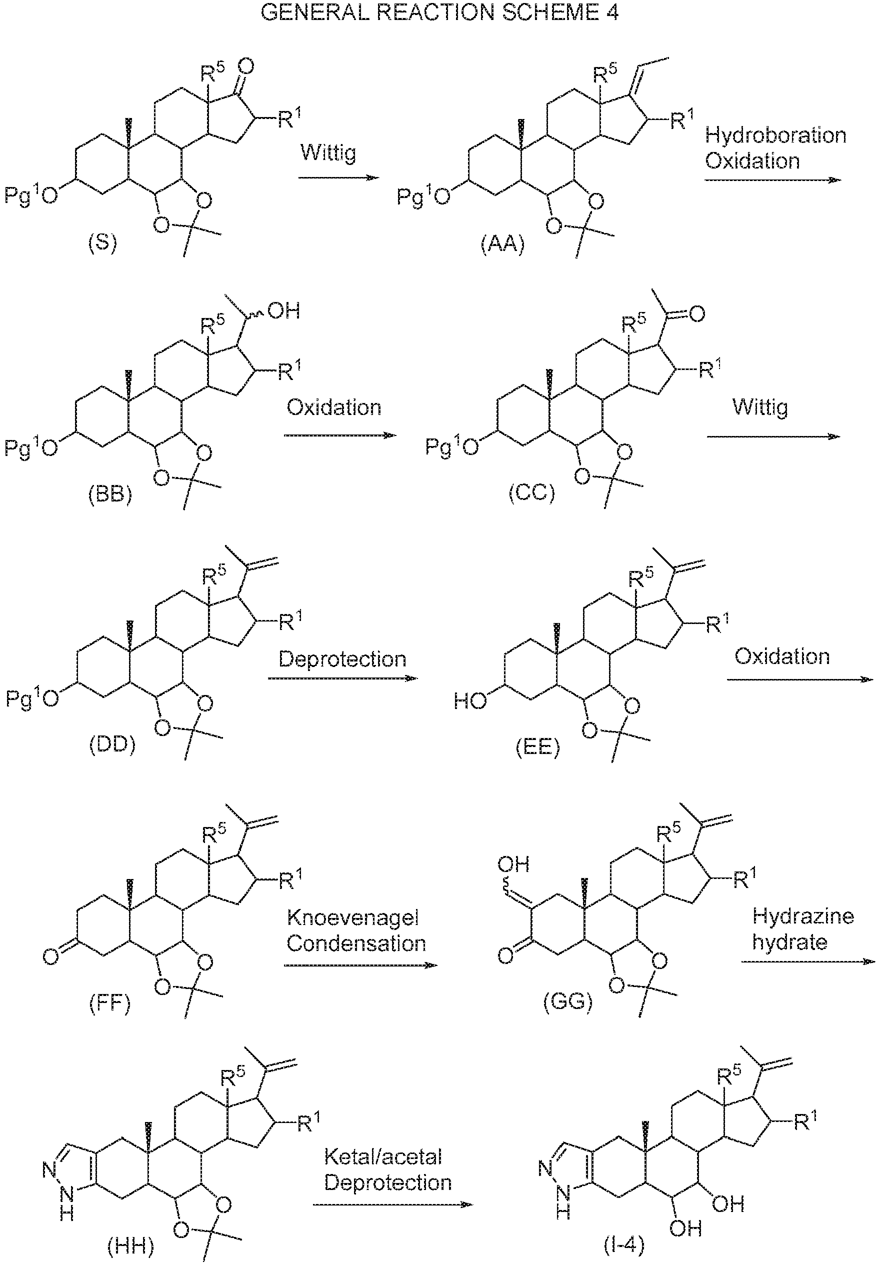

Compounds of formula (S) may be prepared according to the methods described herein or by methods known to one skilled in the art.

In general, compounds of formula (I-4) are prepared by first treating a compound of formula (S) under appropriate General Procedure G conditions to yield a compound of formula (AA), which is then treated under standard hydroboration conditions to yield a compound of formula (BB), which is then treated under appropriate General Procedure H conditions to yield a compound of formula (CC), which is then treated under appropriate General Procedure G conditions to yield a compound of formula (DD), which is then treated under appropriate General Procedure L conditions to yield a compound of formula (EE), which is then treated under appropriate General Procedure H conditions to yield a compound of formula (FF), which is then treated under appropriate General Procedure J conditions to yield a compound of formula (GG), which is then treated under appropriate General Procedure K conditions to yield a compound of formula (HH), which is then treated under appropriate General Procedure E conditions to yield a compound of formula (I-4).

An embodiment of General Reaction Scheme 4 is described in more detail below in Synthetic Example 4.

### General Reaction Scheme 5

Compounds of formula (I-5) are compounds of formula (I), as described above in the Summary of the Invention, and may be prepared according the following General Reaction Scheme 5 wherein R¹ and R⁵ are as described above in the Summary of the Invention, and Pg¹ is an oxygen protecting group, such as *tert*-butydimethylsilyl or *tert-*butyldiphenylsilyl:

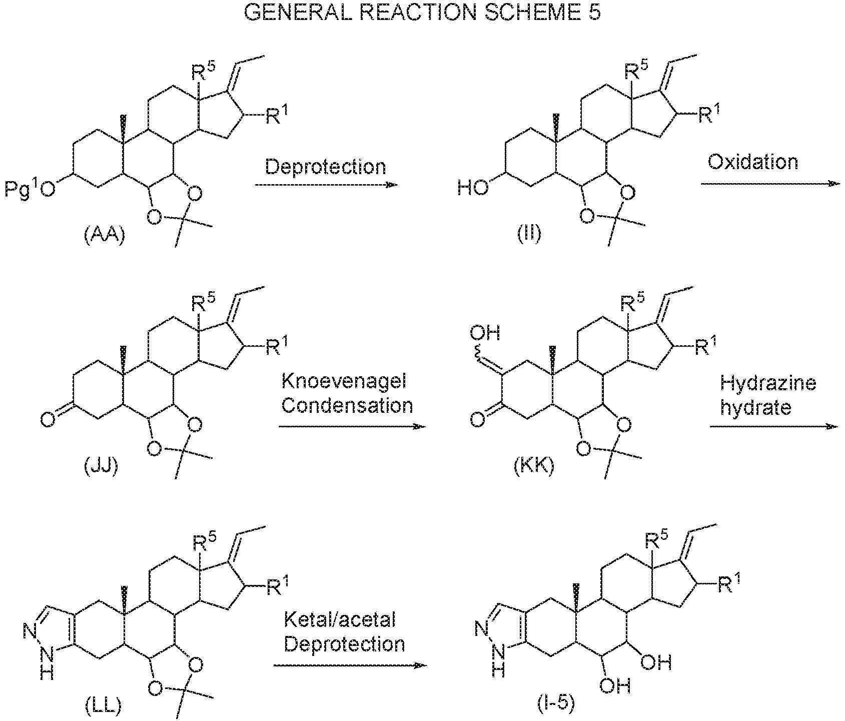

Compounds of formula (AA) may be prepared according to the methods described herein or by methods known to one skilled in the art.

In general, compounds of formula (I-5) are prepared by first treating a compound of formula (AA) under the appropriate General Procedure L conditions to y a compound of formula (II), which is then treated under the appropriate General Procedure H conditions to yield a compound of formula (JJ), which is then treated under the appropriate General Procedure J conditions to yield a compound of formula (KK), which is then treated under the appropriate General Procedure K conditions to yield a compound of formula (LL), which is then treated under the appropriate General Procedure E conditions to yield a compound of formula (I-5).

An embodiment of General Reaction Scheme 5 is described in more detail below in Synthetic Example 5.

### General Reaction Scheme 6

Compounds of formula (I-6) are compounds of formula (I), as described above in the Summary of the Invention, and may be prepared according the following General Reaction Scheme 6 wherein R¹ is hydrogen, R⁵ is as described above in the Summary of the Invention, R^{4a} is optionally substituted aryl or optionally substituted heteroaryl, and Pg¹ is an oxygen protecting group, such as *tert*-butydimethylsilyl or *tert-*butyldiphenylsilyl:

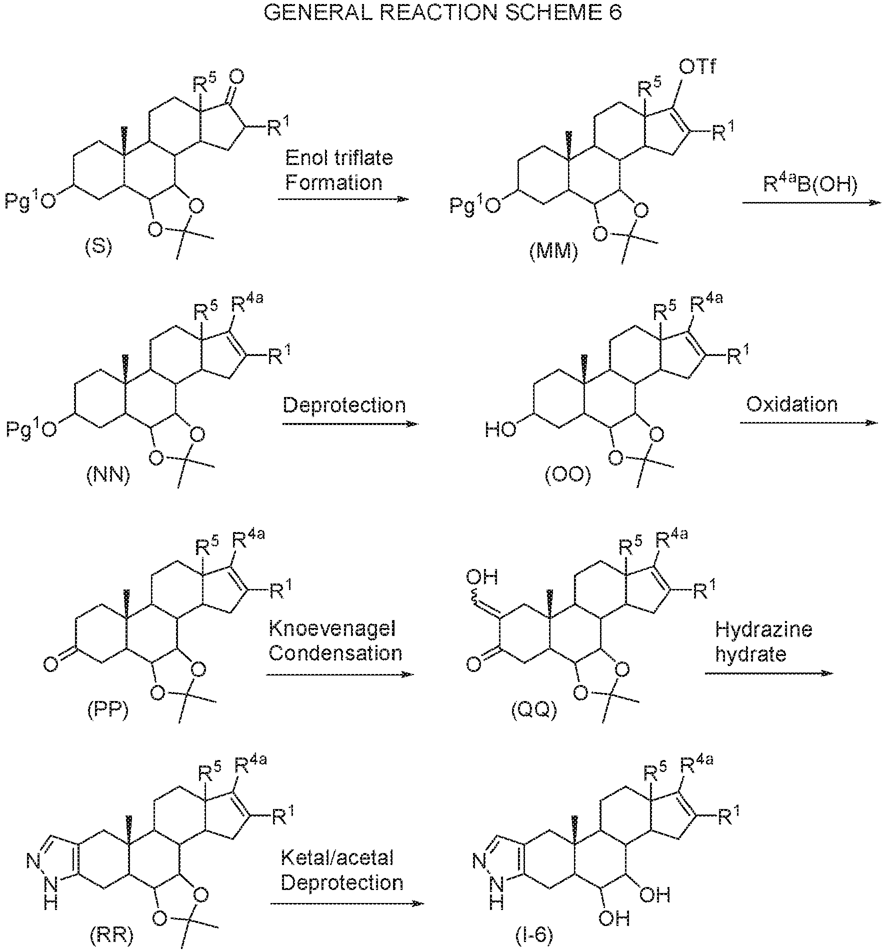

Compounds of formula (S) may be prepared according to the methods described herein or by methods known to one skilled in the art.

In general, compounds of formula (I-6) are prepared by first treating a compound of formula (S) under appropriate enol triflate formation conditions to yield a compound of formula (MM), which is then treated with R^{4a}B(OH) under the appropriate conditions to yield a compound of formula (NN), which is then treated under appropriate General Procedure L conditions to yield a compound of formula (OO), which is then treated under appropriate General Procedure H conditions to yield a compound of formula (PP), which is then treated under appropriate General Procedure J conditions to yield a compound of formula (QQ), which is then treated under appropriate General Procedure K conditions to yield a compound of formula (RR), which is then treated under appropriate General Procedure E conditions to yield a compound of formula (I-6).

An embodiment of General Reaction Scheme 6 is described in more detail below in Synthetic Example 6.

### General Reaction Scheme 7

Compounds of formula (I-7) are compounds of formula (I), as described above in the Summary of the Invention, and may be prepared according the following General Reaction Scheme 7 wherein R¹ and R⁵ are as defined above in the Summary of the Invention, Pg¹ is an oxygen protecting group, such as *tert*-butydimethylsilyl or *tert-*butyldiphenylsilyl and X is halo, preferably chloro:

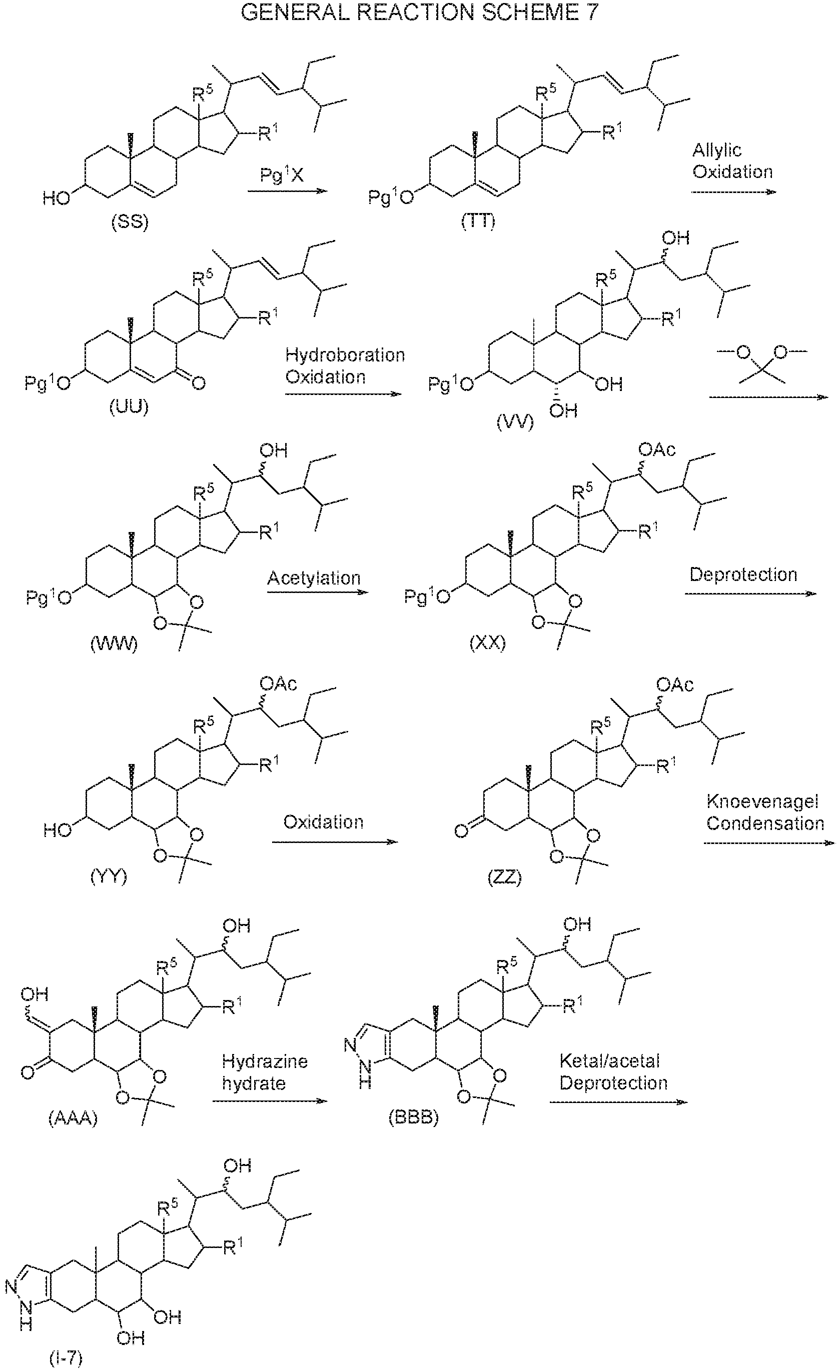

Compounds of formula (SS), such as stigmasterol, are commercially available or can be prepared accordingly to methods known to one skilled in the art.

In general, compounds of formula (I-7) are prepared by first treating a compound of formula (SS) under the appropriate General Procedure B conditions to yield a compound of formula (TT), which is then treated under the appropriate General Procedure C conditions to yield a compound of formula (UU), which is then treated under the appropriate General Procedure D conditions to yield a compound of formula (VV), which is then treated under the appropriate General Procedure F conditions to yield a compound of formula (WW), which is then treated under the appropriate General Procedure A conditions to yield a compound of formula (XX), which is then treated under the appropriate General Procedure L conditions to yield a compound of formula (YY), which is then treated under the appropriate General Procedure H conditions to yield a compound of formula (ZZ), which is then treated under the appropriate General Procedure J conditions to yield a compound of formula (AAA), which is then treated under the appropriate General Procedure K conditions to yield a compound of formula (BBB), which is then treated under the appropriate General Procedure E conditions to yield a compound of formula (I-7).

An embodiment of General Reaction Scheme 7 is described in more detail below in Synthetic Example 8.

### General Reaction Scheme 8

Compounds of formula (I-8) are compounds of formula (I), as described above in the Summary of the Invention, and may be prepared according the following General Reaction Scheme 8 wherein R¹ and R⁵ are as defined above in the Summary of the Invention:

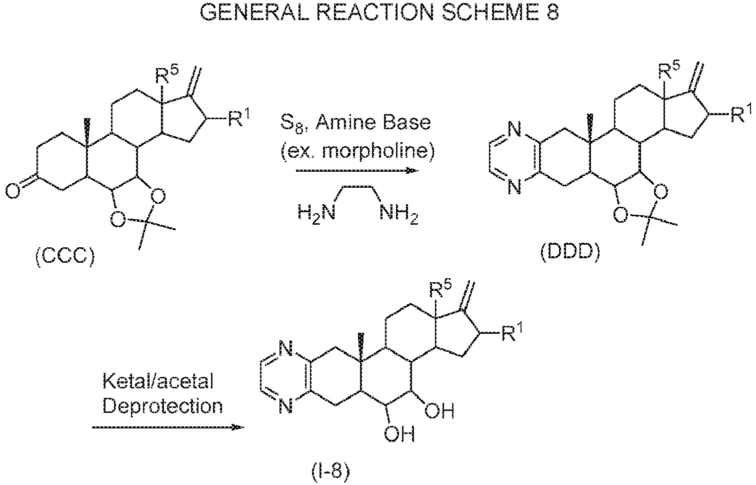

Compounds of formula (CCC) may be prepared according to the methods disclosed in U.S. Pat. No. 9,765,085 or by methods known to one skilled in the art.

In general, compounds of formula (I-8) are prepared by first treating a compound of formula (CCC) with 1,2-ethanediamine under appropriate cyclization conditions to yield a compound of formula (DDD), which is then treated under appropriate General Procedure E conditions to yield a compound of formula (I-8).

An embodiment of General Reaction Scheme 8 is described in more detail below in Synthetic Example 10.

### General Reaction Scheme 9

Compounds of formula (I-9) are compounds of formula (I), as described above in the Summary of the Invention, and may be prepared according the following General Reaction Scheme 9 wherein R¹ and R⁵ are as described above in the Summary of the Invention, R^{4a} is hydrogen, alkyl, alkenyl, optionally substituted aryl or optionally substituted heteroaryl, and R^{4b} is hydrogen or alkyl:

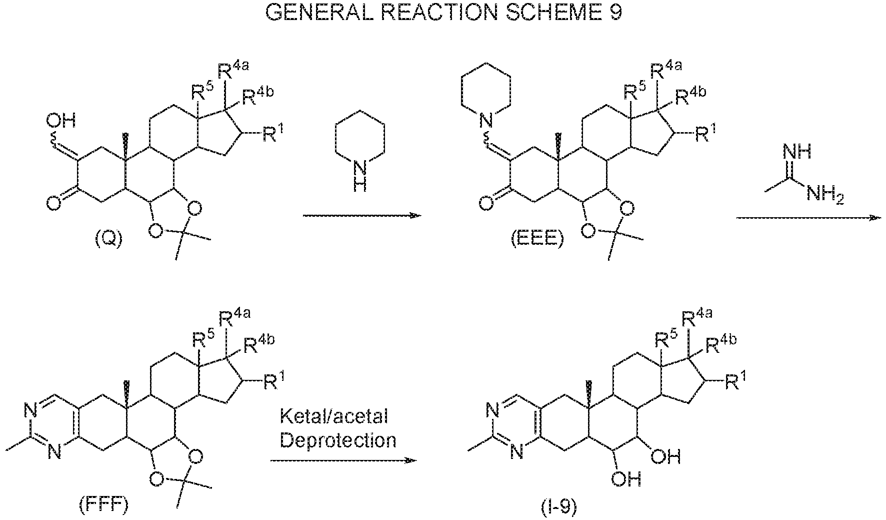

Compounds of formula (Q) can be prepared by methods disclosed herein or by methods known to one skilled in the art.

In general, compounds of formula (I-9) are prepared by first treating a compound of formula (Q) with piperidine under appropriate enamine formation conditions to yield a compound of formula (EEE), which is then treated with acetamidine under appropriate condensation / cyclization conditions to yield a compound of formula (FFF), which is then treated under appropriate General Procedure E conditions to yield a compound of formula (I-9).

An embodiment of General Reaction Scheme 9 is described in more detail below in Synthetic Example 11.

### General Reaction Scheme 10

Compounds of formula (I-10) are compounds of formula (I), as described above in the Summary of the Invention, and may be prepared according the following General Reaction Scheme 10 wherein R¹ and R⁵ are as described above in the Summary of the Invention, R^{4a} is hydrogen, alkyl, alkenyl, optionally substituted aryl or optionally substituted heteroaryl and R^{4b} is hydrogen or alkyl:

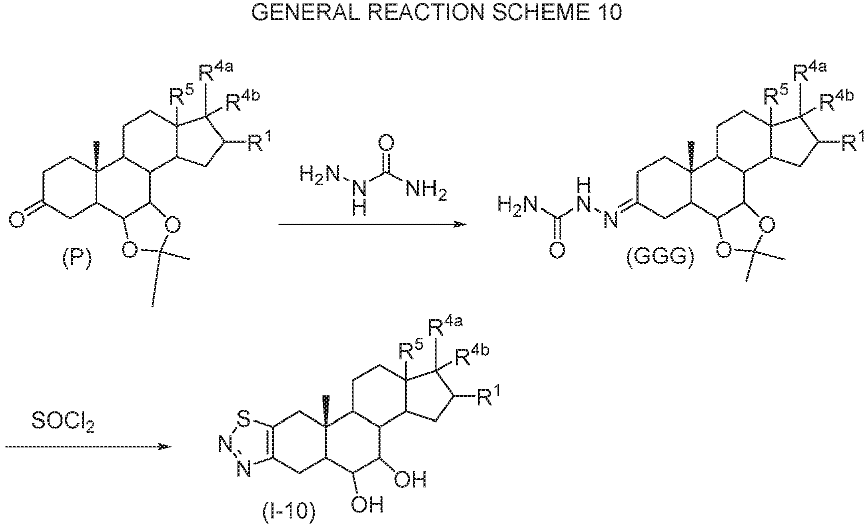

Compounds of formula (P) may be prepared by methods disclosed herein or by methods known to one skilled in the art.

In general, compounds of formula (I-10) are prepared by first treating a compound of formula (P) with semicarbazide under appropriate condensation conditions to yield a compound of formula (GGG), which is then treated with sulfonyl chloride under appropriate cyclization conditions to yield a compound of formula (I-10).

An embodiment of General Reaction Scheme 10 is described in more detail below in Synthetic Example 12.

### General Reaction Scheme 11

Compounds of formula (I-11) are compounds of formula (I), as described above in the Summary of the Invention, and may be prepared according the following General Reaction Scheme 11 wherein R¹ and R⁵ are as described above in the Summary of the Invention, R^{4a} is hydrogen, alkyl, alkenyl, optionally substituted aryl or optionally substituted heteroaryl and R^{4b} is hydrogen or alkyl:

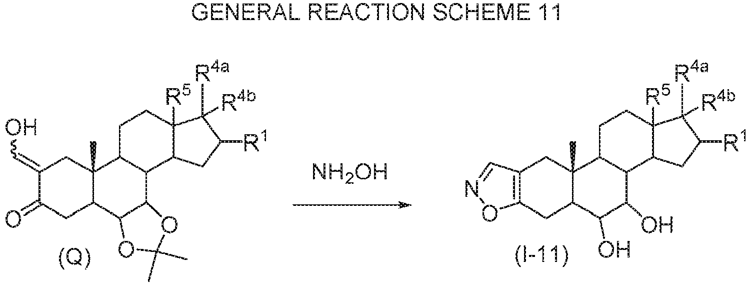

Compounds of formula (Q) may be prepared by methods disclosed herein or by methods known to one skilled in the art.

In general, compounds of formula (I-11) are prepared by treating a compound of formula (Q) with hydroxylamine under appropriate condensation / cyclization conditions to yield a compound of formula (I-11).

An embodiment of General Reaction Scheme 11 is described in more detail below in Synthetic Example 13.

### General Reaction Scheme 12

Compounds of formula (I-12) are compounds of formula (I), as described above in the Summary of the Invention, and may be prepared according the following General Reaction Scheme 12 wherein R¹ and R⁵ are as defined above in the Summary of the Invention:

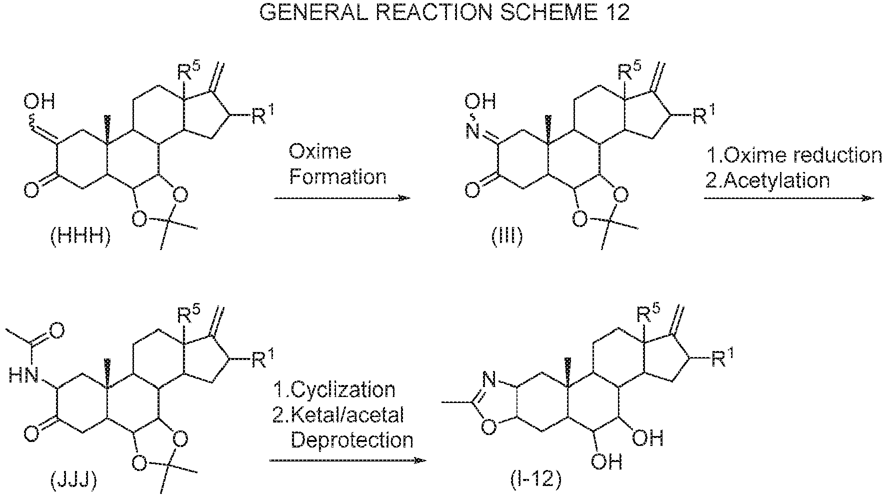

Compounds of formula (HHH) may be prepared by methods similar to the methods disclosed in U.S. Patent No. 9,765,085, or by methods known to one skilled in the art.

In general, compounds of formula (I-12) are prepared by first treating a compound of formula (HHH) under appropriate oxime formation conditions to yield a compound of formula (III), which is then treated under appropriate oxime reduction followed by acetylation conditions to yield a compound of formula (JJJ), which is then treated under appropriate cyclization conditions, followed by treatment under General Procedure E to yield a compound of formula (I-12).

An embodiment of General Reaction Scheme 12 is described in more detail below in Synthetic Example 14.

### General Reaction Scheme 13

Compounds of formula (I-13) are compounds of formula (I), as described above in the Summary of the Invention, and may be prepared according the following General Reaction Scheme 13 wherein R¹ and R⁵ are as defined above in the Summary of the Invention:

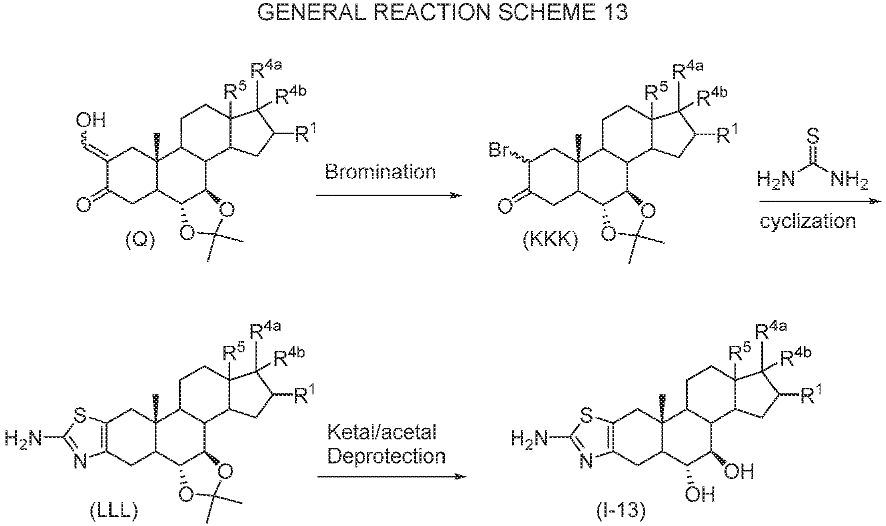

Compounds of formula (Q) may be prepared by methods disclosed herein or by methods known to one skilled in the art.

In general, compounds of formula (I-13) are prepared by first treating a compound of formula (Q) under appropriate bromination conditions to yield a compound of formula (KKK), which is then treated with thiourea under appropriate cyclization conditions to yield a compound of formula (LLL), which is then treated under appropriate General Procedure E conditions to yield a compound of formula (I-13).

An embodiment of General Reaction Scheme 13 is described in more detail below in Synthetic Example 15.

### General Reaction Scheme 14

Compounds of formula (I-14a) and formula (I-14b) are compounds of formula (I), as described above in the Summary of the Invention, and may be prepared according the following General Reaction Scheme 14 wherein R¹ and R⁵ are as defined above in the Summary of the Invention and Pg¹ is an oxygen protecting group, such as tert-butydimethylsilyl or *tert*-butyldiphenylsilyl:

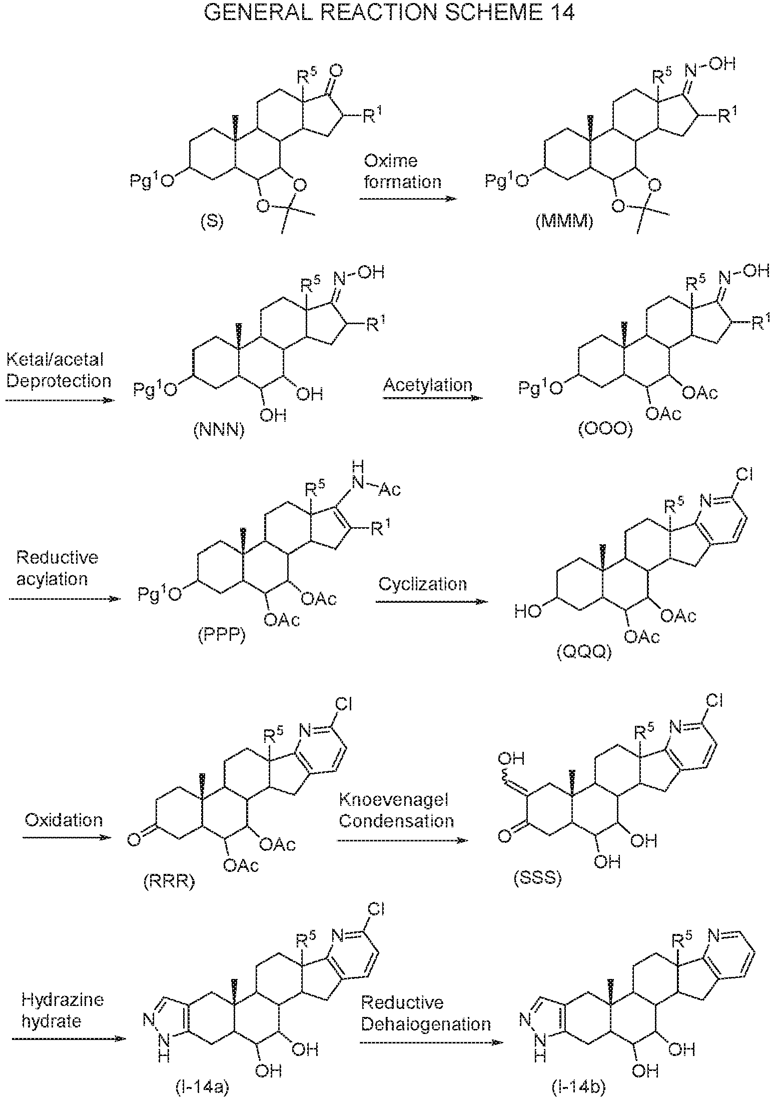

Compounds of formula (S) may be prepared according to the methods disclosed herein or by methods known to one skilled in the art.

In general, compounds of formula (I-14a) and (I-14b) are prepared by first treating a compound of formula (S) under standard oxime formation conditions to yield a compound of formula (MMM), which is then treated under the appropriate General Procedure E conditions to yield a compound of formula (NNN), which is then treated under the appropriate General Procedure A conditions to yield a compound of formula (OOO), which is then treated under the appropriate reductive acylation conditions to yield a compound of formula (PPP), which is then treated under cyclization conditions to yield a compound of formula (QQQ), which is then treated under the appropriate General Procedure H conditions to yield a compound of formula (RRR), which is then treated under the appropriate General Procedure J conditions to yield a compound of formula (SSS), which is then treated under the appropriate General Procedure K conditions to yield a compound of formula (I-14a), which is then treated under standard reductive dehalogenation conditions to yield a compound of formula (I-14b).

An embodiment of General Reaction Scheme 14 is described in more detail below in Synthetic Example 16.

The compounds of formulae (I-1), (I-2), (I-3), (I-4), (I-5), (I-6), (I-7), (I-8), (I-9), (I-10), (I-11), (I-12), (I-13), (I-14a) and (I-14b), as prepared as described above in the General Reaction Schemes 1-13, may be converted to compounds of formula (I) wherein R² and R³ are -N(R⁷)₂ by methods known to one skilled in the art.

All of the compounds described herein as being prepared which may exist in free base or acid form may be converted to their pharmaceutically acceptable salts by treatment with the appropriate inorganic or organic base or acid. Salts of the compounds prepared below may be converted to their free base or acid form by standard techniques. Furthermore, all compounds of the invention which contain an acid or an ester group can be converted to the corresponding ester or acid, respectively, by methods known to one skilled in the art or by methods described herein.

Representative compounds of the invention which were prepared by the methods disclosed herein include (but are not limited to) the compounds la-1, la-2, la-3, la-4, la-7 and la-8 listed below in Table 1. The compound (Cpd) numbers in this table correspond to the compound numbers in Synthetic Examples 1-16 below. Compounds other than la-1, la-2, la-3, la-4, la-7 and la-8 listed below and prepared according to the synthetic examples 1-16 below do not form part of the present invention and are disclosed here for the purposes of comparison only.

**TABLE 1**

| Cpd No. | Compound Name |
|---|---|
| la-1 | (3a*S*,3b*R*,4*R*,5*R*,5a*S*,10a*R*,10b*S*,12a*R*)-10a,12a-dimethyl-3,3a,3b,4,5,5a,6,7,10,10a,10b,11,12,12a-tetradecahydrocyclopenta[5,6]naphtho[1,2-*f*]indazole-4,5-diol |
| la-2 | (1*R*,3a*S*,3b*S*,4*R*,5*R*,5a*S*,10a*R*,12a*R*)-10a,12a-dimethyl-1-((*R*)-6-methylheptan-2-yl)-1,2,3,3a,3b,4,5,5a,6,7,10,10a, 10b, 11,12,12a-hexadecahydrocyclopenta[5,6]naphtho[1,2-*f*]indazole-4,5-diol |
| la-3 | (2*R*,3b*R*,4*R*,5*R*,5a*S*,10a*R*,10b*S*)-1,1,10a-trimethyl-1,2,3,3b,4,5,5a,6,7,10,10a,10b,11,12-tetradecahydrocyclopenta[5,6]naphtho[1,2-*f*]indazole-2,4,5-triol |
| la-4 | (1*R*,3a*S*,3b*S*,4*R*,5*R*,5a*S*,10a*R*,10b*S*,12a*S*)-10a,12a-dimethyl-1-(prop-1-en-2-yl)-1,2,3,3a,3b,4,5,5a,6,7,10,10a,10b,11,12,12a-hexadecahydrocyclopenta[5,6]naphtho[1,2-*f*]indazole-4,5-diol |
| la-5 | (3a*S*,3b*R*,4*R*,5*R*,5a*S*,10a*R*,10b*S*,12a*S*,*E*)-1-ethylidene-10a,12a-dimethyl-1,2,3,3a,3b,4,5,5a,6,7,10,10a,10b,11,12,12a-hexadecahydrocyclopenta[5,6]naphtho[1,2-*f*]indazole-4,5-diol |
| la-6 | (3a*S*,3b*R*,4*R*,5*R*,5a*S*, 10a*R*, 10b*S*,12a*S*)-10a,12a-dimethyl-1-phenyl-3,3a,3b,4,5,5a,6,7,10,10a,10b,11,12,12a-tetradecahydrocyclopenta[5,6]naphtho[1,2-*f*]indazole-4,5-diol |
| la-7 | (1*S*,3a*S*,3b*R*,4*R*,5*R*,5a*S*,10a*R*, 10b*S*, 12a*S*)-1,10a, 12a-trimethyl-1,2,3,3a,3b,4,5,5a,6,7,10,10a,10b,11,12,12a-hexadecahydrocyclopenta[5,6]naphtho[1,2-*f*]indazole-1,4,5-triol |
| la-8 | (1*R*,3a*S*,3b*S*,4*R*,5*R*,5a*S*,10a*R*,10b*S*,12a*R*)-1-((2*S*,5*R*)-5-ethyl-3-hydroxy-6-methylheptan-2-yl)-10a, 12a-dimethyl-1,2,3,3a,3b,4,5,5a,6,7,10,10a,10b,11,12,12a-hexadecahydrocyclopenta[5,6]naphtho[1,2-*f*]indazole-4,5-diol |
| la-9 | (1*S*,3a*S*,3b*R*,4*R*,5*R*,5a*S*,10a*R*,10b*S*,12a*S*)-10a,12a-dimethyl-1-(thiazol-2-yl)-1,2,3,3a,3b,4,5,5a,6,7,10,10a, 10b, 11,12,12a-hexadecahydrocyclopenta[5,6]naphtho[1,2-*f*]indazole-1,4,S-triol |
| la-10 | (1*S*,3a*S*,3b*R*,4*R*,5*R*,5a*S*,10a*R*,10b*S*,12a*S*)-10a,12a-dimethyl-1-(pyridin-2-yl)-1,2,3,3a,3b,4,5,5a,6,7,10,10a, 10b, 11,12,12a-hexadecahydrocyclopenta[5,6]naphtho[1,2-*f*]indazole-1,4,5-triol |
| Ib-1 | (3a*S*,3b*R*,4*R*,5*R*,5a*S*,11a*R*,11b*S*,13a*S*)-11a,13a-dimethyl-1-methylene-2,3,3a,3b,4,5,5a,6,11,11a,11b,12,13,13a-tetradecahydro-1*H*-cyclopenta[5,6]naphtho[1,2-*g*]quinoxaline-4,5-diol |
| Ib-2 | (1*R*,3a*S*,3b*S*,4*R*,5*R*,5a*S*,11a*R*,11b*S*,13a*R*)-11a,13a-dimethyl-1-((*R*)-6-methylheptan-2-yl)-2,3,3a,3b,4,5,5a,6,11,11a,11b,12,13,13a-tetradecahydro-1*H* cyclopenta[5,6]naphtho[1,2-*g*]quinoxaline-4,5-diol |
| Ic-1 | (1*R*,3a*S*,3b*S*,4*R*,5*R*,5a*S*,11a*R*,11b*S*,13a*R*)-8,11a,13a-trimethyl-1-((*R*)-6-methylheptan-2-yl)-2,3,3a,3b,4,5,5a,6,11,11a,11b,12,13,13a-tetradecahydro-1*H* cyclopenta[5,6]naphtho[1,2-*g*]quinazoline-4,5-diol |
| Ic-2 | (1*R*,3a*S*,3b*S*,4*R*,5*R*,5a*S*,11a*R*,11b*S*,13a*R*)-8-amino-11a,13a-dimethyl-1-((*R*)-6-methylheptan-2-yl)-2,3,3a,3b,4,5,5a,6,11,11a,11b,12,13,13a-tetradecahydro-1*H-*cyclopenta[5,6]naphtho[1,2-*g*]quinazoline-4,5-diol |
| Ic-3 | (1*R*,3a*S*,3b*S*,4*R*,5*R*,5a*S*,11a*R*,11b*S*,13a*R*)-11a,13a-dimethyl-1-((*R*)-6-methylheptan-2-yl)-2,3,3a,3b,4,5,5a,6,11,11a,11b,12,13,13a-tetradecahydro-1*H-*cyclopenta[5,6]naphtho[1,2-*g*]quinazoline-4,5-diol |
| Io-4 | (3a*S*,3b*R*,4*R*,5*R*,5a*S*,11a*R*,11b*S*,13a*S*)-8,11a,13a-trimethyl-1 - methylene-2,3,3a,3b,4,5,5a,6,11,11a,11b,12,13,13a-tetradecahydro-1*H*-cyclopenta[5,6]naphtho[1,2-*g*]quinazoline-4,5-diol |
| Ic-5 | (3a*S*,3b*R*,4*R*,5*R*,5a*S*, 11a*R*,11b*S*, 13a*S*)-11a, 13a-dimethyl-1-methylene-2,3,3a,3b,4,5,5a,6,11,11a,11b,12,13,13a-tetradecahydro-1*H*-cyclopenta[5,6]naphtho[1,2-*g*]quinazoline-4,5-diol |
| Ic-6 | (3a*S*,3b*R*,4*R*,5*R*,5a*S*,11a*R*,11b*S*,13a*S*)-8-amino-11a, 13a-dimethyl-1-methylene-2,3,3a,3b,4,5,5a,6,11,11a, 11b,12,13,13a-tetradecahydro-1*H*-cyclopenta[5,6]naphtho[1,2-*g*]quinazoline-4,5-diol |
| Id-1 | (1*R*,3a*S*,3b*S*,4*R*,5*R*,5a*S*,10a*R*,10b*S*,12a*R*)-10a,12a-dimethyl-1-((*R*)-6-methylheptan-2-yl)-2,3,3a,3b,4,5,5a,6,10,10a,10b,11,12,12a-tetradecahydro-1*H-*cyclopenta[7,8]phenanthro[2,3-*d*][1,2,3]thiadiazole-4,5-diol |
| Ie-1 | (1*R*,3a*S*,3b*S*,4*R*,5*R*,5a*S*,10a*R*,10b*S*,12a*R*)-10a,12a-dimethyl-1-((*R*)-6-methylheptan-2-yl)-2,3,3a,3b,4,5,5a,6,10,10a,10b,11,12,12a-tetradecahydro-1*H-*cyclopenta[7,8]phenanthro[3,2-*d*]isoxazole-4,5-diol |
| If-1 | (3a*S*,3b*R*,4*R*,5*R*,5a*S*,10a*R*,10b*S*,12a*S*)-8,10a,12a-trimethyl-1-methylene-2,3,3a,3b,4,5,5a,6,10,10a, 10b, 11,12,12a-tetradecahydro-1*H*-cyclopenta[7,8]phenanthro[3,2-*d*]oxazole-4,5-diol |
| Ig-1 | (1*R*,3a*S*,3b*S*,4*R*,5*R*,5a*S*, 10a*R*, 10b*S*, 12a*R*)-8-amino-10a, 12a-dimethy)-1-((*R*)-6-methy)heptan-2-y))-2,3,3a,3b,4,5,5a,6,10,10a,10b,11,12,12a-tetradecahydro-1*H-*cyclopenta[7,8]phenanthro[2,3-*d*]thiazole-4,5-diol |
| 1h-1 | (5a*S*,5b*R*,6*R*,7*R*,7a*S*,12a*R*,12b*S*,14a*S*)-2-chloro-12a,14a-dimethyl-5,5a,5b,6,7,7a,8,9,12,12a, 12b, 13,14,14a-tetradecahydropyrido[2",3":3',4']cyclopenta[1',2':5,6]naphtho[1,2-*f*]indazole-6,7 -diol |
| 1h-2 | (5a*S*,5b*R*,6*R*,7*R*,7a*S*, 12a*R*, 12b*S*, 14a*S*)-12a, 14a-dimethyl-5,5a,5b,6,7,7a,8,9,12,12a,12b,13,14,14a-tetradecahydropyrido[2",3":3',4']cyclopenta[1',2':5,6]naphtho[1,2-*f*]indazole-6,7-diol |

The following Synthetic Examples, which are directed to the synthesis of the compounds of the invention, in as far as they relate to compounds la-1, la-2, la-3, la-4, la-7 and la-8; and the following Biological Examples, which are directed to representative biological assays for the compounds of the invention, are provided as a guide to assist in the practice of the invention, and are not intended as a limitation on the scope of the invention.

### SYNTHETIC EXAMPLE 1

### Synthesis of (3aS,3bR,4R,5R,5aS, 10aR, 10bS, 12aR)-10a, 12a-dimethyl-3,3a, 3b,4,5,5a,6,7,10,10a, 10b, 11,12,12a-tetradecahydrocyclopenta[5,6]naphtho[1,2-f]indazole-4,5-diol (Compound la-1)

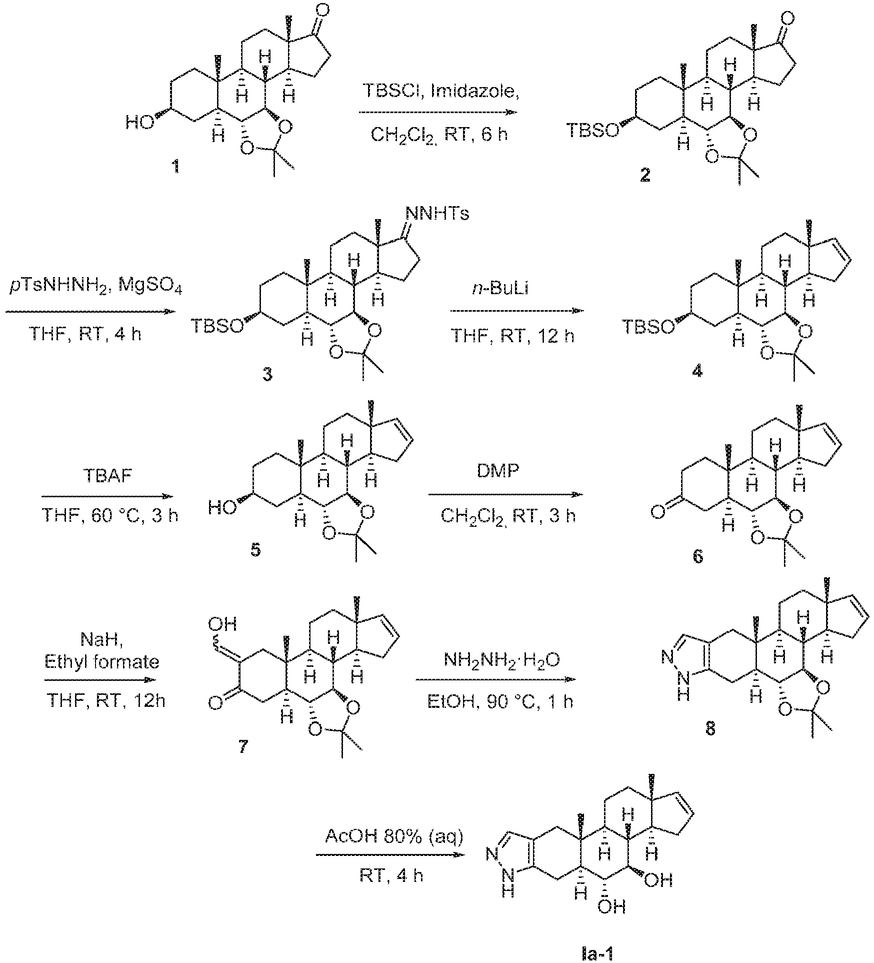

A. Using General Procedure B as described above with (2*S*,4a*R*,4b*S*,6a*S*,9a*S*,9b*R*,9c*R*,12a*R*,12b*S*)-2-hydroxy-4a,6a,11,11-tetramethylhexadecahydro-7*H-*cyclopenta[1,2]phenanthro[9,10-*d*][1,3]dioxol-7-one (Compound 1, as prepared in U.S. Pat. No. 6,046,185, 15 g, 41.43 mmol, 1 eq), TBSCI (6.24 g, 41.43 mmol, 1 eq), imidazole (8.46 g, 124.31 mmol, 3 eq) and dichloromethane (150 mL) gave the desired silyl ether (2*S*,4a*R*,4b*S*,6a*S*,9a*S*,9b*R*,9c*R*,12a*R*,12b*S*)-2-((*tert*-butyldimethylsilyl)oxy)-4a,6a, 11,11-tetramethylhexadecahydro-7*H*-cyclopenta[1,2]phenanthro[9,10-*d*][1,3]dioxol-7-one (Compound 2, 12 g, 61 %) as a white solid after purification by flash column chromatography (230-400 mesh silica gel, eluted with 0-10 % pet ether / ethyl acetate).
B. To a solution of Compound 2 (12 g, 25.19 mmol, 1 eq) in THF (100 mL) taken in a 500 mL three-necked RB flask was added magnesium sulphate (7.57 g, 62.98 mmol, 2.5 eq) and *p*-toluenesulfonyl hydrazide (7.03 g, 37.78 mmol,1.5 eq). Reaction mass was stirred with the help of a magnetic stirrer and the reaction was carried out under a nitrogen atmosphere. The reaction mass was stirred for 4 h at room temperature. Completion of the reaction was monitored by TLC analysis. The reaction mass was cooled and quenched with ice cold water (100 mL) then extracted with ethyl acetate (2 x 100 mL). The organic phase was washed with brine solution (2 x 100 mL), dried over anhydrous sodium sulphate and concentrated on a rotary evaporator at 45 °C to get a crude colorless gum. It was further purified by column chromatography (230-400 mesh silica gel, eluted with 0-40 % pet ether / ethyl acetate) to afford *N*'-((2*S*,4a*R*,4b*S*,6a*S*,9a*S*,9b*R*,9c*R*,12a*R*,12b*S*,*Z*)-2-((*tert*butyldimethylsilyl)oxy)-4a,6a,11,11 -tetramethylhexadecahydro-7*H-*cyclopenta[1,2]phenanthro[9,10-*d*][1,3]dioxol-7-ylidene)-4-methylbenzenesulfonohydrazide (Compound 3, 12 g, 74 %) as a white solid.
C. To a solution of Compound 3 (12 g, 18.63 mmol, 1 eq) in THF (100 mL) taken in a 500 mL three-necked RB flask was added *n*-butyl lithium (2 M in hexane, 93.2 mL, 186.3 mmol, 10 eq) slowly dropwise at -78 °C. Reaction mass was externally cooled with the help of dry ice bath. Reaction mass was stirred with the help of a magnetic stirrer and the reaction was carried out under a nitrogen atmosphere. The reaction mass was slowly warmed to room temperature and stirred for 12 h. Completion of the reaction was monitored by TLC analysis. The reaction mass was cooled to -78 °C quenched with saturated aqueous NH₄Cl solution (150 mL) and extracted with ethyl acetate (2 x 200 mL). The organic phase was washed with brine solution (2 x 200 mL), dried over anhydrous sodium sulphate and concentrated on a rotary evaporator at 45 °C to get a crude colorless gum. It was further purified by column chromatography (230-400 mesh silica gel, eluted with 0-30 % pet ether / ethyl acetate) to afford *tert*-butyldimethyl(((2*S*,4a*R*,4b*S*,6a*R*,9a*S*,9b*R*,9c*R*,12a*R*,12b*S*)-4a,6a,11,11-tetramethyl-2,3,4,4a,4b,5,6,6a,9,9a,9b,9c,12a,12b-tetradecahydro-1H-cyclopenta[1,2]phenanthro[9,10-d][1,3]dioxol-2-yl)oxy)silane (Compound 4, 5 g, 58 %) as a white solid.
D. Using General Procedure L as described above with Compound 4 (5 g, 10.86 mmol,1 eq), TBAF solution (1M in THF, 21.7 mL, 21.72 mmol, 2 eq) and THF (50 mL) gave the desired alcohol (2*S*,4a*R*,4b*S*,6a*R*,9a*S*,9b*R*,9c*R*,12a*R*,12b*S*)-4a,6a,11,11-tetramethyl-2,3,4,4a,4b,5,6,6a,9,9a,9b,9c,12a,12b-tetradecahydro-1*H-*cyclopenta[1,2]phenanthro[9,10-*d*][1,3]dioxol-2-ol (Compound 5, 3.2 g, 85 %) as a pale yellow solid after purification by flash column chromatography (230-400 mesh silica gel, eluted with 0-60 % pet ether / ethyl acetate).
E. Using General Procedure I with Compound 5 (3.2 g, 9.24 mmol, 1 eq), Dess-Martin periodinane ( 4.74 g, 11.09 mmol, 1.2 eq) and dichloromethane (32 mL) gave the desired ketone (4a*R*,4b*S*,6a*R*,9a*S*,9b*R*,9c*R*,12a*R*,12b*S*)-4a,6a,11,11-tetramethyl-1,3,4,4a,4b,5,6,6a,9,9a,9b,9c,12a,12b-tetradecahydro-2*H-*cyclopenta[1,2]phenanthro[9,10-*d*][1,3]dioxol-2-one (Compound 6, 2.3 g, 72 %) as a white solid after purification by flash column chromatography (230-400 mesh silica gel, eluted with 0-30 % pet ether / ethyl acetate).
F. Using General Procedure J with Compound 6 (4 g, 6.68 mmol, 1 eq), sodium hydride (60 % in paraffin oil, 1.1 g, 26.74 mmol, 4 eq), ethyl formate (3.26 mL, 40.11 mmol, 6 eq) and THF (30 mL) gave the desired ketone (4a*R*,4b*S*,6a*R*,9a*S*,9b*R*,9c*R*,12a*R*,12b*S*,*Z*)-3-(hydroxymethylene)-4a,6a,11,11-tetramethyl-1,3,4,4a,4b,5,6,6a,9,9a,9b,9c,12a,12b-tetradecahydro-2*H-*cyclopenta[1,2]phenanthro[9,10-*d*][1,3]dioxol-2-one (Compound 7, 2 g, 81 %) as a yellow solid after purification by flash column chromatography (230-400 mesh silica gel, eluted with 0-15 % pet ether / ethyl acetate).
G. Using General Procedure K with Compound 7 (2 g, 5.37 mmol,1 eq), hydrazine hydrate (0.4 g, 8.06 mmol,1.5 eq) and ethanol (20 mL) gave the desired pyrazole (3a*S*,3b*R*,3c*R*,6a*R*,6b*S*, 11a*R*,11 b*S*, 13a*R*)-5,5,11 a, 13a-tetramethyl-3,3a,3b,3c,6a,6b,7,8,11,11a,11b,12,13,13a-tetradecahydrocyclopenta[5,6][1,3]dioxolo[4',5':3,4]naphtho[1,2-*f*]indazole (Compound 8, 1.8 g, 92 %) as a pale yellow solid after purification by flash column chromatography (230-400 mesh silica gel, eluted with 0-60 % pet ether / ethyl acetate).
H. Using General Procedure E with Compound 8 (1.8 g, 4.89 mmol, 1 eq), and acetic acid (80 % aqueous solution, 20 mL) gave the desired diol (3a*S*,3b*R*,4*R*,5*R,*5a*S*,10a*R*,10b*S*,12a*R*)-10a,12a-dimethyl-3,3a, 3b,4,5,5a,6,7,10,10a, 10b, 11,12,12a-tetradecahydrocyclopenta[5,6]naphtho[1,2-*f*]indazole-4,5-diol (Compound la-1, 1.5 g, 89 %) as a pale yellow solid after purification by flash column chromatography (230-400 mesh silica gel, eluted with 0-10 % dichloromethane / methanol).
   LCMS: (Method 1a) MS m/z: 329.2 (M+1), t_{R}: 2.351 min, Purity: 99.87 % (ELSD);
   ¹H-NMR (400 MHz, DMSO-d6): δ 12.21 (s, 1H), 7.21 (s, 1H), 5.78 (d, *J*= 6.0 Hz, 1H), 5.71 (d, *J*= 5.6 Hz, 1H), 4.55 (s, 1H), 4.25 (d, *J*= 5.2 Hz, 1H), 4.09 (d, *J*= 5.2 Hz, 1H), 3.17-3.09 (m, 2H), 2.99-2.92 (m, 2H), 2.44-2.32 (m, 1H), 2.23-2.02 (m, 3H), 1.75-1.72 (m, 1H), 1.62-1.23 (m, 5H), 1.02-1.01 (m, 1H), 0.74 (s, 3H), 0.71 (s, 3H).

### SYNTHETIC EXAMPLE 2

### Synthesis of (1R,3aS,3bS,4R,5R,5aS,10aR,12aR)-10a,12a-dimethyl-1-((R)-6-methylheptan-2-yl)-1,2,3,3a,3b,4,5,5a,6,7,10,10a, 10b, 11,12,12a-hexadecahydrocyclopenta[5,6]naphtho[1,2-f]indazole-4,5-diol (Compound la-2)

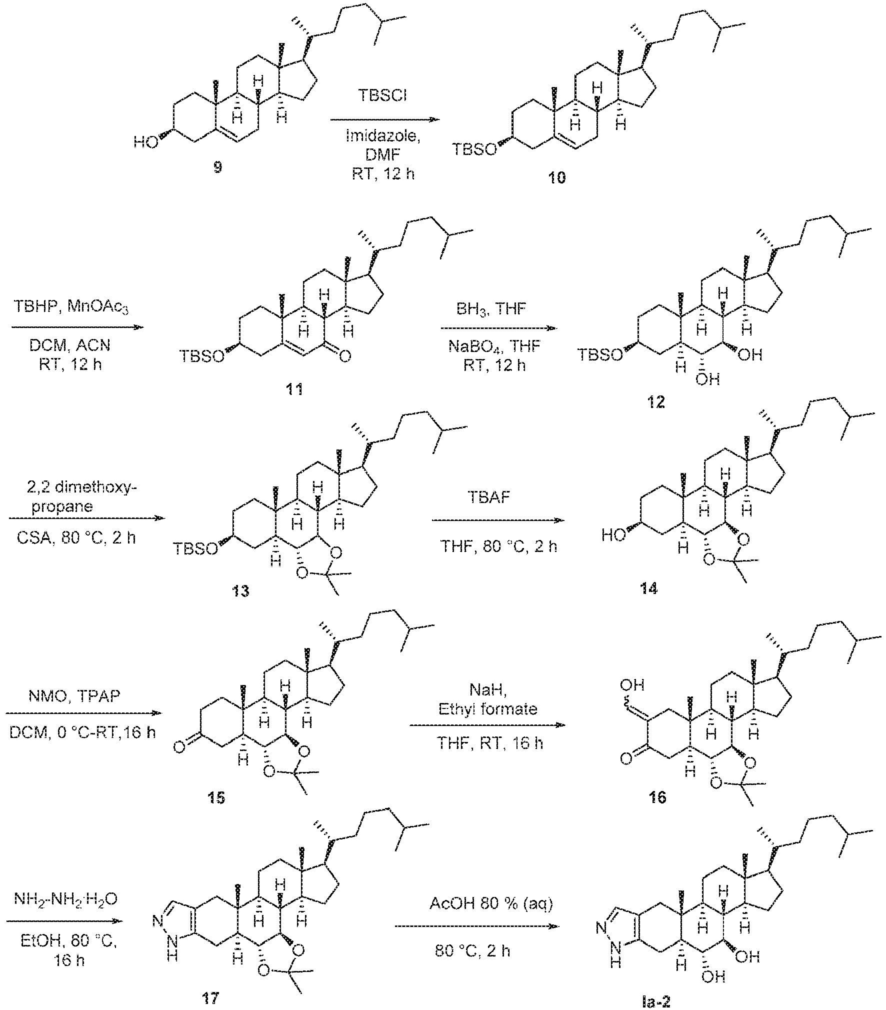

A. Using General Procedure B with cholesterol (Compound 9, 25 g, 64 mmol, 1 eq), imidazole (6.61 g, 97 mmol, 1.5 eq), TBSCl (10 g, 71 mmol, 1.1 eq) and DMF (200 mL) gave the desired silyl ether *tert*-butyl(((3*S*,8*S*,9*S*,10*R*,13*R*,14*S*,17*R*)-10,13-dimethyl-17-((*R*)-6-methylheptan-2-yi)-2,3,4,7,8,9,10,11,12,13,14,15,16,17-tetradecahydro-1*H-*cyclopenta[a]phenanthren-3-yl)oxy)dimethylsilane (Compound 10, 27 g, 84 %) as an off-white solid after purification by flash column chromatography (60-120 mesh silica gel, eluted with 0-40 % pet ether / ethyl acetate).
B. Using General Procedure C with Compound 10 (20 g, 40 mmol, 1 eq), imidazole (6.61 g, 97 mmol, 1.5 eq), TBHP in decane (36 mL, 20 mol, 5 eq), manganese (III) acetate dihydrate (1.0 g, 4 mmol, 0.1 eq), molecular sieves (4 Å, 6 g), acetonitrile (100 mL) and dichloromethane (100 mL) gave the desired enone (3*S*,9*S*,10*R*,13*R*,14*S*,17*R*)-3-((*tert*-butyldimethylsilyl)oxy)-10,13-dimethyl-17-((*R*)-6-methylheptan-2-yl)-1,2,3,4,8,9,10,11,12,13,14,15,16,17-tetradecahydro-7*H-*cyclopenta[a]phenanthren-7-one (Compound 11, 16 g, 78 %) as an off-white solid after purification by flash column chromatography (230-400 mesh silica gel, eluted at 0-50 % pet ether / ethyl acetate).
C. Using General Procedure D with Compound 11 (16 g, 31.1 mmol, 1 eq), Borane in THF (1 M, 5.3 g, 62 ml, 62 mmol, 2 eq), sodium perborate tetrahydrate (14 g, 93 mmol, 3 eq) and THF (150 mL) gave the desired enone (3*S*,5*S*,6*R*,7*R*,8*S*,9*S*, 10*R*, 13*R*, 14*S*, 17*R*)-3-((tert-butyldimethylsilyl)oxy)-10,13-dimethyl-17-((*R*)-6-methylheptan-2-yl)hexadecahydro-1*H*-cyclopenta[*a*]phenanthrene-6,7-diol (Compound 12, 14 g, 84 %) as an off-white solid after purification by flash column chromatography (230-400 mesh silica gel, eluted with 0-50 % pet ether / ethyl acetate).
D. Using General Procedure F with Compound 12 (70.0 g, 130.86 mmol), camphorsulfonic acid (3.04 g, 13.09 mmol) and 2,2-dimethoxypropane (642 mL, 5234.4 mmol), followed by purification by column chromatography on silica gel (60-120 mesh, 10-15 % pet ether / ethyl acetate) to afford *tert-*butyldimethyl(((2*S*,4a*R*,4b*S*,6a*R*,7*R*,9a*S*,9b*S*,12a*R*,12b*S*)-4a,6a,11,11-tetramethyl-7-((*R*)-6-methylheptan-2-yl)hexadecahydro-1*H*-cyclopenta[1,2]phenanthro[9,10-d][1,3]dioxol-2-yl)oxy)silane (Compound 13, 56.0 g, 75 %) as a white solid.
E. Following the General Procedure L with Compound 13 (56.0 g, 97.39 mmol), TBAF (1 M in THF, 195 mL, 195 mmol) and THF (560 mL), gave the desired alcohol (2*S*,4a*R*,4b*S*,6a*R*,7*R*,9a*S*,9b*S*,9c*R*,12a*R*,12b*S*)-4a,6a,11,11-tetramethyl-7-((*R*)-6-methylheptan-2-yl)hexadecahydro-1*H-*cyclopenta[1,2]phenanthro[9,10-d][1,3]dioxol-2-ol (Compound 14, 27.0 g, 60 %) as a white solid after purification by column chromatography (60-120 mesh silica gel, eluted with 30-50 % pet ether / ethyl acetate).
F. Using General Procedure H Compound 14 (27.0 g, 58.60 mmol), NMO (8.92 g, 76.18 mmol), 4 Å molecular sieves (27.0 g), TPAP (6.18 g, 17.58 mmol) and CH₂Cl₂ (270 mL) gave the desired ketone (4a*R*,4b*S*,6a*R*,7*R*,9a*S*,9b*S*,9c*R*,12a*R*,12b*S*)-4a,6a,11,11-tetramethyl-7-((*R*)-6-methylheptan-2-yl)hexadecahydro-2*H*- cyclopenta[1,2]phenanthro[9,10-*d*][1,3]dioxol-2-one (Compound 15, 20.0 g, 66 %) as an off-white solid after purification by column chromatography (60-120 mesh silica gel, eluted with 10-15 % pet ether / ethyl acetate).
G. Using General Procedure J with Compound 15 (20.0 g, 43.60 mmol), NaH (60 % in paraffin oil, 8.72 g, 217.99 mmol), ethyl formate (7.04 mL, 87.20 mmol) and THF (200 mL), gave the desired ketone (4a*R*,4b*S,*6a*R*,7*R*,9a*S*,9b*S*,9c*R*,12a*R*)-3-(hydroxymethylene)-4a,6a,11,11-tetramethyl-7-((*R*)-6-methylheptan-2-yl)hexadecahydro-2*H*-cyclopenta[1,2]phenanthro[9,10-d][1,3]dioxol-2-one (Compound 16, 15 g, 71 %) as a white solid which was used in the next step without purification.
H. Using General Procedure K with Compound 16 (12.0 g, 24.65 mmol) and hydrazine hydrate (2.42 mL, 49.31 mmol) in EtOH (120 mL) gave the desired pyrazole (1*R*,3a*S*,3b*S*,3c*R*,6a*R*,6b*S*,11a*R*,11 b*S*,13a*R*)-5,5, 11a, 13a-tetramethyl-1-((*R*)-6-methylheptan-2-yl)-1,2,3,3a,3b,3c,6a,6b,7,8,11,11a,11b,12,13,13a-hexadecahydrocyclopenta[5,6][1,3]dioxolo[4',5':3,4]naphtho[1,2-f]indazole (Compound 17, 6.0 g, 52 %) as an off white solid after purification by column chromatography (230-400 mesh silica gel, eluted with 50-60 % pet ether / ethyl acetate).
I. Using General Procedure E with Compound 17 (6.0 g, 12.43 mmol) in AcOH (80 %, 60 mL) gave the desired dialcohol (1*R*,3a*S*,3b*S*,4*R*,5*R*,5a*S*,10a*R*,10b*S*,12a*R*)-10a,12a-dimethyl-1-((*R*)-6-methylheptan-2-yl)-1,2,3,3a,3b,4,5,5a,6,7,10,10a,10b,11,12,12a-hexadecahydrocyclopenta[5,6]naphtho[1,2-*f*]indazole-4,5-diol (Compound la-2, 6.0 g) a yellow gummy solid.
   LCMS: (Method 1f) MS m/z: 443.5 (M+1), t_{R}: 4.203 min, Purity: 95.44 % (ELSD).

### SYNTHETIC EXAMPLE 3

### Synthesis of (2R,3bR,4R,5R,5aS,10aR,10bS)-1,1,10a-trimethyl-1,2,3,3b,4,5,5a,6,7,10,10a,10b,11,12-tetradecahydrocyclopenta[5,6]naphtho[1,2-f]indazole-2,4,5-triol (Compound la-3)

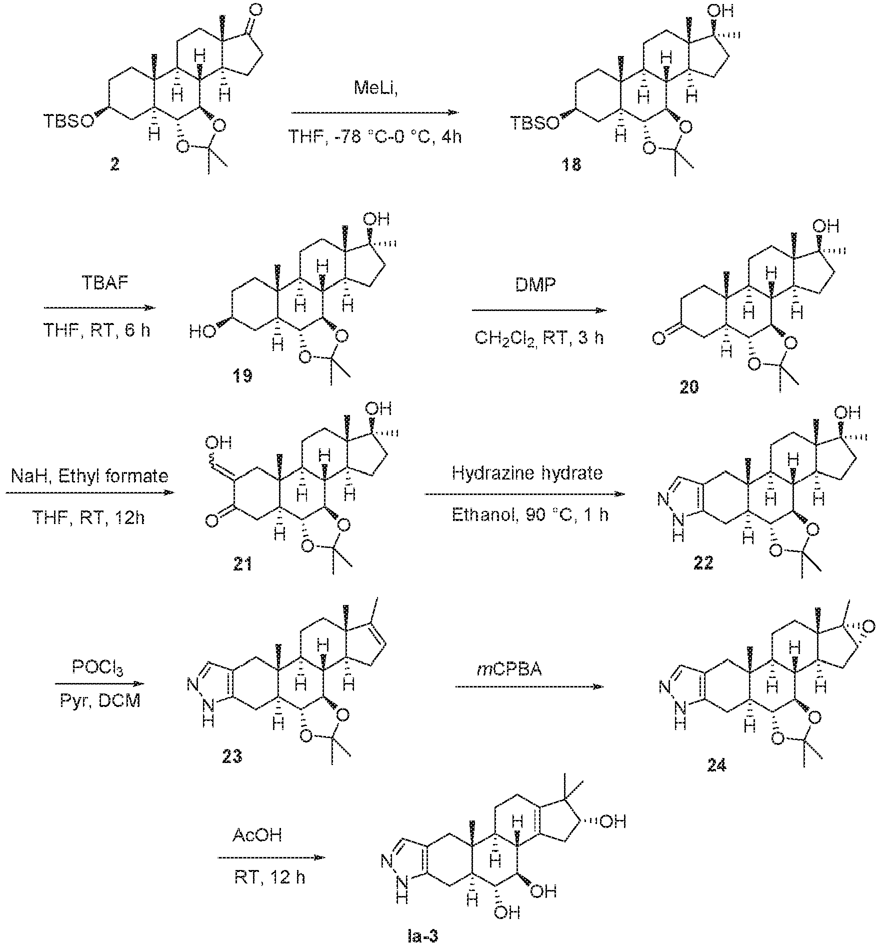

A. To a stirred solution of Compound 2 (from Example 1, 10.0 g, 21.0 mmol) in THF (100 mL) was added methyllithium (1.6 M in ether, 39.33 mL, 62.92 mmol) dropwise at -78 °C. The reaction mixture was stirred at 0 °C for 4 hours. The reaction mixture was diluted with saturated aqueous solution of Na₂SO₄ (50 g dissolved in 100 mL of water) and the aqueous layer was extracted with EtOAc (2 x 100 mL) then washed consecutively with water (1 × 100 mL) and brine (1 × 100 mL). The organic layer was dried over Na₂SO₄, filtered and concentrated. The residue was purified by column chromatography on silica gel (230-400 mesh, 30-40 % EtOAc / pet ether) to afford (2*S*,4a*R*,4b*S*,6a*S*,7*S*,9a*S*,9b*R*,9c*R*,12a*R*,12b*S*)-2-((*tert*butyldimethylsilyl)oxy)-4a,6a,7,11,11-pentamethylhexadecahydro-1*H-*cyclopenta[1,2]phenanthro[9,10-*d*][1,3]dioxol-7-ol (Compound 18, 8.0 g, 77 %) as a white solid.
B. Following the General Procedure L with Compound 18 (8.0 g, 16.23 mmol), TBAF (1 M in THF, 32.47 mL, 32.47mmol) and THF (80 mL) afforded (2*S*,4a*R*,4b*S*,6a*S*,7*S*,9a*S*,9b*R*,9c*R*,12a*R*,12b*S*)-4a,6a,7,11,11-pentamethylhexadecahydro-1*H*-cyclopenta[1,2]phenanthro[9,10-*d*][1,3]dioxole-2,7-diol (Compound 19, 4.9 g, 79 %) as a white solid following purification by column chromatography on silica gel (230-400 mesh, 70-80 % EtOAc / pet ether).
C. Following the General Procedure I with Compound 19 (4.9 g, 12.94 mmol), Dess-Martin periodane (8.24 g, 19.42 mmol) and DCM (50 mL) afforded (4a*R*,4b*S*,6a*S*,7*S*,9a*S*,9b*R*,9c*R*,12a*R*,12b*S*)-7-hydroxy-4a,6a,7,11,11-pentamethylhexadecahydro-2*H*-cyclopenta[1,2]phenanthro[9,10-*d*][1,3]dioxol-2-one (Compound 20, 3.3 g, 67 %) as a white solid following purification by column chromatography on silica gel (230-400 mesh, 20-30 % EtOAc I pet ether).
D. Following the General Procedure J with Compound 20 (3.3 g, 8.76 mmol), NaH (60 % in paraffin oil, 1.4 g, 35.06 mmol), ethyl formate (4.25 mL, 52.58 mmol) and THF (30 mL) afforded (4a*R*,4b*S*,6a*S*,7*S*,9a*S*,9b*R*,9c*R*,12a*R*,12b*S*)-7-hydroxy-3-(hydroxymethylene)-4a,6a,7,11,11-pentamethylhexadecahydro-2/7-cyclopenta[1,2]phenanthro[9,10-*d*][1,3]dioxol-2-one (Compound 21, 3.1 g, 88 %) as a white solid following purification by column chromatography on silica gel (230-400 mesh, 20-30 % EtOAc / pet ether).
E. Following the General Procedure K with Compound 21 (3.1 g, 7.66 mmol), hydrazine hydrate (0.56 mL, 11.49 mmol) and ethanol (30 mL) afforded (1*S*,3a*S*,3b*R*,3c*R*,6a*R*,6b*S*,11a*R*,11b*S*,13a*S*)-1,5,5,11a,13a-pentamethyl-1,2,3,3a,3b,3c,6a,6b,7,8,11,11a,11b, 12,13,13a-hexadecahydrocyclopenta[5,6][1,3] dioxolo[4',5':3,4]naphtho[1,2-*f*]indazol-1-ol (Compound 22, 2.8 g, 91 %) as a white solid following purification by column chromatography on silica gel (230-400 mesh, 0-5 % MeOH / CH₂Cl₂).
F. To a stirred solution of Compound 22 (2.8 g, 6.99 mmol) in pyridine:DCM (1:1, 30 mL) was added POCl₃(1.96 mL, 20.97 mmol) dropwise at 0 °C. The resultant solution was stirred at room temperature for 12 hours. The reaction mixture was evaporated under reduced pressure and residue was diluted with saturated solution of NaHCO₃ (15 g dissolved in 30 mL water). The aqueous layer was extracted with EtOAc (2 x 30 mL) which was then washed consecutively with water (1 x 30 mL) and brine (1 x 30 mL). The organic layer was dried over Na₂SO₄, filtered and concentrated. The residue was purified by column chromatography on silica gel (230-400 mesh, 0-5 % MeOH / CH₂Cl₂) to afford (3a*S*,3b*R*,3c*R*,6a*R*,6b*S*,11a*R*,11b*S*,13a*S*)-1,5,5,11a,13a-pentamethyl-3,3a,3b,3c,6a,6b,7,8,11,11a,11b,12,13,13a-tetradecahydrocyclopenta[5,6][1,3]dioxolo[4',5':3,4]naphtho[1,2-*f*]indazole solid (Compound 23, 0.85 g, 32 %) as an off white solid.
G. To a stirred solution of Compound 23 (0.85 g, 2.22 mmol) in CH₂Cl₂ (10 mL) was added *meta*-chloroperoxybenzoic acid (1.15 g, 6.67 mmol) portion wise at 0 °C. The resultant mixture was stirred at room temperature for 2 hours. The reaction mixture was evaporated under reduced pressure and residue was diluted with saturated solution of NaHCO₃ (5 g dissolved in 10 mL water). The aqueous was extracted with CH₂Cl₂ (2 x 10 mL) then washed consecutively with water (1 x 10 mL) and brine (1 x 10 mL). The organic layer was dried over Na₂SO₄, filtered and concentrated. The residue was purified by column chromatography on silica gel (230-400 mesh, 5-10 % MeOH / CH₂Cl₂) to afford (3a*R*,3b*S*,8*R*,8b*S*,10a*S*,10b*S*,11a*R*,12a*S*,12b*R*,12c*R*)-2,2,8a,10a,10b-pentamethyl-3a,3b,4,5,8,8a,8b,9,10,10a,10b,11a,12,12a,12b,12c-hexadecahydro-[1,3]dioxolo[4',5':3,4]oxireno[2",3":3',4']cyclopenta[1',2':5,6]naphtho[1,2-*f*]indazole (Compound 24, 0.5 g, 57 %) as a white solid.
H. Following the General Procedure E with Compound 24 (0.5 g, 1.25 mmol) and AcOH (80 %, 5 mL) afforded (2*R*,3b*R*,4*R*,5*R*,5a*S*,10a*R*,10b*S*)-1,1,10a-trimethyl-1,2,3,3b,4,5,5a,6,7,10,10a,10b,11,12-tetradecahydrocyclopenta[5,6]naphtho[1,2-*f*]indazole-2,4,5-triol (Compound la-3, 0.23 g, 51 %) as a white solid following purification by column chromatography on silica gel (230-400 mesh, 10-20 % MeOH / CH₂Cl₂).
   LCMS: (Method 1a) MS m/z: 359.2 (M+1), t_{R}: 2.026 min, Purity: 85.83 % (ELSD).

### SYNTHETIC EXAMPLE 4

### Synthesis of (1R,3aS,3bS,4R,5R,5aS,10aR,10bS,12aS)-10a,12a-dimethyl-1 -(prop-1-en-2-yl)-1,2,3,3a,3b,4,5,5a,6,7,10,10a,10b,11,12,12a-hexadecahydrocyclopenta[5,6]naphtho[1,2-f]indazole-4,5-diol (Compound la-4)

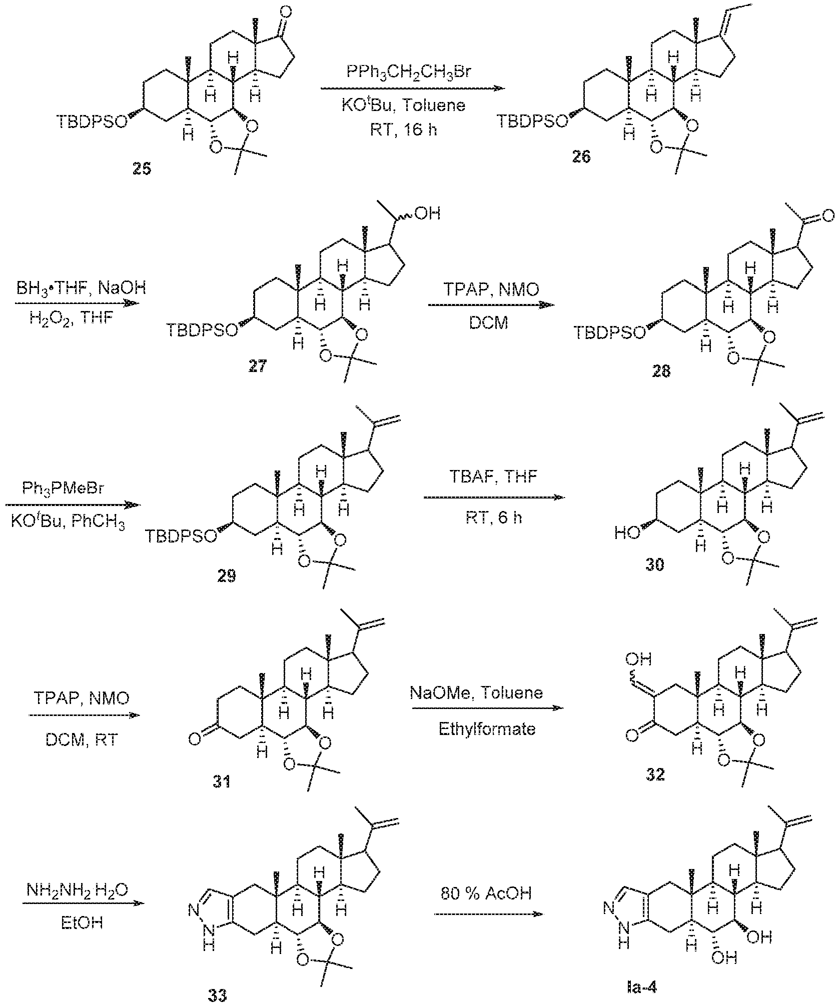

A. Following the General Procedure G with (2*S*,4a*R*,4b*S*,6a*S*,9a*S*,9b*R*,9c*R*,12a*R*,12b*S*)-2-((*tert*-butyldiphenylsilyl)oxy)-4a,6a,11,11-tetramethylhexadecahydro-7*H*-cyclopenta[1,2]phenanthro[9,10-*d*][1,3]dioxol-7-one (Compound 25, as prepared in U.S. Pat. No. 9,765,085, 5.0 g, 8.32 mmol), ethyltriphenylphosphonium bromide (15.44 g, 41.60 mmol), potassium-*tert-*butoxide (4.66 g, 41.60 mmol) and toluene (50 mL) gave the desired alkene *tert-*butyl(((2S,4a*R*,4b*S*,6a*S*,9a*S*,9b*R*,9c*R*,12a*R*,12b*S*,*E*)-7-ethylidene-4a,6a,11,11-tetramethylhexadecahydro-1*H*-cyclopenta[1,2]phenanthro[9,10-*d*][1,3]dioxol-2-yl)oxy)diphenylsilane (Compound 26, 4.0 g, 79 %) as a yellow solid after purification by column chromatography on silica gel (230-400 mesh, 0-20 % pet ether / ethyl acetate).
B. To a stirred solution of Compound 26 (4.0 g, 6.53 mmol) in THF (40 mL) at 0 °C was added borane in THF (1 M, 13.05 mL, 13.05 mmol) dropwise. The reaction mixture was stirred at room temperature for 1 hour. The mixture was cooled to 0 °C and added sodium hydroxide solution (10 %, aqueous, 45.94 mL) followed by hydrogen peroxide (30 %, 30.63 mL) dropwise. The resultant solution was stirred at room temperature for 2 hours. The aqueous was extracted with EtOAc (2 x 40 mL) and washed consecutively with water (1 x 40 mL) and brine (1 x 40 mL). The organic layer was dried over Na₂SO₄, filtered and concentrated. The crude was purified by column chromatography on silica gel (230-400 mesh, 0-30 % pet ether / ethyl acetate) to afford 1-((2*S*,4a*R*,4b*S*,6a*S*,9a*S*,9b*S*,9c*R*,12a*R*,12b*S*)-2-((*tert*-butyldiphenylsilyl)oxy)-4a,6a,11,1 1-tetramethyl hexadecahydro-1*H*-cyclopenta[1,2]phenanthro[9,10-*d*][1,3]dioxol-7-yl)ethan-1-ol (Compound 27, 3.3 g, 80 %) as an off-white solid.
C. Using General Procedure H with Compound 27 (3.0 g, 4.75 mmol), NMO (1.29 g, 11.01 mmol), 4 Å molecular sieves (3.0 g) and TPAP (0.17 g, 0.48 mmol) in CH₂Cl₂ (30 mL) gave the desired ketone 1-((2*S*,4a*R*,4b*S*,6a*S*,9a*S*,9b*R*,9c*R*,12a*R*,12b*S*)-2-((*tert*-butyldiphenylsilyl)oxy)-4a,6a,11,11-tetramethylhexadecahydro-1*H*-cyclopenta[1,2]phenanthro[9,10-d][1,3]dioxol-7-yl)ethan-1-one (Compound 28, 2.5 g, 36 %) as an off-white solid after purification by column chromatography (230-400 mesh silica gel, eluted with 15-20 % pet ether / ethyl acetate).
D. Following the General Procedure G with Compound 28 (2.5 g, 3.97 mmol), methyltriphenylphosphonium bromide (7.10 g, 19.87 mmol), potassium-*tert-*butoxide (2.23 g, 19.87 mmol) and toluene (30 mL) gave the desired alkene *tert-*butyldiphenyl(((2*S*,4a*R*,4b*S*,6a*S*,9a*S*,9b*S*,9c*R*,12a*R*,12b*S*)-4a,6a,11,11-tetramethyl-7-(prop-1-en-2-yl)hexadecahydro-1*H*-cyclopenta[1,2]phenanthro[9,10-*d*][1,3]dioxol-2-yl)oxy)silane (Compound 29, 2.2 g, 88 %) as a pale yellow solid after purification by column chromatography on silica gel (230-400 mesh, 0-10 % pet ether / ethyl acetate).
E. Following the General Procedure L with Compound 29 (2.2 g, 3.51 mmol) and TBAF (1 M in THF, 7.02 mL, 7.02 mmol) in THF (20 mL), gave the desired alcohol (2*S*,4a*R*,4b*S*,6a*S*,9a*S*,9b*S*,9c*R*,12a*R*,12b*S*)-4a,6a,11,11-tetramethyl-7-(prop-1-en-2-yl)hexadecahydro-1*H*-cyclopenta[1,2]phenanthro[9,10-*d*][1,3]dioxol-2-ol (Compound 30, 1.0 g, 73 %) as a white solid after purification by column chromatography (230-400 mesh silica gel, eluted with 0-30 % pet ether / ethyl acetate).
F. Using General Procedure H with Compound 30 (1.0 g, 2.57 mmol), NMO (0.69 g, 5.15 mmol), 4 Å molecular sieves (1.0 g) and TPAP (0.09 g, 0.26 mmol) in CH₂Cl₂ (10 mL) gave the desired ketone (4a*R*,4b*S*,6a*S*,9a*S*,9b*S*,9c*R*,12a*R*,12b*S*)-4a,6a,11,11-tetramethyl-7-(prop-1-en-2-yl)hexadecahydro-2*H-*cyclopenta[1,2]phenanthro[9,10-*d*][1,3]dioxol-2-one (Compound 31, 0.90 g, 91 %) as an off-white solid after purification by column chromatography (230-400 mesh silica gel, eluted with 15-25 % pet ether / ethyl acetate).
G. To a stirred solution of Compound 31 (0.90 g, 2.33 mmol) in toluene (10 mL) at 0 °C were added sodium methoxide solution (25 % wt. in MeOH, 1.51 mL, 6.60 mmol) and ethyl formate (0.94 mL, 11.65 mmol) dropwise. The resultant solution was stirred at room temperature for 16 hours. The mixture was evaporated under reduced pressure and the residue was diluted with ice cold water (1 x 10 mL). The aqueous was extracted with EtOAc (2 x 10 mL) and washed with brine (1 x 10 mL). The organic layer was dried over Na₂SO₄, filtered and concentrated to afford (4a*R*,4b*S*,6a*S*,9a*S*,9b*S*,9c*R*,12a*R*,12b*S*)-3-(hydroxymethylene)-4a,6a,11,11-tetramethyl-7-(prop-1-en-2-yl)hexadecahydro-2*H*-cyclopenta[1,2]phenanthro[9,10-d][1,3]dioxol-2-one (Compound 32, 0.9 g, 93 %) as a brown gummy solid which was used in the next step without purification.
H. Using General Procedure K with Compound 32 (0.9 g, 2.17 mmol) and hydrazine hydrate (0.53 mL, 10.86 mmol) in EtOH (10 mL) gave the desired pyrazole (3a*S*,3b*S*,3c*R*,6a*R*,6b*S*,11a*R*,11b*S*,13a*S*)-5,5,11a, 13a-tetramethyl-1 -(prop- 1-en-2-yl)-1,2,3,3a,3b,3c,6a,6b,7,8,11,11a,11b,12,13,13a-hexadecahydrocyclopenta[5,6][1,3]dioxolo[4',5':3,4]naphtho[1,2-*f*]indazole (Compound 33, 0.8 g, 90 %) as an off-white solid after purification by column chromatography on silica gel (230-400 mesh, 0-10 % dichloromethane / methanol).
I. Using General Procedure E with Compound 33 (0.8 g, 1.95 mmol) in AcOH (80 %, 10 mL) gave the desired dialcohol (1*R*,3a*S*,3b*S*,4*R*,5*R*,5a*S*,10a*R*,10b*S*,12a*S*)-10a,12a-dimethyl-1-(prop-1-en-2-yl)-1,2,3,3a,3b,4,5,5a,6,7,10,10a,10b,11,12,12a-hexadecahydrocyclopenta[5,6]naphtho[1,2-*f*]indazole-4,5-diol (Compound la-4, 0.7 g, 97 %) as an off-white solid after purification by column chromatography (230-400 mesh silica gel, eluted with 10-15 % methanol / dichloromethane).
   LCMS: (Method 1e) MS m/z: 371.2 (M+1), t_{R}: 3.182 min, Purity: 94.99 % (UV).
   HPLC: (Method 2e) t_{R}: 6.876 min, Purity: 98.00 % (UV).
   ¹H-NMR (400 MHz, CD₃OD): δ 7.29 (s, 1H), 4.76 (s, 1H), 3.30-3.27 (m, 1H), 3.16-3.07 (m, 2H), 2.67-2.63 (m, 1H), 2.32-2.04 (m, 4H), 1.93-1.82 (m, 1H), 1.79-1.51 (m, 10H), 1.42-1.05 (m, 4H), 0.82 (s, 3H), 0.66 (s, 3H).

### SYNTHETIC EXAMPLE 5

### Synthesis of (3aS,3bR,4R,5R,5aS,10aR,10bS,12aS,E)-1-ethylidene-10a, 12a-dimethyl-1,2,3,3a,3b,4,5,5a,6,7,10,10a,10b,11,12,12a-hexadecahydrocyclopenta[5,6]naphtho[1,2f]indazole-4,5-diol (Compound la-5)

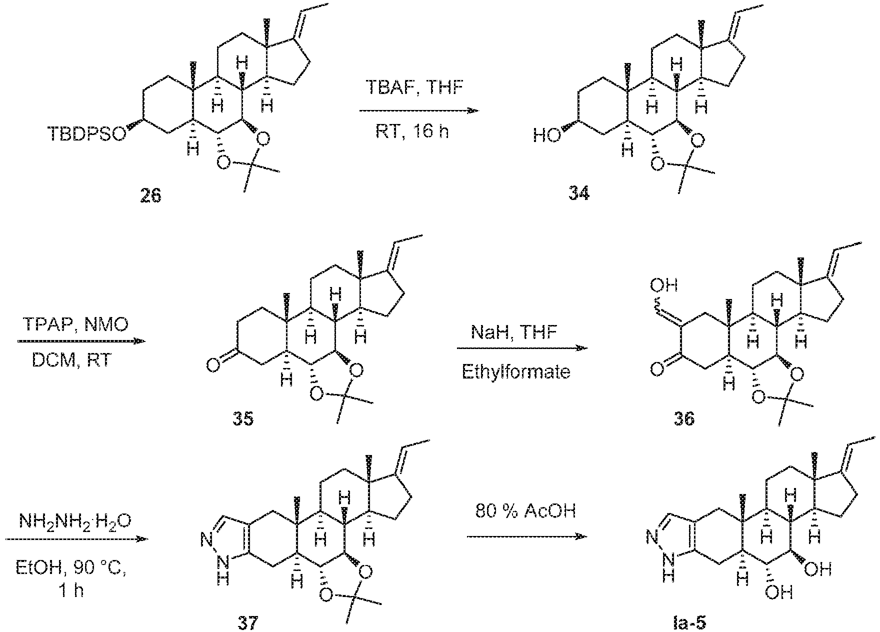

A. Following the General Procedure L with Compound 26 (from Example 4, 4.0 g, 6.53 mmol) and TBAF (1 M in THF, 13.05 mL, 13.05 mmol) in THF (40 mL) gave the desired alcohol (2*S*,4a*R*,4b*S*,6a*S*,9a*S*,9b*R*,9c*R*,12a*R*,12b*S*,*E*)-7-ethylidene-4a,6a,11,11-tetramethylhexadecahydro-1*H*-cyclopenta[1,2]phenanthro[9,10-*d*][1,3]dioxol-2-ol (Compound 34, 2.3 g, 94 %) as an off-white solid after purification by column chromatography (230-400 mesh silica gel, eluted with 0-30 % pet ether / ethyl acetate).
B. Using General Procedure H with Compound 34 (2.3 g, 6.14 mmol), NMO (1.44 g, 10.65 mmol), 4 Å molecular sieves (2.0 g) and TPAP (0.22 g, 0.61 mmol) in CH₂Cl₂ (20 mL) gave the desired ketone (4a*R*,4b*S*,6a*S*,9a*S*,9b*R*,9c*R*,12a*R*,12b*S*,*E*)-7-ethylidene-4a,6a,11,11-tetramethylhexadecahydro-2*H*-cyclopenta[1,2]phenanthro[9,10-*d*][1,3]dioxol-2-one (Compound 35, 2.0 g, 88 %) as an off-white solid after purification by column chromatography (230-400 mesh silica gel, eluted with 15-25 % pet ether / ethyl acetate).
C. Following the General Procedure J with Compound 35 (2.0 g, 5.37 mmol), NaH (60 % in paraffin oil, 0.64 g, 16.11 mmol), ethyl formate (2.17 mL, 26.85 mmol) and THF (20 mL) gave the desired ketone (3*Z*,4a*R*,4b*S*,6a*S*,7*E*,9a*S*,9b*R*,9c*R*,12a*R*,12b*S*,*E*)-7-ethylidene-3-(hydroxymethylene)-4a,6a,11,11-tetramethylhexadecahydro-2*H*-cyclopenta[1,2]phenanthro[9,10-d][1,3]dioxol-2-one (Compound 36, 2.0 g, 93 %) as a brown gummy which was used in the next step without purification.
D. Using General Procedure K with Compound 36 (2.0 g, 4.99 mmol) and hydrazine hydrate (0.98 mL, 19.97 mmol) in EtOH (20 mL) gave the desired pyrazole (3a*S*,3b*R*,3c*R*,6a*R*,6b*S*,11a*R*,11b*S*,13a*S*,*E*)-1-ethylidene-5,5,11a,13a-tetramethyl-1,2,3,3a,3b,3c,6a,6b,7,8,11,11a,11b,12,13,13a-hexadecahydrocyclopenta[5,6][1,3]dioxolo[4',5':3,4]naphtho[1,2-*f*]indazole (Compound 37, 1.5 g, 76 %) as an off-white solid after purification by column chromatography (230-400 mesh silica gel, eluted with 30-40 % pet ether / ethyl acetate).
E. Using General Procedure E with Compound 37 (1.5 g, 3.78 mmol) in AcOH (80 %,15 mL) gave the desired dialcohol (3a*S*,3b*R*,4*R*,5*R*,5a*S*,10a*R*,10b*S*,12a*S*,*E*)-1-ethylidene-10a, 12a-dimethyl-1,2,3,3a,3b,4,5,5a,6,7,10,10a,10b,11,12,12a-hexadecahydrocyclopenta[5,6]naphtho[1,2-*f*]indazole-4,5-diol (Compound la-5, 1.3 g, 97 %) as an off-white solid after purification by column chromatography (230-400 mesh silica gel, eluted with 0-5 % methanol / dichloromethane).
   LCMS: (Method 1e) MS m/z: 357.2 (M+1), t_{R}: 3.077 min, Purity: 96.18 % (UV).
   HPLC: (Method 2e) t_{R}: 5.605 min, Purity: 99.61 % (UV).
   ¹H-NMR (400 MHz, CD₃OD): δ 7.29 (s, 1H), 5.18-5.16 (m, 1H), 3.37-3.3 (m 1H), 3.2-3.10 (m, 2H), 2.68-2.64 (m, 1H), 2.39-2.07 (m, 6H), 1.79-1.54 (m, 9H), 1.42-1.31 (m, 1H), 1.11-1.05 (m, 1H), 0.97 (s, 3H), 0.82 (s, 3H).

### SYNTHETIC EXAMPLE 6

### Synthesis of ((3aS,3bR,4R,5R,5aS,10aR,10bS,12aS)-10a,12a-dimethyl-1-phenyl-3,3a, 3b,4,5,5a,6,7,10,10a, 10b, 11,12,12a-tetradecahydrocyclopenta[5,6]naphtho[1,2-f]indazols-4,5-diol (Compound la-6)

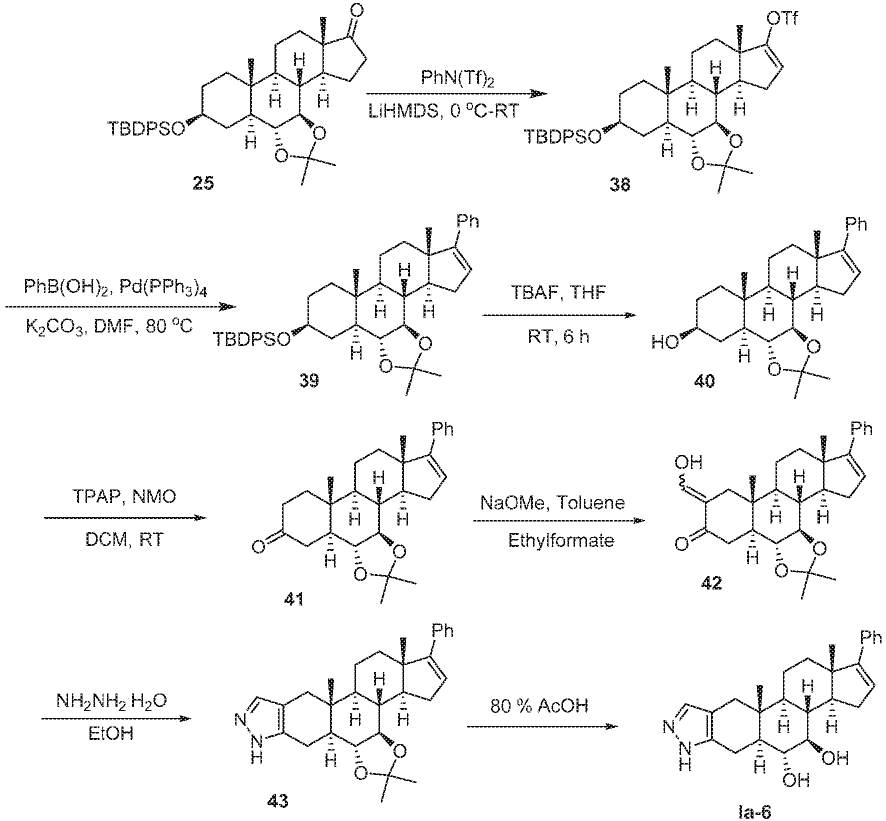

A. To a stirred solution of Compound 25 (from Example 4, 5.0 g, 8.32 mmol) in THF (50 mL) at 0 °C were added *N-*phenyl-bis(trifluoromethanesulfonimide) (5.95 g, 16.64 mmol) and lithium *bis*(trimethylsilyl)amide (1 M in THF, 24.96 mL, 24.96 mmol) dropwise. The resultant solution was stirred at room temperature for 2.5 hours. The mixture was diluted with ice cold water (1 x 50 mL) and the aqueous was extracted with EtOAc (2 x 50 mL) and washed with brine (1 x 50 mL). The organic layer was dried over Na₂SO₄, filtered and concentrated. The residue was purified by column chromatography on silica gel (230-400 mesh, 0-5 % pet ether / ethyl acetate) to afford (2*S*,4a*R*,4b*S*,6a*S*,9a*S*,9b*R*,9c*R*, 12a*R*, 12b*S*)-2-( (*tert*-butyldiphenylsilyl)oxy)-4a,6a,11,11-tetramethyl-2,3,4,4a,4b,5,6,6a,9,9a,9b,9c,12a,12b-tetradecahydro-1*H-*cyclopenta[1,2]phenanthro[9,10-*d*][1,3]dioxol-7-yl trifluoromethanesulfonate (Compound 38, 5.1 g, 84 %) as an off-white solid.
B. To a stirred solution of Compound 38 (5.1 g, 6.96 mmol) in DMF (50 mL) were added phenyl boronic acid (1.02 g, 8.35 mmol) and potassium carbonate (1.92 g, 13.92 mmol) at room temperature. The reaction mixture was degassed with nitrogen for 20 minutes. To the solution was added tetrakis(triphenylphosphine)palladium(0) (0.80 g, 0.70 mmol) and the reaction was heated to 80 °C and stirred for 3 hours. The reaction mixture was filtered through the bed of CELITE^{®} and the filtrate was evaporated under reduced pressure. The residue was diluted with EtOAc (2 x 50 mL) then washed consecutively with water (1 x 10 mL) and brine (1 x 10 mL). The organic layer was dried over Na₂SO₄, filtered and concentrated. The crude material was purified by column chromatography on silica gel (230-400 mesh, 0-10 % pet ether / ethyl acetate) to afford *tert-*butyldiphenyl(((2*S*,4a*R*,4b*S*,6a*S*,9a*S*,9b*R*,9c*R*,12a*R*,12b*S*)-4a,6a,11,11-tetramethyl-7-phenyl-2,3,4,4a,4b,5,6,6a,9,9a,9b,9c, 12a, 12b-tetradecahydro-1*H-*cyclopenta[1,2]phenanthro[9,10-*d*][1,3]dioxol-2-yl)oxy)silane (Compound 39, 3.1 g, 67 %) as an off-white solid.
C. Following the General Procedure L with Compound 39 (3.0 g, 4.54 mmol) and TBAF (1 M in THF, 9.08 mL, 9.08 mmol) in THF (30 mL), gave the desired alcohol (2*S*,4a*R*,4b*S*,6a*S*,9a*S*,9b*R*,9c*R*,12a*R*,12b*S*)-4a,6a,11,11-tetramethyl-7-phenyl-2,3,4,4a,4b,5,6,6a,9,9a,9b,9c,12a,12b-tetradecahydro-1*H-*cyclopenta[1,2]phenanthro[9,10-*d*][1,3]dioxol-2-ol (Compound 40, 1.6 g, 84 %) as a white solid after purification by column chromatography (230-400 mesh silica gel, eluted with 0-50 % pet ether / ethyl acetate).
D. Using General Procedure H with Compound 40 (1.6 g, 3.79 mmol), NMO (0.89 g, 6.58 mmol), 4 Å molecular sieves (1.6 g) and TPAP (0.13 g, 0.38 mmol) in CH₂Cl₂ (15 mL), gave the desired ketone (4a*R*,4b*S*,6a*S*,9a*S*,9b*R*,9c*R*,12a*R*,12b*S*)-4a,6a,11,11-tetramethyl-7-phenyl-1,3,4,4a,4b,5,6,6a,9,9a,9b,9c,12a,12b-tetradecahydro-2H-cyclopenta[1,2]phenanthro[9,10-*d*][1,3]dioxol-2-one (Compound 41, 1.5 g, 94 %) as a white solid after purification by column chromatography (230-400 mesh silica gel, eluted with 0-20 % pet ether / ethyl acetate).
E. To a stirred solution of Compound 41 (1.5 g, 3.57 mmol) in toluene (15 mL) at 0 °C were added sodium methoxide solution (25 % wt. in MeOH, 1.54 mL, 5.64 mmol) and ethyl formate (1.44 mL, 17.85 mmol) dropwise. The resultant solution was stirred at room temperature for 3.5 hours. The mixture was evaporated under reduced pressure and the residue was diluted with ice cold water (1 x 15 mL). The aqueous was extracted with EtOAc (2 x 15 mL) and washed with brine (1 x 15 mL). The organic layer was dried over Na₂SO₄, filtered and concentrated to give the desired ketone (4a*R*,4b*S*,6a*S*,9a*S*,9b*R*,9c*R*,12a*R*,12b*S*)-3-(hydroxymethylene)-4a,6a,11,11-tetramethyl-7-phenyl-1 ,3,4,4a,4b,5,6,6a,9,9a,9b,9c,12a,12b-tetradecahydro-2*H-*cyclopenta[1,2]phenanthro[9,10-*d*][1,3]dioxol-2-one (Compound 42, 1.5 g, 94 %) as a brown gummy solid which was taken for next step without purification.
F. Using General Procedure K with Compound 42 (1.5 g, 3.34 mmol) and hydrazine hydrate (0.33 mL, 6.69 mmol) in EtOH (15 mL) gave the desired pyrazole (3a*S*,3b*R*,3c*R*,6a*R*,6b*S*,11a*R*,11b*S*,13aS)-5,5,11a,13a-tetramethyl-1-phenyl-3,3a,3b,3c,6a,6b,7,8,11,11a,11b,12,13,13a-tetradecahydrocyclopenta[5,6][1,3]dioxolo[4',5':3,4]naphtho[1,2-*f*]indazole (Compound 43, 1.3 g, 88 %) as an off-white solid after purification by column chromatography (230-400 mesh silica gel, eluted with 40-50 % pet ether / ethyl acetate).
G. Using General Procedure E with Compound 43 (1.3 g, 2.92 mmol) in AcOH (80 %, 10 mL) gave the desired dialcohol (3a*S*,3b*R*,4*R*,5*R*,5a*S*,10a*R*,10b*S*,12a*S*)-10a,12a-dimethyl-1-phenyl-3,3a,3b,4,5,5a,6,7, 10, 10a, 10b, 11, 12,12a-tetradecahydrocyclopenta[5,6]naphtho[1,2-*f*]indazole-4,5-diol (Compound la-6, 0.8 g, 68 %) as an off-white solid after purification by column chromatography (230-400 mesh silica gel, eluted with 0-5 % methanol / dichloromethane).
   LCMS: (Method 1d) MS m/z: 405.2 (M+1), t_{R}: 2.106 min, Purity: 93.14 % (UV).
   HPLC: (Method 2b) t_{R}: 11.323 min, Purity: 94.81 % (UV).
   ¹H-NMR (400 MHz, CD₃OD): δ 7.39-7.36 (m, 2H), 7.31-7.27 (m, 3H), 7.24-7.22 (m, 1H), 5.94-5.93 (m, 1H), 3.19-3.13 (m, 2H), 2.67-2.63 (m, 1H), 2.53-2.31 (m, 3H), 2.20-2.05 (m, 2H), 1.86-1.58 (m, 5H), 1.57-1.31 (m, 2H), 1.16-1.11 (m, 4H), 0.88 (s, 3H).

### SYNTHETIC EXAMPLE 7

### Synthesis of (1S,3aS,3bR,4R,5R,5aS,10aR,10bS,12aS)-1,10a,12a-trimethyl-1,2,3,3a,3b,4,5,5a,6,7,10,10a,10b,11,12,12a-hexadecahydrocyclopenta[5,6]naphtho[1,2-f]indazole-1,4,5-triol (Compound la-7)

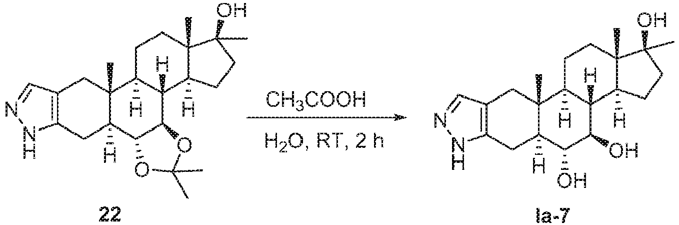

Using General Procedure E with Compound 22 (from Example 3, 3.7 g, 9.24 mmol, 1 eq) and acetic acid (80 % aqueous, 20 mL) gave the desired triol (1*S*,3a*S*,3b*R*,4*R*,5*R*,5a*S*,10a*R*,10b*S*,12a*S*)-1,10a,12a-trimethyl-1,2,3,3a,3b,4,5,5a,6,7,10,10a, 10b, 11,12,12a-hexadecahydrocyclopenta[5,6]naphtho[1,2-*f*]indazole-1,4,5-triol (Compound la-7, 3.2 g) as a crude brown solid.
LCMS: (Method 1b) MS m/z: 361.2 (M+1), t_{R}: 1.957 min, Purity: 98.84 % (ELSD).
¹H-NMR (400 MHz, DMSO-d6): δ 12.28 (s, 1H), 7.22 (s, 1H), 4.04-3.99 (m, 1H), 3.10-3.05 (m, 1H), 2.98-2.87 (m, 1H), 2.15-2.01 (m, 1H), 1.99 (s, 6H), 1.91-1.83 (m, 2H), 1.71-1.16 (m, 8H), 1.08 (s, 3H), 0.90-0.84 (m, 1H), 0.76 (s, 3H), 0.69 (s, 3H).

### SYNTHETIC EXAMPLE 8

### Synthesis of (1R,3aS,3bS,4R,5R,5aS,10aR,10bS,12aR)-1-((2S,5R)-5-ethyl-3-hydroxy-6-methylheptan-2-yl)-10a,12a-dimethyl-1,2,3,3a,3b,4,5,5a,6,7,10,10a,10b,11,12,12a-hexadecahydrocyclopenta[5,6]naphtho[1,2-f]indazole-4,5-diol (Compound la-8)

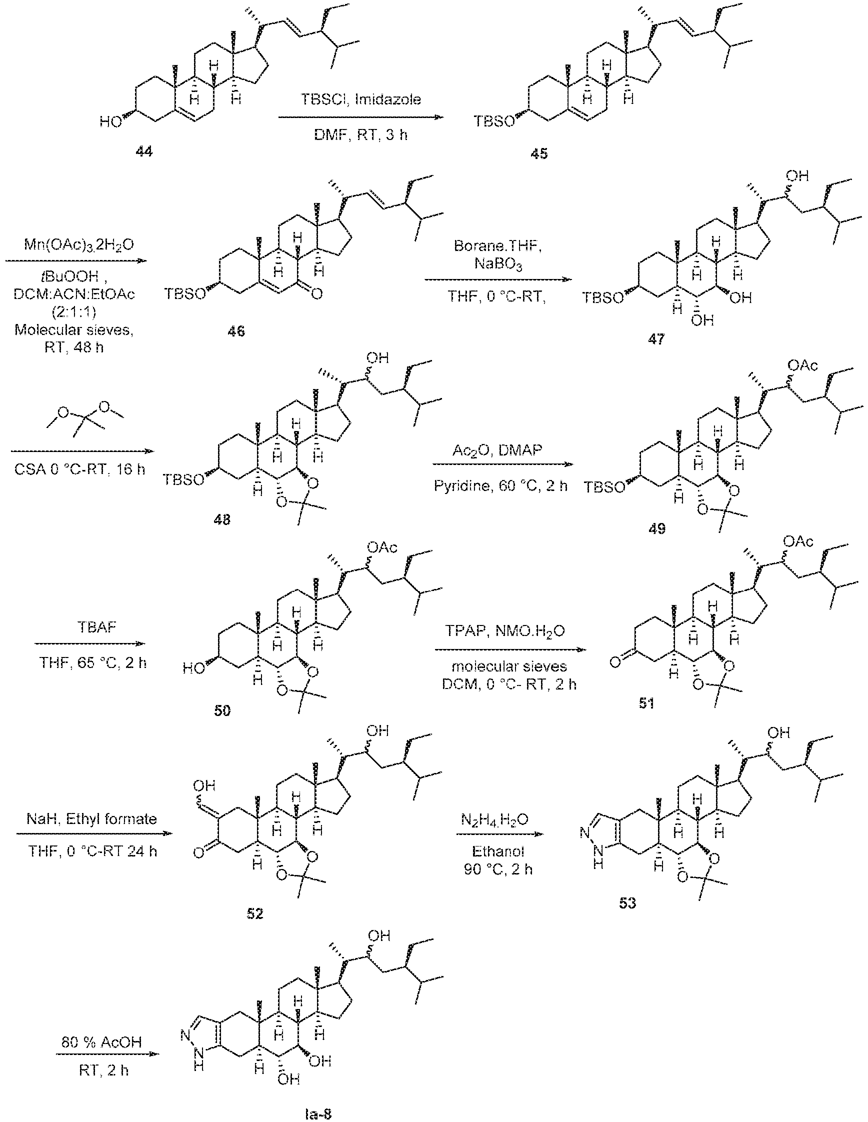

A. Using General Procedure B with Stigmasterol (Compound 44, 30.0 g, 72.75 mmol), imidazole (12.37 g, 181.73 mmol) and TBSCI (16.43 g, 109.04 mmol) in DMF (300 mL) followed by purification by column chromatography on silica gel (230-400 mesh, 0-5 % pet ether / ethyl acetate) afforded *tert-*butyl(((3*S*,8*S*,9*S*,10*R*,13*R*,14*S*,17*R*)-17-((2*R*,5*S*,*E*)-5-ethyl-6-methylhept-3-en-2-yl)-10,13-dimethyl-2,3,4,7,8,9,10,11,12,13,14,15,16,17-tetradecahydro-1*H-*cyclopenta[a]phenanthren-3-yl)oxy)dimethylsilane (Compound 45, 38.0 g, 99 %) as a colourless gummy solid.
B. Using General Procedure C with Compound 45 (38.0 g, 72.11 mmol), TBHP (5 M in decane, 75.0 mL, 374.97 mmol), 4 Å molecular sieves (38.0 g) and manganese(III) acetate dihydrate (1.93 g, 7.21 mmol) in CH₂Cl₂:ACN:EtOAc (2:1:1, 380 mL), gave the desired ketone (3S, 8*S*,9*S*,10*R*,13*R*,14*S*,17*R*)-3-((*tert-*butyldimethylsilyl)oxy)-17-((2*R*,5*S*,*E*)-5-ethyl-6-methylhept-3-en-2-yl)-10,13-dimethyl-1,2,3,4,8,9,10,11,12,13,14,15,16,17-tetradecahydro-7*H*-cyclopenta[a]phenanthren-7-one (Compound 46, 22.5 g, 58 %) as an off-white solid after purification by column chromatography on silica gel (230-400 mesh, 0-5 % pet ether / ethyl acetate).
C. Using General Procedure D with Compound 46 (22.5 g, 41.59 mmol), borane in THF (1 M, 92.75 mL, 92.75 mmol) and sodium perborate tetrahydrate (19.20 g, 124.78 mmol) in THF (200 mL) gave the desired dialcohol (3*S*,5*S*,6*R*,7*R*,8*S*,9*S*,10*R*,13*R*,14*S*,17*R*)-3-((*tert*-butyldimethylsilyl)oxy)-17-((2*S*,5*R*)-5-ethyl-3-hydroxy-6-methylheptan-2-yl)-10,13-dimethylhexadecahydro-1*H-*cyclopenta[a]phenanthrene-6,7-diol (Compound 47, 24.0 g) as a white solid which was used in the next step without purification.
D. Using General Procedure F with Compound 47 (24.0 g, 41.45 mmol) and camphorsulfonic acid (0.96 g, 4.15 mmol) in 2,2-dimethoxypropane (203 mL) followed by purification by column chromatography on silica gel (230-400 mesh, 10-15 % pet ether / ethyl acetate) afforded (2*S*,5*R*)-2-((2*S*,4a*R*,4b*S*,6a*R*,7*R*,9a*S*,9b*S*,9c*R*,12a*R*,12bS)-2-((*tert*-butyldimethylsilyl)oxy)-4a,6a,11,11-tetramethylhexadecahydro-1*H*-cyclopenta[1,2]phenanthro[9,10-*d*][1,3]dioxol-7-yl)-5-ethyl-6-methylheptan-3-ol (Compound 48, 12.0 g, 47 %) as a white solid.
E. Using General Procedure A with Compound 48 (12.0 g, 19.38 mmol), DMAP (0.24 g, 1.94 mmol) and Ac₂O (5.42 mL, 57.34 mmol) in pyidine (100 mL) gave the desired ester (2*S*,5*R*)-2-((2*S*,4a*R*,4b*S*,6a*R*,7R,9a*S*,9b*S*,9c*R*,12a*R*,12b*S*)-2-((*tert*butyldimethylsilyl)oxy)-4a,6a,11,11-tetramethylhexadecahydro-1*H-*cyclopenta[1,2]phenanthro[9,10-*d*][1,3]dioxol-7-yl)-5-ethyl-6-methylheptan-3-yl acetate (Compound 49, 11.0 g, 86 %) as a yellow gummy solid which was used in the next step without purification.
F. Following the General Procedure L with Compound 49 (11.0 g, 16.64 mmol) and TBAF (1 M in THF, 33.28 mL, 33.28 mmol) in THF (100 mL), gave the desired alcohol (2*S*,5*R*)-5-ethyl-2((2*S*,4a*R*,4b*S*,6a*R*,7R,9a*S*,9b*S*,9c*R*,12a*R*,12b*S*)-2-hydroxy-4a,6a,11,11-tetramethylhexadecahydro-1*H*-cyclopenta[1,2]phenanthro[9,1 0-d][1,3]dioxol-7-yl)-6-methylheptan-3-yl acetate as a white solid (Compound 50, 5.0 g, 55 %) after purification by column chromatography on silica gel (230-400 mesh, 40-45 % pet ether / ethyl acetate).
G. Using General Procedure H with Compound 50 (5.0 g, 9.14 mmol), NMO (2.14 g, 18.29 mmol), 4 Å molecular sieves (5.0 g) and TPAP (0.32 g, 0.914 mmol) in CH₂Cl₂ (50 mL) gave the desired ketone (2*S*,5*R*)-5-ethyl-6-methyl-2-((4aR,4bS,6aR,7R,9aS,9bS,9cR,12aR,12bS)-4a,6a,11,11-tetramethyl-2-oxohexadecahydro-1*H*-cyclopenta[1,2]phenanthro[9,10-*d*][1,3]dioxol-7-yl)heptan-3-yl acetate (Compound 51, 3.8 g, 76 %) as an off-white solid after purification by column chromatography on silica gel (230-400 mesh, 5-10 % pet ether / ethyl acetate).
H. Using General Procedure J with Compound 51 (3.8 g, 6.97 mmol), sodium hydride (60 % in paraffin oil, 1.39 g, 34.87 mmol) and ethyl formate (3.94 mL, 48.82 mmol) in THF (40 mL) gave the desired ketone (4*a*R,4b*S,*6a*R*,7R,9a*S*,9b*S*,9c*R*,12a*R*,12b*S*)-7-((2*S*,5*R*)-5-ethyl-3-hydroxy-6-methylheptan-2-yl)-3-(hydroxymethylene)-4a,6a,11,11-tetramethylhexadecahydro-2H-cyclopenta[1,2]phenanthro[9,10-*d*][1,3]dioxol-2-one (Compound 52, 3.0 g, 81 %) as a brown gummy solid which was used in the next step without purification.
I. Using General Procedure K with Compound 52 (3.0 g, 5.65 mmol) and hydrazine hydrate (0.55 mL, 11.30 mmol) in EtOH (30 mL) gave the desired pyrazole (2*S*,5*R*)-5-ethyl-6-methyl-2-((1*R*,3a*S*,3b*S*,3c*R*,6a*R*,6b*S*,11a*R*,11b*S*,13a*R*)-5,5,11a,13a-tetramethyl-1,2,3,3a,3b,3c,6a,6b,7,8,11,11a,11b,12,13,13a-hexadecahydrocyclopenta[5,6][1,3]dioxolo[4',5':3,4]naphtho[1,2-*f*]indazol-1-yl)heptan-3-ol (Compound 53, 1.1 g, 37 %) as an off-white solid after purification by column chromatography on silica gel (230-400 mesh, 40-50 % pet ether / ethyl acetate).
J. Using General Procedure E with Compound 53 (1.1 g, 2.09 mmol) in AcOH (80 %, 10 mL) gave the desired dialcohol (1*R*,3a*S*,3b*S*,4*R*,5*R*,5a*S*,10a*R*,10b*S*,12a*R*)-1-((2*S*,5*R*)-5-ethyl-3-hydroxy-6-methylheptan-2-yl)-10a,12a-dimethyl-1,2,3,3a, 3b, 4,5,5a,6,7,10,10a, 10b, 11,12,12a-hexadecahydrocyclopenta[5,6]naphtho[1,2-*f*]indazole-4,5-diol (Compound 1a-8, 0.9 g, 89 %) as an off-white solid after purification by column chromatography on silica gel (230-400 mesh, 10-15 % methanol / dichloromethane).

### SYNTHETIC EXAMPLE 9

### Synthesis of (1S,3aS,3bR,4R,5R,5aS,10aR,10bS,12aS)-10a,12a-dimethyl-1-(thiazol-2-\yl)-1,2,3,3a,3b,4,5, 5a,6,7,10,10a,10b,11,12,12a-hexadecahydrocyclopenta[5,6]naphtho[1,2-f]indazole-1,4,5-triol (Compound la-9)

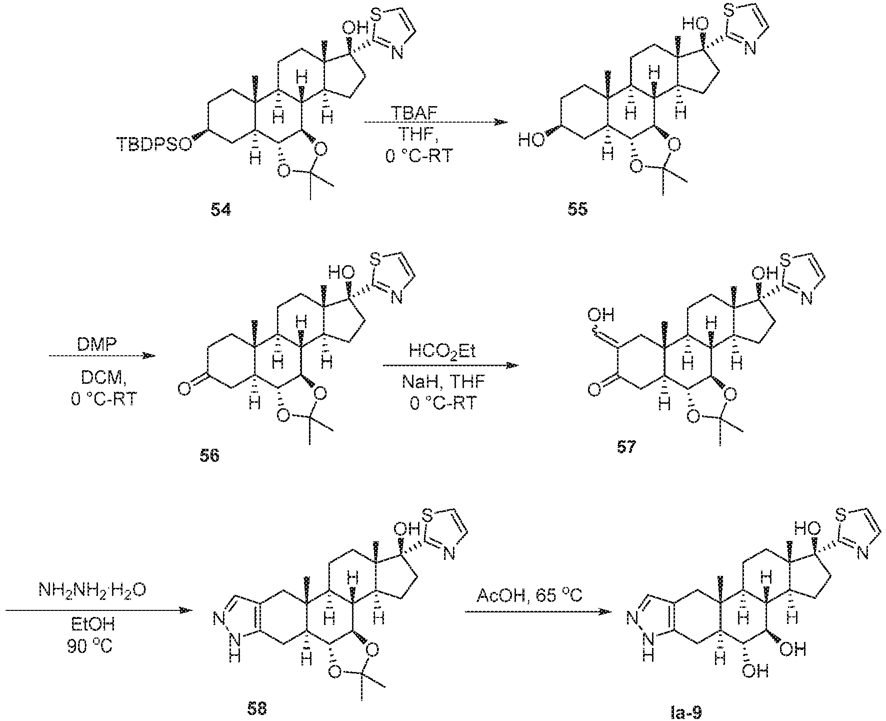

A. Using General Procedure L with (2S,4aR,4bS,6aS,7S,9aS,9bR,9cR,12aR, 12bS)-2-((*tert*-butyldiphenylsilyl)oxy)-4a,6a,11,11-tetramethyl-7-(thiazol-2-yl)hexadecahydro-1*H-*cyclopenta[1,2]phenanthro[9,10-*d*][1,3]dioxol-7-ol(Compound 54, as prepared in U.S. Pat. No. 9,765,085, 3.8 g, 5.54 mmol), TBAF solution (1 M in THF, 11.07 mL, 11.07 mmol), and THF (35 mL) gave the desired alcohol (2*S*,4a*R*,4b*S*,6a*S*,7*S*,9a*S*,9b*R*,9c*R*,12a*R*,12b*S*)-4a,6a,11,11-tetramethyl-7-(thiazol-2-yl)hexadecahydro-1*H*-cyclopenta[1,2]phenanthro[9,10-*d*][1,3]dioxole-2,7-diol (Compound 55, 3.16 g) as a yellow gummy solid which was used in the next step without purification.
B. Following the General Procedure I with Compound 55 (3.16 g, 7.06 mmol) and Dess-Martin periodane (5.99 g, 14.12 mmol) in DCM (30 mL) gave the desired ketone (4a*R*,4b*S*,6a*S*,7*S*,9a*S*,9b*R*,9c*R*,12a*R*,12b*S*)-7-hydroxy-4a,6a,11,11-tetramethyl-7-(thiazol-2-yl)hexadecahydro-2*H*- cyclopenta[1,2]phenanthro[9,10-d][1,3]dioxol-2-one (Compound 56, 2.23 g, 71 %) as a white solid after purification by column chromatography on silica gel (230-400 mesh, 20-30 % pet ether / ethyl acetate).
C. Using General Procedure J with Compound 56 (2.23 g, 5.00 mmol), NaH (60 % in paraffin oil, 0.80 g, 20.02 mmol), ethyl formate (2.43 mL, 30.03 mmol) and THF (20 mL) gave the desired ketone (4a*R*,4b*S*,6a*S*,7*S*,9a*S*,9b*R*,9c*R*,12a*R*,12b*S*)-7-hydroxy-3-(hydroxymethylene)-4a,6a, 11, 11 -tetramethyl-7-(thiazol-2-yl)hexadecahydro-2*H-*cyclopenta[1,2]phenanthro[9,10-*d*][1,3]dioxol-2-one (Compound 57, 2.28 g, 96 %) as a white solid after purification by column chromatography on silica gel (230-400 mesh, 20-30 % pet ether / ethyl acetate).
D. Using General Procedure K with Compound 57 (2.28 g, 4.81 mmol) and hydrazine hydrate (0.94 mL, 19.26 mmol) in EtOH (20 mL) gave the desired pyrazole (1*S*,3a*S*,3b*R*,3c*R*,6a*R*,6b*S*,11a*R*,11b*S*,13a*S*)-5,5,11a,13a-tetramethyl-1-(thiazol-2-yl)-1,2,3,3a,3b,3c,6a,6b,7,8,11,11a,11b,12,13,13a-hexadecahydrocyclopenta[5,6][1,3]dioxolo[4',5':3,4]naphtho[1,2-*f*]indazol-1-ol (Compound 58, 2.16 g, 96 %) as an off-white solid after purification by column chromatography (230-400 mesh silica gel, eluted with 50-60 % pet ether / ethyl acetate).
E. Using General Procedure E with Compound 58 (2.16 g, 4.60 mmol) in AcOH (80 %, 20 mL) gave the desired trialcohol (1*S*,3a*S*,3b*R*,4*R*,5*R*,5a*S*,10a*R*,10b*S*,12a*S*)-10a, 12a-dimethyl-1-(thiazol-2-yl)-1,2,3,3a,3b,4,5,5a,6,7,10,10a,10b,11,12,12a-hexadecahydrocyclopenta[5,6]naphtho[1,2-*f*]indazole-1,4,5-triol (Compound la-9, 1.3 g, 66 %) as an off-white solid after purification by column chromatography (230-400 mesh silica gel, eluted with 0-10 % dichloromethane / methanol).
   LCMS: (Method 1d) MS m/z: 430.2 (M+1), t_{R}: 1.242 min, Purity: 95.91 % (UV).
   HPLC: (Method 2b) t_{R}: 6.987 min, Purity: 95.07 % (UV).
   ¹H-NMR (400 MHz, CD₃OD): δ 7.72 (d, *J*= 3.2 Hz, 1H), 7.47 (d, *J*= 3.2 Hz, 1H), 7.24 (s, 1H), 3.29-3.09 (m, 3H), 2.58-2.26 (m, 4H), 2.13-1.91 (m, 5H), 1.67-1.43 (m, 5H), 1.06 (s, 3H), 0.77 (s, 3H), 0.40-0.39 (m, 1H).

### SYNTHETIC EXAMPLE 9.1

### Synthesis of (1S,3aS,3bR,4R,5R,5aS,10aR,10bS,12aS)-10a,12a-dimethyl-1-(pyridin-2-yl)-1,2,3,3a,3b,4,5,5a,6,7,10,10a,10b,11,12,12a-hexadecahydrocyclopenta[5,6]naphtho[1,2-f]indazole-1,4,5-triol (Compound la-10)

Following the procedure as described in Synthetic Example 9 and making non-critical variations a) using (2*S*,4a*R*,4b*S*,6a*S*,7*S*,9a*S*,9b*R*,9c*R*,12a*R*,12b*S*)-2-((*tert-*butyldiphenylsilyl)oxy)-4a,6a,11,11 -tetramethyl-7-(pyridin-2-yl)hexadecahydro-1*H-*cyclopenta[1,2]phenanthro[9,10-*d*][1,3]dioxol-7-ol (Compound 59 as prepared in U. S. Pat. No. 9,765,085) to replace Compound 54, b) altering the conditions for conversion of Compound 55 to 56 to treatment with TPAP and NMO H₂O, and c) altering the conditions for conversion of Compound 56 to 57, to treatment with sodium methoxide and ethyl formate in toluene, the title compound (1*S*,3a*S*,3b*R*,4*R*,5*R*,5a*S*,10a*R*,10b*S*,12a*S*)-10a,12a-dimethyl-1-(pyridin-2-yl)-1,2,3,3a,3b,4,5,5a,6,7,10,10a,10b,11,12,12a-hexadecahydrocyclopenta[5,6]naphtho[1,2-*f*]indazole-1,4,5-triol (Compound la-10, 2.0 g, 88 %) was obtained as an off-white solid after purification by column chromatography (230-400 mesh silica gel, eluted with 10-15 % methanol / dichloromethane).
LCMS: (Method 1e) MS m/z: 424.1 (M+1), t_{R}: 2.185 min, Purity: 92.18 % (UV).
HPLC: (Method 2e) t_{R}: 5.464 min, Purity: 91.32 % (UV).
¹H-NMR (400 MHz, CD₃OD): δ 8.54-8.53 (m, 1H), 7.81-7.77 (m, 1H), 7.57-7.55 (m, 1H), 7.29-7.26 (m, 1H), 7.23 (s, 1H), 3.29-3.06 (m, 3H), 2.55-2.25 (m, 4H), 2.09-1.95 (m, 3H), 1.70-1.40 (m, 6H), 1.10 (s, 3H), 0.80 (s, 3H), 0.70-0.64 (m, 1H), 0.19-0.11 (m, 1H).

### SYNTHETIC EXAMPLE 10

### Synthesis of (3aS,3bR,4R,5R,5aS,11aR,11bS,13aS)-11a,13a-dimethyl-1-methylene-2,3,3a,3b,4,5,5a,6,11,11a,11b,12,13,13a-tetradecahydro-1H-cyclopenta[5,6]naphtho[1,2-g]quinoxaline-4,5-diol (Compound Ib-1)

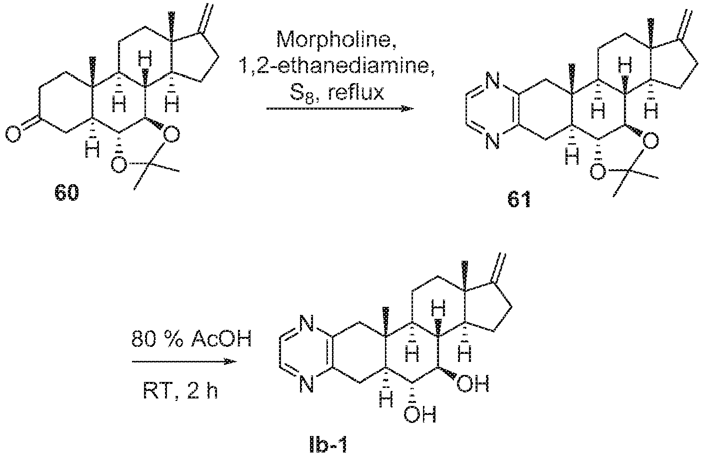

A. To a stirred solution of Compound 60 (as prepared in U.S. Pat. No. 9,765,085, 10.0 g, 27.89 mmol) in morpholine (100 mL) were added sulphur (S₈, 8.93 g, 278.92 mmol) and 1,2-ethanediamine (16.76 g, 278.92 mmol). The reaction mixture was refluxed for 16 hours. The mixture was diluted with ice cold water (1 x 100 mL) and the aqueous was extracted with EtOAc (2 x 100 mL) and washed with brine (1 x 100 mL). The organic layer was dried over Na₂SO₄, filtered and concentrated. The crude was purified by column chromatography on silica gel (60-120 mesh, 0-10 % pet ether / ethyl acetate) to afford (3a*S*,3b*R*,3c*R*,6a*R*,6b*S*,12a*R*,12b*S*,14a*S*)-5,5,12a,14a-tetramethyl-1-methylene-2,3,3a,3b,3c,6a,6b,7,12,12a, 12b, 13,14,14a-tetradecahydro-1*H-*cyclopenta[5,6][1,3]dioxolo[4',5':3,4]naphtho[1,2-*g*]quinoxaline (Compound 61, 4.08 g, 37 %) as a yellow solid.
B. Using General Procedure E with Compound 61 (4.0 g, 10.14 mmol) in AcOH (80 %, 40 mL) gave the desired dialcohol (3a*S*,3b*R*,4*R*,5*R*,5a*S*,11a*R*,11b*S*,13a*S*)-11a,13a-dimethyl-1-methylene-2,3,3a,3b,4,5,5a,6,11,11a,11b,12,13,13a-tetradecahydro-1*H-*cyclopenta[5,6]naphtho[1,2-*g*]quinoxaline-4,5-diol (Compound Ib-1, 2.79 g, 78 %) as an off-white solid after purification by column chromatography on silica gel (230-400 mesh, 0-5 % dichloromethane / methanol).
   LCMS: (Method 1e) MS m/z: 355.2 (M+1), t_{R}: 2.749 min, Purity: 96.92 % (UV).
   HPLC: (Method 2e) t_{R}: 6.616 min, Purity: 95.28 % (UV).
   ¹H-NMR (400 MHz, CD₃OD): δ 8.39 (d, *J*= 2.4 Hz, 2H), 4.69-4.68 (m, 2H), 3.41-3.35 (m, 2H), 3.19-3.14 (m, 1H), 3.02-2.98 (m, 1H), 2.80-2.65 (m, 2H), 2.56-2.49 (m, 1H), 2.31-2.3 (m, 1H), 2.18-2.11 (m, 1H), 1.95-1.60 (m, 6H), 1.33-1.28 (m, 3H), 0.87 (m, 6H).

### SYNTHETIC EXAMPLE 10.1

### Synthesis of (1R,3aS,3bS,4R,5R,5aS,11aR,11bS,13aR)-11a,13a-dimethyl-1-((R)-6-methylheptan-2-yl)-2,3,3a,3b,4,5,5a,6,11,11a,11b,12,13,13a-tetradecahydro-1H-cyclopenta[5,6]naphtho[1,2-g]quinoxaline-4,5-diol (Compound Ib-2)

Following the procedure as described in Synthetic Example 10 and making non-critical variations, using (4a*R*,4b*S*,6a*R*,7*R*,9a*S*,9b*S*,9c*R*,12a*R*,12b*S*)-4a,6a,11,11-tetramethyl-7-((R)-6-methylheptan-2-yl)hexadecahydro-2*H-*cyclopenta[1,2]phenanthro[9,10-*d*][1,3]dioxol-2-one (Compound 15, from Example 2) to replace Compound 60, the title compound (1*R*,3a*S*,3b*S*,4*R*,5*R*,5a*S*,11a*R*,11b*S*,13a*R*)-11a,13a-dimethyl-1-((*R*)-6-methylheptan-2-yl)-2,3,3a,3b,4,5,5a,6,11,11a,11b,12,13,13a-tetradecahydro-1*H-*cyclopenta[5,6]naphtho[1,2-*g*]quinoxaline-4,5-diol (Ib-2, 1.6 g, 88 %) was obtained as an off-white solid after purification by column chromatography (230-400 mesh silica gel, eluted with 5-10 % methanol / dichloromethane).
LCMS: (Method 1f) MS m/z: 455.3 (M+1), t_{R}: 2.577 min, Purity: 99.94 % (ELSD).
HPLC: (Method 2b) t_{R}: 18.463 min, Purity: 96.86 % (UV).
¹H-NMR (400 MHz, CD₃OD): δ 8.40-8.39 (m, 2H), 3.40-3.39 (m, 1H), 3.15-3.10 (m, 1H), 3.00-2.95 (m, 1H), 2.69-2.64 (m, 2H), 2.15-2.10 (m 1H), 2.09-1.97 (m 1H), 1.93-1.84 (m 1H), 1.77-1.69 (m, 2H), 1.59-1.51 (m, 4H), 1.43-1.41 (m, 3H), 1.32-1.07 (m, 10H), 1.00-0.99 (m, 3H), 0.92-0.88 (m, 9H), 0.78 (s, 3H).

### SYNTHETIC EXAMPLE 11

### Synthesis of (1R,3aS,3bS,4R,5R,5aS,11aR,11bS,13aR-8,11a,13a-tdmethyl-1-((R)-6-methylheptan-2-yl)-2,3,3a,3b,4,5,5a,6,11,11a,11b,12,13,13a-tetradecahydro-1H-cyclopenta[5,6]naphtho[1,2-g]quinazoline-4,5-diol (Compound Ic-1)

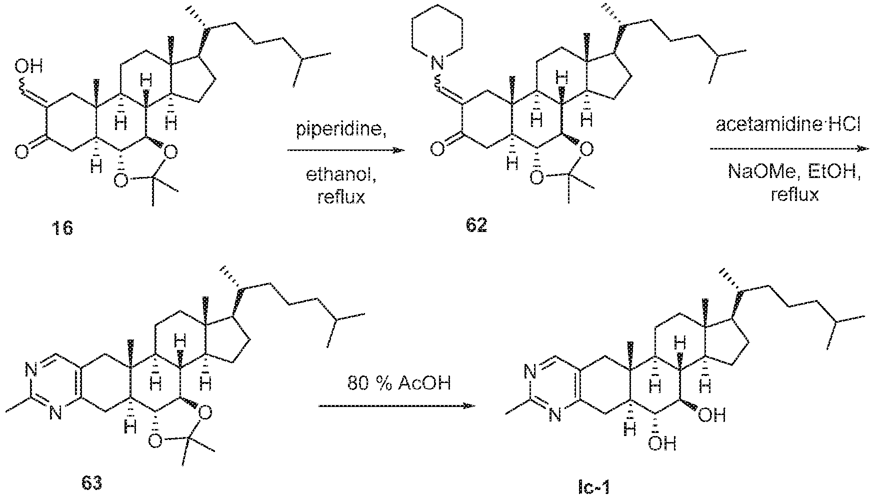

A. To a stirred solution of Compound 16 (from Example 2, 6 g, 12.37 mmol) in EtOH (60 mL) was added at room temperature piperidine (1.46 mL, 14.79 mmol) dropwise. The resultant solution was refluxed for 2 hours. The mixture was evaporated under reduced pressure to afford the desired ketone (4a*R*,4b*S*,6a*R*,7*R*,9a*S*,9b*S*,9c*R*,12a*R*,12b*S*)-4a,6a,11,11-tetramethyl-7-((*R*-6 methylheptan-2-yl)-3-(piperidin-1-ylmethylene)hexadecahydro-2*H-*cyclopenta[1,2]phenanthro[9,10-*d*][1,3]dioxol-2-one (Compound 62, 6.1 g, 89 %) as a brown gummy solid which was taken for next step without purification.
B. To a stirred solution of sodium methoxide (0.66 g, 12.22 mmol) and acetamidine hydrochloride (0.58 g, 6.14 mmol) in EtOH (10 mL) was added at room temperature Compound 62 (3.4 g, 6.13 mmol) in EtOH (25 mL) dropwise. The reaction mixture was refluxed for 12 hours. The mixture was evaporated under reduced pressure and the residue was diluted with EtOAc (2 x 30 mL) and washed consecutively with water (1 x 30 mL) and brine (1 x 30 mL). The organic layer was dried over Na₂SO₄, filtered and concentrated. The crude was purified by column chromatography on silica gel (230-400 mesh, 30-40 % pet ether / ethyl acetate) to afford (1*R*,3a*S*,3b*S*,3c*R*,6a*R*,6b*S*,12a*R*,12b*S*,14a*R*)-5,5,9,12a,14a-pentamethyl-1-((*R*)-6-methylheptan-2-yi)-2,3,3a,3b,3c,6a,6b,7,12,12a, 12b, 13,14,14a-tetradecahydro-1*H*-cyclopenta[5,6][1,3]dioxolo[4',5':3,4]naphtho[1,2-*g*]quinazoline (Compound 63, 2.6 g, 83 %) as an off-white solid.
C. Using General Procedure E with Compound 63 (2.6 g, 5.11 mmol) in AcOH (80 %, 25 mL), gave the desired dialcohol (1*R*,3a*S*,3b*S*,4*R*,5*R*,5a*S*,11a*R*,11b*S*,13a*R*)-8,11a,13a-trimethyl-1-((*R*)-6-methylheptan-2-yl)-2,3,3a,3b,4,5,5a,6,11,11a,11b,12,13,13a-tetradecahydro-1*H* cyclopenta[5,6]naphtho[1,2-*g*]quinazoline-4,5-diol (Compound Ic-1, 2.2 g, 92 %) as an off-white solid after purification by column chromatography on silica gel (230-400 mesh, 0-10 % dichloromethane / methanol).
   LCMS: (Method 1d) MS m/z: 469.4 (M+1), t_{R}: 2.584 min, Purity: 97.92 % (ELSD).
   HPLC: (Method 2a) t_{R}: 5.383 min, Purity: 93.51 % (UV).
   ¹H-NMR (400 MHz, CD₃OD): δ 8.38 (s, 1H), 3.34-3.10 (m, 3H), 2.85-2.81 (m, 1H), 2.63 (s, 3H), 2.44-2.40 (m, 1H), 2.14-1.65 (m, 5H), 1.60-1.41 (m, 7H), 1.31-1.06 (m, 10H), 1.02-0.98 (m, 3H), 0.92-0.90 (m, 6H), 0.82 (s, 3H), 0.77 (s, 3H).

### SYNTHETIC EXAMPLE 11.1

### Synthesis of (1R,3aS,3bS,4R,5R,5aS,11aR,11bS,13aR)-8-amino-11a,13a-dimethyl-1-((R)-6-methylheptan-2-yl)-2,3,3a,3b,4,5,5a,6,11,11a,11b, 12,13,13a-tetradecahydro-1H-cyclopenta[5,6]naphtho[1,2-g]quinazoline-4,5-diol (Compound Ic-2)

Following the procedure as described in Synthetic Example 11 and making non-critical variations using guanidine·HCl to replace acetamidine·HCl in the conversion of Compound 62 to Compound 63, the title compound (1*R*,3a*S*,3b*S*,4*R*,5*R*,5a*S*,11a*R*,11b*S*,13a*R*)-8-amino-11a,13a-dimethyl-1-((*R*)-6-methylheptan-2-yl)-2,3,3a,3b,4,5,5a,6,11,11a,11b,12,13,13a-tetradecahydro-1*H-*cyclopenta[5,6]naphtho[1,2-*g*]quinazoline-4,5-diol (Compound Ic-2, 0.40 g, 14 %) was obtained as an off white solid after purification by preparative HPLC (Method 3a). LCMS: (Method 1c) MS m/z: 470.3 (M+1), t_{R}: 1.675 min, Purity: 95.33 % (UV).

### SYNTHETIC EXAMPLE 11.2

### Synthesis of (1R,3aS,3bS,4R,5R,5aS,11aR,11bS,13aR)-11a,13a-dimethyl-1-((R)-6-methylheptan-2-yl)-2,3,3a,3b,4,5,5a,6,11,11a,11b,12,13,13a-tetradecahydro-1H-cyclopenta[5,6]naphtho[1,2-g]quinazoline-4,5-diol (Compound Ic-3)

Following the procedure as described in Synthetic Example 11 and making non-critical variations using formamidine·HCl to replace acetamidine·HCl in the conversion of Compound 62 to Compound 63, the title compound (1R,3aS,3bS,4R,5R,5aS,11aR,11bS,13aR)-11a,13a-dimethyl-1-((R)-6-methylheptan-2-yl)-2,3,3a,3b,4,5,5a,6,11,11a,11b,12,13,13a-tetradecahydro-1*H-*cyclopenta[5,6]naphtho[1,2-*g*]quinazoline-4,5-diol (Compound Ic-3, 2.3 g, 93 %) was obtained as a brown solid.
LCMS: (Method 1b) MS m/z: 455.4 (M+1), t_{R}: 5.828 min, Purity: 98.62 % (UV).
HPLC: (Method 2a) t_{R}: 5.997 min, Purity: 97.11 % (UV)
¹H-NMR (400 MHz, CD₃OD): δ 8.89 (s, 1H), 8.52 (s, 1H), 3.29-3.26 (m, 1H), 3.10 (t, *J* = 18.4 Hz, 1H), 2.88 (d, *J*= 16.5 Hz, 1H), 2.69-2.61 (m, 1H), 2.48 (d, *J*= 16.7 Hz, 1H), 2.13-2.10 (m, 1H), 2.03-1.95 (m, 1H), 1.92-1.83 (m, 1H), 1.79-1.64 (m, 2H), 1.60-1.50 (m, 3H), 1.41-1.40 (m, 3H), 1.34-1.13 (m, 8H).

### SYNTHETIC EXAMPLE 11.3

### Synthesis of (3aS,3bR,4R,5R,5aS,11aR,11bS,13aS)-8,11a,13a-trimethyl-1-methylene-2,3,3a,3b,4,5,5a,6,11,11a,11b,12,13,13a-tetradecahydro-1H-cyclopenta[5,6]naphtho[1,2-g]quinazoline-4,5-diol (Compound Ic-4)

Following the procedure as described in Synthetic Example 11 and making non-critical variations using (4a*R*,4b*S*,6a*S*,9a*S*,9b*R*,9c*R*,12a*R*,12b*S*)-3-(hydroxymethylene)-4a,6a,11,11-tetramethyl-7-methylenehexadecahydro-2*H-*cyclopenta[1,2]phenanthro[9,10-*d*][1,3]dioxol-2-one (Compound 64, as prepared in U.S. Pat. No. 9,765,085) to replace Compound 16 as the starting material, the title compound (3a*S*,3b*R*,4*R*,5*R*,5a*S*,11a*R*,11b*S*,13a*S*)-8,11a, 13a-trimethyl-1-methylene-2,3,3a,3b,4,5,5a,6,11,11a,11b,12,13,13a-tetradecahydro-1*H-*cyclopenta[5,6]naphtho[1,2-*g*]quinazoline-4,5-diol (Compound Ic-4, 29 mg, 4 %) was obtained as an off-white solid after purification by preparative HPLC (Method 3a). LCMS: (Method 1c) MS m/z: 369.2 (M+1), t_{R}: 2.254 min, Purity: 76.94 % (UV).
HPLC: (Method 2d) t_{R}: 8.105 min, Purity: 97.55 % (ELSD).
¹H-NMR (400 MHz, CD₃OD): δ 8.63 (s, 1H), 4.68 (d, *J*= 1.9 Hz, 2H), 3.40-3.38 (m, 1H), 3.17-2.93 (m, 2H), 2.74 (s, 3H), 2.53-2.18 (m, 4H), 1.92-1.59 (m, 6H), 1.35-1.11 (m, 5H), 1.01-0.82 (m, 6H).

### SYNTHETIC EXAMPLE 11.4

### Synthesis of (3aS,3bR,4R,5R,5aS,11aR,11bS,13aS)-11a,13a-dimethyl-1-methylene-2,3,3a,3b,4,5,5a,6,11,11a,11b,12,13,13a-tetradecahydro-1H-cyclopenta[5,6]naphtho[1,2-g]quinazoline-4,5-diol (Compound Ic-5)

Following the procedure as described in Synthetic Example 11 and making non-critical variations using a) Compound 64 (from Example 11.3) to replace Compound 16 as starting material and b) formamidine·HCl to replace acetamidine·HCl in the conversion of Compound 62 to Compound 63, the title compound (3aS,3bR,4R,5R,5aS, 11aR,11bS,13aS)-11a,13a-dimethyl-1-methylene-2,3,3a,3b,4,5,5a,6,11,11a,11b,12,13,13a-tetradecahydro-1*H-*cyclopenta[5,6]naphtho[1,2-g]quinazoline-4,5-diol (Compound Ic-5, 26 mg, 4 %) was obtained as an off-white solid after purification by preparative HPLC (Method 3a). LCMS: (Method 1e) MS m/z: 355.2 (M+1), t_{R}: 2.747 min, Purity: 90.30 % (UV). HPLC: (Method 2d) t_{R}: 9.650 min, Purity: 85.17 % (UV).
¹H-NMR (400 MHz, CD₃OD): δ 8.88 (s, 1H), 8.51 (s, 1H), 4.68 (d, *J*= 2.4 Hz, 2H), 3.29-3.12 (m, 3H), 2.92-2.88 (m, 1H), 2.70-2.49 (m, 3H), 2.29-2.06 (m, 2H), 1.95-1.59 (m, 6H), 1.32-1.10 (m, 3H), 0.86 (s, 6H).

### SYNTHETIC EXAMPLE 11.5

### Synthesis of (3aS,3bR,4R,5R,5aS,11aR,11bS,13aS)-8-amino-11a,13a-dimethyl-1-methylene-2,3,3a,3b,4,5,5a,6,11,11a,11b,12,13,13a-tetradecahydro-1H cyclopenta[5,6]naphtho[1,2-g]quinazoline-4,5-diol (Compound Ic-6)

Following the procedure as described in Synthetic Example 11 and making non-critical variations using a) Compound 64 (from Example 11.3) to replace Compound 16 as starting material and b) guanidine·HCl to replace acetamidine·HCl in the conversion of Compound 62 to Compound 63, the title compound (3a*S*,3b*R*,4*R*,5*R*,5a*S*,11a*R*,11b*S*,13a*S*)-8-amino-11a, 13a-dimethyl-1 -methylene-2,3,3a,3b,4,5,5a,6,11,11a,11b,12,13,13a-tetradecahydro-1*H-*cyclopenta[5,6]naphtho[1,2-g]quinazoline-4,5-diol (Compound Ic-6) was obtained.

LCMS: (Method 1c) MS m/z: 370.2 (M+1), t_{R}: 1.807 min, Purity: 90.55 % (UV).

### SYNTHETIC EXAMPLE 12

### Synthesis of (1R,3aS,3bS,4R,5R,5aS,10aR,10bS,12aR)-10a,12a-dimethy)-1-((R)-6-methylheptan-2-yl)-2,3,3a,3b,4,5,5a,6,10,10a, 10b, 11,12,12a-tetradecahydro-1H-cyclopenta[7,8]phenanthro[2,3-d][1,2,3]thiadiazole-4,5-diol (Compound Id-1)

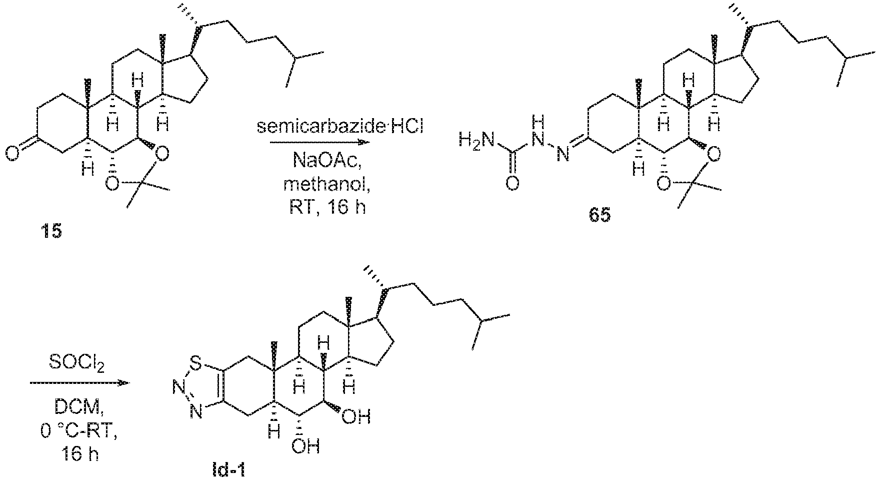

A. To a stirred solution of sodium acetate (1.61 g, 19.62 mmol) and semicarbazide·HCl (1.75 g, 15.70 mmol) in MeOH (10 mL) was added at room temperature Compound 15 (from Example 2, 6.0 g, 13.08 mmol) in MeOH (50 mL) dropwise. The reaction mixture was stirred at room temperature for 16 hours. The mixture was evaporated under reduced pressure and the residue was diluted with EtOAc (2 x 60 mL) and washed consecutively with water (1 x 60 mL) and brine (1 x 60 mL). The organic layer was dried over Na₂SO₄, filtered and concentrated. The crude was purified by column chromatography on silica gel (230-400 mesh, 40-50 % pet ether / ethyl acetate) to afford 2-((4a*R*,4b*S*,6a*R*,7*R*,9a*S*,9b*S*,9c*R*,12a*R*,12b*S*)-4a,6a,11,11-tetramethyl-7-((*R*)-6-methylheptan-2-yl)hexadecahydro-2*H-*cyclopenta[1,2]phenanthro[9,10-*d*][1,3]dioxol-2-ylidene)hydrazine-1-carboxamide (Compound 65, 6.5 g, 96 %) as a white gummy solid.
B. To a stirred solution of Compound 65 (6.5 g, 12.60 mmol) in CH₂Cl₂ (60 mL) at 0 °C was added thionyl chloride (18.28 mL, 252.05 mmol) dropwise. The reaction mixture was stirred at room temperature for 16 hours. The mixture was evaporated under reduced pressure and the residue was diluted with a saturated aqueous NaHCO₃ solution (1 x 60 mL), extracted with CH₂Cl₂ (2 x 60 mL) and washed consecutively with water (1 x 60 mL) and brine (1 x 60 mL). The organic layer was dried over Na₂SO₄, filtered and concentrated. The crude was purified by column chromatography on silica gel (230-400 mesh, 40-50 % pet ether / ethyl acetate) to afford (1*R*,3a*S*,3b*S*,4*R*,5*R*,5a*S*, 10a*R*, 10b*S*,12a*R*)-10a,12a-dimethyl-1-((R)-6-methylheptan-2-yl)-2,3,3a,3b,4,5,5a,6,10,10a,10b,11,12,12a-tetradecahydro-1*H-*cyclopenta[7,8]phenanthro[2,3-*d*][1,2,3]thiadiazole-4,5-diol (Compound Id-1, 4.0 g, 69 %) as a white solid.
   LCMS: (Method 1f) MS m/z: 461.4 (M+1), t_{R}: 4.567min, Purity: 98.74 % (UV).
   HPLC: (Method 2d) t_{R}: 20.953 min, Purity: 95.26 % (UV).
   ¹H-NMR (400 MHz, DMSO-d6): δ 3.66 (dd, *J*= 5.0, 17.1 Hz, 1H), 3.38-3.36 (m, 1H), 3.24 (d, *J*= 17.2 Hz, 1H), 3.11 (t, *J*= 9.4 Hz, 1H), 2.76-2.69 (m, 1H), 2.56 (d, *J* = 17.2 Hz, 1H), 2.11 (d, *J*= 12.6 Hz, 1H), 2.02-1.98 (m, 1H), 1.88-1.85 (m, 1H), 1.71-1.62 (m, 2H), 1.60-1.48 (m, 4H), 1.40-1.35 (m, 3H), 1.34-1.06 (m, 9H), 1.03-0.98 (m, 3H), 0.91-0.89 (m, 6H), 0.85 (s, 3H), 0.76 (s, 3H).

### SYNTHETIC EXAMPLE 13

### Synthesis of (1R,3aS,3bS,4R,5R,5aS,10aR,10bS,12aR)-10a,12a-dimethyl-1-((R)-6-methylheptan-2-yl)-2,3,3a,3b,4,5,5a,6,10,10a,10b,11,12,12a-tetradecahydro-1H-cyclopenta[7,8]phenanthro[3,2-d]isoxazole-4,5-diol (Compound le-1)

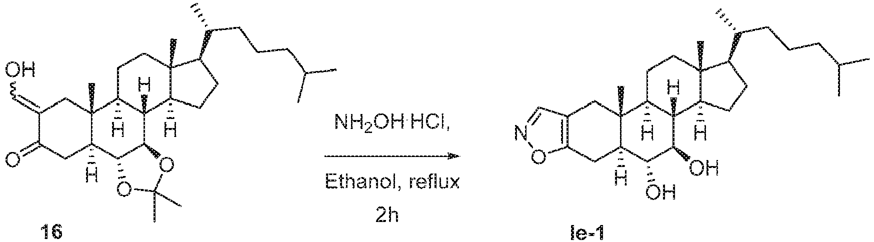

To a stirred solution of Compound 16 (from Example 2, 2.0 g, 4.10 mmol) in ethanol (20 mL) was added hydroxylamine hydrochloride (0.42 g, 6.04 mmol) at room temperature. The resultant solution was heated to reflux for 2 hours. The reaction mixture was evaporated under reduced pressure. The residue was diluted with EtOAc (40 mL) and washed consecutively with water (1 x 20 mL) and brine (1 x 20 mL). The organic layer was dried over Na₂SO₄, filtered and concentrated to give the desired isoxazole (1*R*,3a*S*,3b*S*,4*R*,5*R*,5a*S*,10a*R*,10b*S*,12a*R*)-10a,12a-dimethyl-1-((*R*)-6-methylheptan-2-yl)-2,3,3a,3b,4,5,5a,6,10,10a,10b,11,12,12a-tetradecahydro-1*H-*cyclopenta[7,8]phenanthro[3,2-*d*]isoxazole-4,5-diol (Compound le-1, 0.88 g, 48 %) as a pale yellow liquid after purification by column chromatography (230-400 mesh silica gel, eluted with 80-95 % pet ether / ethyl acetate).
LCMS: (Method 1c) MS m/z: 444.3 (M+1), t_{R}: 3.738 min, Purity: 88.84 % (UV).
HPLC (Method 2a) t_{R}: 6.949 min, Purity: 94.10 % (UV)
¹H-NMR (400 MHz, CD₃OD): δ 8.18 (s, 1H), 3.18-3.05 (m, 2H), 2.56-2.52 (m, 1H), 2.41-2.34 (m, 1H), 2.17-2.09 (m, 2H), 2.04-1.96 (m, 1H), 1.93-1.86 (m, 1H), 1.66-1.64 (m, 1H), 1.59-1.47 (m, 3H), 1.45-1.38 (m, 3H), 1.35-1.29 (m, 3H), 1.27-1.10 (m, 9H), 1.02-0.97 (m, 3H), 0.91 (dd, *J*= 1.40, 6.6 Hz, 6H), 0.83 (s, 3H), 0.76 (s, 3H).

### SYNTHETIC EXAMPLE 14

### Synthesis of (3aS,3bR,4R,5R,5aS,10aR,10bS,12aS)-8,10a,12a-trimethyl-1-methylene-2,3,3a,3b,4,5,5a,6,10,10a,10b,11,12,12a-tetradecahydro-1H-cyclopenta[7,8]phenanthro[3,2-d]oxazole-4,5-diol (Compound If-1)

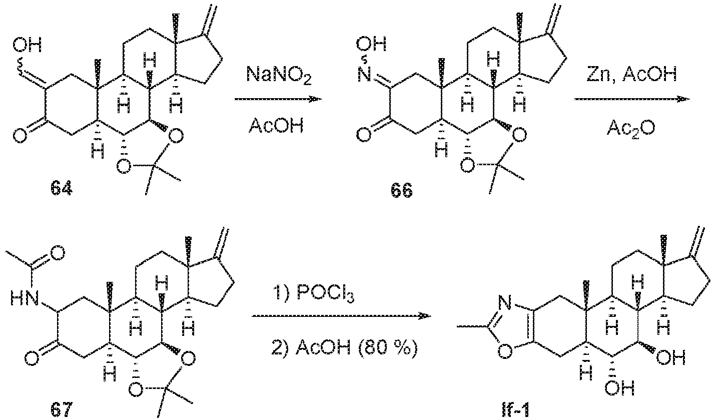

The title compound (3a*S*,3b*R*,4*R*,5*R*,5a*S*,10a*R*,10b*S*,12a*S*)-8,10a,12a-trimethyl-1 -methylene-2,3,3a,3b,4,5,5a,6,10,10a, 10b, 11,12,12a-tetradecahydro-1*H-*cyclopenta[7,8]phenanthro[3,2-*d*]oxazole-4,5-diol (Compound If-1) was obtained using Compound 64 (from Example 11.3), followed by oxime formation to obtain Compound 66, oxime reduction and acetylation to obtain Compound 67, and finally cyclization followed by deprotection.
LCMS: (Method 1c) MS m/z: 358.2 (M+1), t_{R}: 2.410 min, Purity: 93.86 % (UV).

### SYNTHETIC EXAMPLE 15

### Synthesis of (1R,3aS,3bS,4R,5R,5aS,10aR,10bS,12aR)-8-amino-10a,12a-dimethyl-1-((R)-6-methylheptan-2-yl)-2,3,3a,3b,4,5,5a,6,10,10a,10b,11,12,12a-tetradecahydro-1H-cyclopenta[7,8]phenanthro[2,3-d]thiazole-4,5-diol (Compound lg-1)

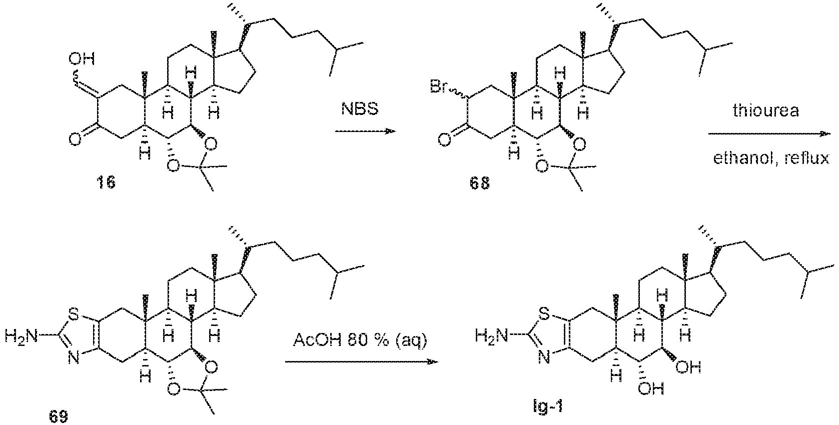

The title compound (1*R*,3a*S*,3b*S*,4*R*,5*R*,5a*S*,10a*R*,10b*S*,12a*R*)-8-amino-10a,12a-dimethyl-1-((*R*)-6-methylheptan-2-yl)-2,3,3a,3b,4,5,5a,6,10,10a,10b,11,12,12a-tetradecahydro-1*H-*cyclopenta[7,8]phenanthro[2,3-*d*]thiazole-4,5-diol (Compound Ig-1) was obtained from NBS treatment of Compound 16 (from Example 2) to obtain Compound 68, followed by thiourea addition and cyclization to obtain Compound 69, and finally deprotection. LCMS: (Method 1c) MS m/z: 475.3 (M+1), t_{R}: 3.115 min, Purity: 99.07 % (UV).

### SYNTHETIC EXAMPLE 16

### Synthesis of (5aS,5bR,6R,7R,7aS,12aR,12bS,14aS)-2-chloro-12a,14a-dimethyl-5,5a,5b,6,7,7a,8,9,12,12a,12b,13,14,14a-tetradecahydropyrido[2",3":3',4']cyclo-penta[1',2':5,6]naphtho[1,2-f]indazole-6,7-diol (Compound 1h-1) and (5aS,5bR,6R,7R,7aS,12aR,12bS,14aS)-12a,14a-dimethyl-5,5a,5b,6,7,7a,8,9,12,12a,12b,13,14,14a-tetradecahydropyrido[2",3":3',4']cyclo-penta[1',2':5,6]naphtho[1,2-f]indazole-6,7-diol (Compound 1h-2)

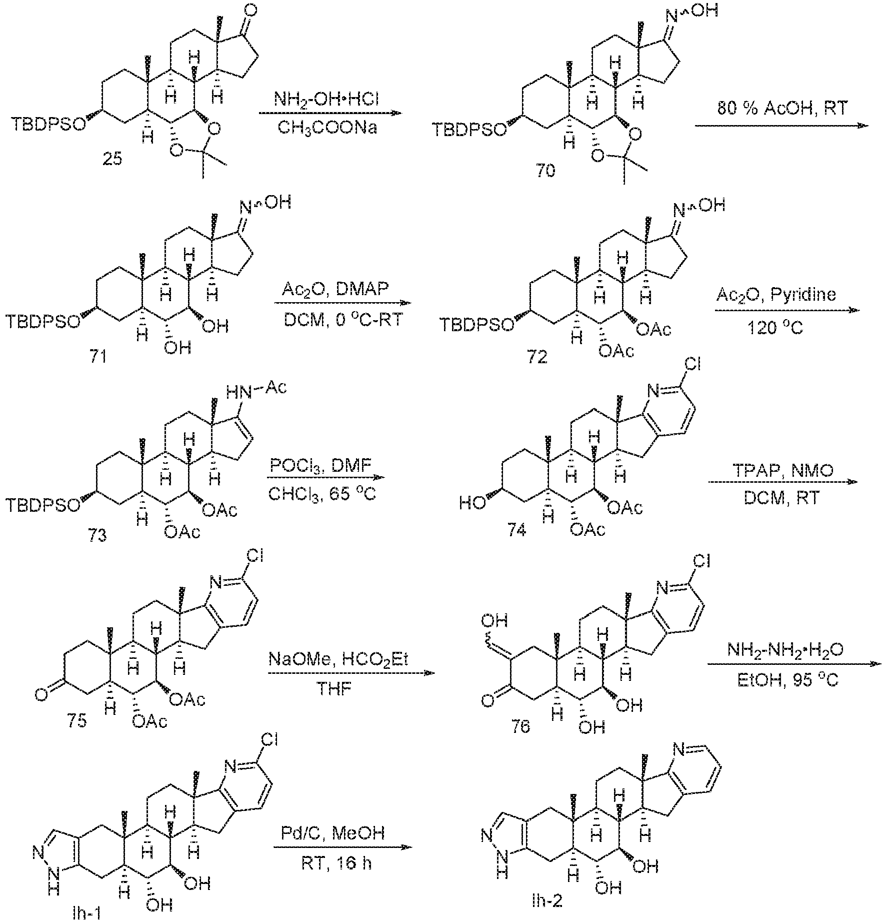

A. Compound 25 (as prepared in U.S. Pat. No. 9,765,085) was converted to (2S,4aR,4bS,6aS,9aS,9bR,9cR,12aR,12bS)-2-((*tert*-butyldiphenylsilyl)oxy)-4a,6a,11,11-tetramethylhexadecahydro-7*H*-cyclopenta[1,2]phenanthro[9,10-*d*][1,3]dioxol-7-one oxime (Compound 70) through oxime formation.
B. Compound 70 was deprotected under acidic conditions to form (3*S*,5*S*,6*R*,7*R*,8*R*,9*S*,10*R*,13*S*,14*S*)-3-((*tert-*butyldiphenylsilyl)oxy)-6,7-dihydroxy-10,13-dimethylhexadecahydro-17*H*-cyclopenta[a]phenanthren-17-one oxime (Compound 71).
C. (3*S*,5*S*,6*R*,7*R*,8*R*,9*S*,10*R*,13*S*,14*S*)-3-((*tert-*butyldiphenylsilyl)oxy)-17-(hydroxylmino)-10,13-dimethylhexadecahydro-1*H*-cyclopenta[a]phenanthrene-6,7-diyl diacetate (Compound 72) was prepared by acetylating Compound 71.
D. Compound 72 was converted to (3*S*,5*S*,6*R*,7*R*,8*R*,9*S*,10*R*,13*S*,14*S*)-17-acetamido-3-((*tert*-butyidiphenylsilyl)oxy)-10,13-dimethyl-2,3,4,5,6,7,8,9,10,11,12,13,14,15-tetradecahydro-1*H*-cyclopenta[a]phenanthrene-6,7-diyl diacetate (Compound 73) using acetic anhydride under basic conditions.
E. Compound 73 was treated with POCl₃ which cyclized to form a chloropyridine, while the TBDPS alcohol protecting group was simultaneously removed to form (1*R*,2*R*,2a*S*,4*S*,6a*R*,6b*S*,8a*S*,13a*S*,13b*R*)-10-chloro-4-hydroxy-6a,8a-dimethyl-2,2a,3,4,5,6,6a,6b,7,8,8a,13,13a,13b-tetradecahydro-1*H*-naphtho[2',1':4,5]indeno[1,2-b]pyridine-1,2-diyl diacetate (Compound 74).
F. Compound 74 was oxidized to form the ketone, (1*R*,2*R*,2a*S*,6a*R*,6b*S*,8a*S*,13a*S*,13b*R*)-10-chloro-6a,8a-dimethyl-4-oxo-2,2a,3,4,5,6,6a,6b,7,8,8a,13,13a,13b-tetradecahydro-1*H*-naphtho[2',1':4,5]indeno[1,2-b]pyridine-1,2-diyl diacetate (Compound 75).
G. Compound 75 underwent a Knoevenagel condensation to form (1*R*,2*R*,2a*S*,6a*R*,6b*S*,8a*S*,13a*S*,13b*R*)-10-chloro-1,2-dihydroxy-5-(hydroxymethylene)-6a,8a-dimethyl-1,2,2a,3,5,6,6a,6b,7,8,8a,13,13a,13b-tetradecahydro-4H-naphtho[2',1':4,5]indeno[1,2-b]pyridin-4-one (Compound 76).
H. The title compound, (5a*S*,5b*R*,6*R*,7*R*,7a*S*,12a*R*,12b*S*,14a*S*)-2-chloro-12a,14a-dimethyl-5,5a,5b,6,7,7a,8,9,12,12a,12b,13,14,14a-tetradecahydropyrido[2",3":3',4']cyGopenta[1',2':5,6]naphtho[1,2-*f*]indazole-6,7-diol (Compound 77), was obtained from the treatment of Compound 76 with hydrazine.
   LCMS: (Method 1e) MS m/z: 414.2 (M+1), t_{R}: 2.682 min, Purity: 76.30 % (UV)
   ¹H-NMR (400 MHz, DMSO-*d*6): δ 12.20 (broad s, 1H), 7.66 (d, *J* = 7.8 Hz, 1H), 7.19 (d, *J*= 7.8 Hz, 2H), 4.68 (d, *J*= 3.8 Hz, 1H), 4.60 (d, *J* = 4.4 Hz, 1H), 3.40 (m, 1H), 3.17-2.92 (m, 4H), 2.08-2.01 (m, 3H), 1.92-1.59 (m, 3H), 1.40-1.35 (m, 2H), 1.10-0.87 (m, 5H), 0.61-0.57 (m, 1H), 0.39 (s, 3H).
I. The title compound, (5a*S*,5b*R*,6*R*,7*R*,7a*S*,12a*R*,12b*S*,14a*S*)-12a,14a-dimethyl-5,5a,5b,6,7,7a,8,9,12,12a,12b,13,14,14a-tetradecahydropyrido[2",3":3',4']cyclopenta[1',2':5,6]naphtho[1,2-*f*]indazole-6,7-diol (Compound 78) was obtained from the reduction of Compound 77.
LCMS: (Method 1e) MS m/z: 380.2 (M+1), t_{R}: 2.140 min, Purity: 97.24 % (UV)
HPLC: (Method 2e) t_{R}: 4.135 min, Purity: 99.95 % (UV).
¹H-NMR (400 MHz, MeOH-d4): δ 8.29 (d, *J=* 5.2 Hz, 1H), 7.65 (d, *J=* 7.2 Hz, 1H), 7.28 (s, 1H), 7.16-7.19 (m, 1H), 3.47 (d, *J*= 16.8 Hz, 1H), 3.23-3.35 (m, 3H), 3.11 (dd, *J*= 5.2, 16.4 Hz, 1H), 2.60-2.66 (m, 2H), 2.13-2.28 (m, 3H), 1.74 (d, *J* = 12.0 Hz, 1H), 1.75-1.59 (m, 2H), 1.11-1.20 (m, 5H), 0.82 (q, *J*= 13.2 Hz, 1H), 0.52 (s, 3H).

### BIOLOGICAL EXAMPLE 1

### Rat Dorsal Root Ganglion Excitability Response of Representative Compounds

Dorsal root ganglions (DRGs) are dissected from adult rats. The tissue is processed, and cells are seeded into cell culture plates (48 center wells of one quadrant in 384-well plates) and cultured for 2 days prior to Electrical field stimulation (EFS). To visualize the neuronal excitability response, intracellular Ca⁺² transients are monitored using the Ca⁺² indicator, Ca5. Ca5 is added to the cultures 1 h prior to EFS. Representative compounds of the invention (*i.e*., test compounds) and the reference compound, tetracaine, can be added in concentration-response format encompassing six concentrations, performed in duplicate. The highest concentration tested is typically 30 µM with 1 in 3 dilutions generating tthe subsequent concentrations.

Effects on DRG excitability by the test compounds and standard are evaluated using four EFS protocols as disclosed in Table 2.

**TABLE 2: EFS PROTOCOL PARAMETERS**

| Protocol # | Voltage (V) | Frequency (Hz) | Pulse duration (ms) | # of pulses |
|---|---|---|---|---|
| 1 | 20 | 5 | 0.3 | 25 |
| 2 | 20 | 30 | 0.3 | 150 |
| 3 | 30 | 5 | 0.3 | 25 |
| 4 | 30 | 30 | 0.3 | 150 |

Experimental results are performed using two seperate sets of plates using separate test compound and standard dilutions, to provide n=2. In the first set of experiments, compounds are added 24 h prior to EFS to increase the possibility to also detect more long-term compound effects. In the second set of experiments the compounds are added directly prior to EFS. Excitability response to EFS is analyzed as the average fluorescence ratio (peak / baseline) change per well. All plates are evaluated using high content imaging equipment post-EFS, imaging the Ca5 background staining to detect any possibile compound-related toxic effects.

Compounds of the invention may be tested in this assay to determine their ability to modulate neuronal excitability.

### BIOLOGICAL EXAMPLE 2

### T Cell Proliferation and Cytokine Release Activity of Representative Compounds

Spleens are obtained from six male, CD-1 outbred mice, approximately 8 weeks old. Cells are harvested under sterile conditions by forcing each spleen though a cell filter (pore size 100 µm diameter). A homogenous cell suspension is obtained by washing the cells in fresh medium and passing cells through a smaller cell filter (pore size 70 µm).

Untouched T cells are isolated using the Pan T cell isolation kit (Miltenyi Biotech). Briefly, cells are counted and incubated with the required volume of antibody cocktail for 5 minutes at 4 °C before addition of microbeads and incubation for 10 minutes at 4°C. Labeled cells are retained in a magnetic column while unlabeled cells (T cells) are passed through the column and are retained for use in the assay.

Cell viability is assessed by Trypan Blue exclusion and found to be >99 % prior to transfer to plates. Isolated T cells are seeded out in 96 well plates at a density of 50,000 cells per well and allowed to incubate for 60 minutes in a humidified cell culture (37°C, 5 % CO₂) incubator prior to compound treatment. The media used for this assay (TexMACS, Miltenyi Biotech) is previously optimized for proliferation of T cells in serum free conditions.

Representative compounds of the invention (i.e., test compounds) are prepared as 30 mM stocks in 100 % DMSO. The compounds are initially diluted in media to yield 3 mM stocks before an additional 1:10 dilution in media is performed to yield working stocks of 300 µM (1 % DMSO). Subsequent dilutions are performed in media (supplemented with 1 % DMSO). When added to the assay plates (1:10 dilution), these yield final concentrations in the assay plates of in 0.1 % DMSO. The reference compound, Cyclosporin A, is prepared in an identical manner as above.

Unstimulated and Stimulated Control wells receive an identical volume of TexMACS media/1 % DMSO at this time, resulting in a final concentration of 0.1 % DMSO across the plate. Mouse anti-CD3/anti-CD28 dynabeads are prepared (following the manufacturers' instructions) in TexMACS media and added to appropriate wells to achieve a final concentration of 1 bead per cell. Unstimulated Control wells then receive an identical volume of TexMACS. Plates are centrifuged 72 hours after addition of dynabeads, (300 x g for 3 minutes) to pellet the cells and 60 % of the supernatant is removed to a fresh plate for analysis by ELISA. After removal of 60 % of the supernatant for subsequent ELISA, cell proliferation is assessed using the CCK-8 assay.

Compounds of the invention may be tested in this assay to determine their antiinflammatory activity and their ability to inhibit T cell activation.

### BIOLOGICAL EXAMPLE 3

### Human Dorsal Root Ganglion (DRG) Excitability Response of Representative Compounds

Human DRGs are transferred into a dissection vessel containing a cold (4 °C), fresh proprietary dissection solution. DRGs are maintained completely submerged in dissection solution followed by dissection by an appropriate method.

Cells are plated into a 96 well plate. Calcium dye (Fluo 8-AM) is loaded in each well for a period of 20 to 25 min, maintaining temperature at ambient. The baseline excitability profile (ability of firing action potentials) of the cells is assessed at both low and high threshold stimulations using optical EFS. Following baseline profiling, cells are subjected to test compounds.

Representative compounds of the invention (*i.e*., test compounds) are added to cells and the cells are stimulated (using EFS) to induce action potentials at regular intervals, according to the parameters outlined in TABLE 3. Four concentrations of each compound are utilized and directly injected into separate wells to allow for determination of a dose response (IC₅₀). At the end of the protocol, the nociceptor positive control compound (capsaicin) is perfused into the cells at 200 nM and signal recorded. TTX (300 nM) is also examined using this protocol in a separate well.

**TABLE 3: EFS PROTOCOL AND RECORDING SEQUENCE**

| **Action** | **Parameter / Detail** |
|---|---|
| Baseline Low Voltage | 2 s recording with stimulation (5 Hz) |
| Rest | 3 min |
| Baseline High Voltage | 2 s recording with stimulation (5 Hz) |
| Preincubation of Test compounds | 5 min |
| Test Compound Low Votage Signal | 2 s recording with stimulation (5 Hz) |
| Rest | 3 min |
| Test Compound High Votage Signal | 2 s recording with stimulation (5 Hz) |
| Washout | 5 min |
| Capsaicin | 20s (recording 3 min) |

Recordings are performed in stream mode aty 100 Hz for the EFS portion of the above protocol and in time lapse model at 0.2 Hz for the final step utilizing capsaicin. For each concentration tested, the number of cells blocked vs baseline will be counted at different thresholds.

Compounds of the invention may be tested in this assay to determine their ability to modulate human DRG excitability.

### BIOLOGICAL EXAMPLE 4

### Pulmonary Lipopolysaccharide (LPS) Challenge Assay of Representative Compounds in Mice

Mice (male, C57BI/6) are acclimatized for a period of about 7 days before initiation of the experiment and are randomized on the day prior to treatment. Mice receive vehicle or a representative compound of the invention (*i.e.*, test compound) once daily for three days by oral gavage on Day -2, Day -1 and on Day 0 (the last dose being 1 h prior to LPS administration). One group of mice receieve the reference standard, dexamethasone, once, IP on Day 0 at 1 h prior to LPS administration. Pulmonary inflammation is induced in all animals except the sham control animals by intratracheal instillation of 20 µg LPS per animal in 50 µl saline. Sham control animals receive 50 µl saline alone.

Twenty-four hours post LPS administration, animals are euthanized and the trachea is cannulated. Cold Hanks Balanced Salt Solution (SIGMA; Catalogue number: H1387), pH 7.2, is infused into the lungs and bronchoalveolar lavage fluid (BALF) is collected.

Total leukocyte counts are performed from the collected BALF using a mini flow-cytometer and differential counts are performed in cytospin smears stained with Leishman's staining manually. ELISA kits are used for quantification of cytokines in the BALF (TNFα, IL-1β, IL-6 and KC). Reagents, samples and standards are prepared as per kit manual. Total protein analysis in BALF samples is performed using the Biorad protein assay reagent.

Compounds of the invention may be tested in this assay to determine their ability to modulate inflammatory markers in BALF, indicating their efficacy against LPS-induced inflammation.

### BIOLOGICAL EXAMPLE 5

### Formalin Pain Assay of Representative Compounds in Mice

Mice (male, C57BU6) are placed singly in a Perspex chamber for approximately 30 min on three successive days to acclimate and thereby reduce stress-induced behaviors. On the fourth day, the experimental animals receive a 25 µl injection of 2.5 % formalin beneath the left plantar skin using a 29-gauge syringe. Animals are administered vehicle, reference standard Tramadol or a representative compound of the invention (i.e., test compound) prior to formalin injection. The total time spent on flinches/licking/biting of the hind paw is recorded by visual observation for every 5 min period/interval for total observation duration of 60 min in two phases, the early phase (0-5 min) and the late phase (15-40 min). Observers are blinded to the treatment group allocation.

Compounds of the invention may be tested in this assay to determine their ability to alleviate nociceptive pain and/or inflammatory pain.

### BIOLOGICAL EXAMPLE 6

### TNBS Colitis Assay of Representative Compounds in Rats

Rats (male, Sprague-Dawley) are anaesthetized and a solution of TNBS (48 mg/kg) in ethanol, is instilled intra-rectally to induce colitis, 1 h after oral dosing of representative compounds of the invention (*i.e*., test compounds). Test compounds are dosed PO (by mouth, *per os*), QD (once of day, *quaque die*) for 7 days, using prednisolone as a reference standard. Rats are observed for body weight loss and fecal output. On Day 7, rats are euthanized and the colon are evaluated for length, weight, wall thickness, ulcer number and length and for the presence of adhesions and strictures. A colonic score is calculated based on the severity of the colonic parameters.

Compounds of the invention may be tested in this assay to determine their ability to modulate colonic parameters, indicating their efficacy against colitis.

### BIOLOGICAL EXAMPLE 7

### Cyclophosphamide-induced Cystitis in rats (Visceral Pain)

Representative compounds of the invention (i.e., test compounds) are administered to female Sprague-Dawley rats, for four days by oral gavage in 0.9% saline. Two hours after the fourth dose, the rats are challenged by intraperitoneal administration of cyclophosphamide (150 mg/kg). Referred mechanical sensitivity is measured four hours later by applying a series of eight von Frey filaments to the lower abdomen, three times each for 1-2 seconds with a 5 second interval between applications. Responses are scored (zero-no response; one-response, two-response and change of position, and three-response, change of position and licking the site or vocalization), totaled and the percent of maximal possible nociceptive score calculated. Ibuprofen is used as a reference standard.

Compounds of the invention may be tested in this assay to determine their ability to modulate nociceptive pain, indicating efficacy against cystitis.

### BIOLOGICAL EXAMPLE 8

### Rat Ketamine Cystitis (Visceral Pain)

Rats (female, Sprague-Dawley) receive daily intraperitoneal injections of saline (sham control) or ketamine (50 mg/kg) for 14 days. Representative compounds of the invention (i.e., test compounds) are administered PO, QD starting on Day 0 at doses of 10, 3 or 1 mg/kg and Tramadol is used as a reference control compound at 10 mg/kg. Referred mechanical sensitivity is assessed on Day 7 and day 14 by application of a series of von Frey filaments to the lower abdomen and the nociceptive threshold was scored.

Compounds of the invention may be tested in this assay to determine their ability to modulate nociceptive pain and/or inflammation, indicating efficacy against cystitis.

### BIOLOGICAL EXAMPLE 9

### Rat Chronic Prostatitis / Chronic Pelvic Pain (Pelvic Pain)

Representative compounds of the invention (i.e., test compounds) are administered for 11 days by oral gavage in 0.9% saline (3, 10 or 30 mg/kg QD; 5 mL/kg dose volume) to male Sprague-Dawley rats. Carrageenan-mediated chronic prostatitis/chronic pelvic pain (CP/CPPS) is established in rats (10 per group) by administration of an intraprostatic injection of carrageenan (~12.5 µL/lobe of a 30 mg/mL solution) into both ventral prostate lobes, on Day 0. Referred mechanical sensitivity is measured on Days 0, 1, 3 and 7, 2 hours after test compound or vehicle (saline) administration, by applying 6 von Frey filaments with increasing forces of 0.16-2 g to the scrotal skin area, 3 times each for 1-2 seconds, with a 5-second interval between applications. Responses are scored (zero-no response; one-reaction of the animal, two-jump, and three-licking the site) and expressed as the nociceptive threshold for each day of assessment. Ibuprofen is used as a reference standard.

Compounds of the invention may be tested in this assay to determine their ability to modulate nociceptive pain, indicating their efficacy against chronic prostatitis / chronic pelvic pain.

### BIOLOGICAL EXAMPLE 10

### Rat Monosodium lodoacetate-Induced Osteoarthritis (Osteoarthritis/Inflammatory Pain)

Representative compounds of the invention (*i.e.*, test compounds) are administered to rats (male, Wistar) PO, QD, at 3 or 30 mg/kg, starting on Day 0 through to Day 21 of the study. Osteoarthritis is modeled by intra-articular injection of 3 mg of monosodium iodoacetate (MIA) in the right knee. Knee swelling, paw withdrawal threshold (mechanical allodynia) and the difference in weight bearing are measured on Day 0 (pre-induction) and on Days 3, 7, 14 and 21 post-MIA injection. Tramadol is used as a reference standard.

Compounds of the invention may be tested in this assay to determine their ability to modulate knee swelling, paw withdrawal threshold and difference in weight bearing, indicating efficacy against osteoarthritis.

### BIOLOGICAL EXAMPLE 11

### Complete Freund's Adjuvant (CFA) Model of Inflammatory Pain in Rats (Inflammatory Pain)

Representative compounds of the invention (*i.e*., test compounds) are tested in this model of inflammatory pain. The rats (male, Wistar) are acclimatized to the instruments (plantar test surface [Hargreaves' method] and dynamic plantar aesthesiometer) on two consecutive days prior to the initiation of the study. On Day 0, basal paw withdrawal latency and paw withdrawal threshold are taken and animals are randomized to different groups. Complete Freund's Adjuvant (CFA) at 1 mg/mL (0.1 mL) was injected into the plantar surface of right hind paw. Test compounds (3 or 30 mg/kg) are administered PO, twice (Day 0 and Day 1) or once, on Day 1 (24 h after CFA). Thermal hyperalgesia and mechanical allodynia are assessed at 0 (baseline), 1, 3 and 6 h after administration of the test compound on Day 1. Diclofenac is used as a reference compound, given once on Day 1.

Compounds of the invention may be tested in this assay to determine their ability to modulate inflammatory pain.

### BIOLOGICAL EXAMPLE 12

### Rat Carrageenan-Induced Hyperalgesia and Paw Edema (Inflammatory Pain)

Representative compounds of the invention (*i.e*., test compounds) are administered PO, QD, for 3 days prior to carrageenan injection, once at 1 h prior to carrageen, or at 5 min or 1 h post-challenge to rats (male, Sprague-Dawley). Rats receive either an intraplantar injection of saline (sham) or carrageen (0.1 mL of a 2% solution [w/v]) in the right hind paw. Mechanical hyperalgesia (using an analgesymeter) and paw volume (using a digital plethysmometer) are measured at baseline (0 hr), 2, 4 and 6 h post carrageenan injection for all the animals. Celecoxib, given 1 h prior to carrageen challenge, is used as a reference standard.

Compounds of the invention may be tested in this assay to determine their ability to modulate mechanical hyperalgesia and paw volume, indicating their efficacy against hyperalgesia.

### BIOLOGICAL EXAMPLE 13

### Spinal Nerve Ligation (Neuropathic Pain)

Rats (male, Sprague-Dawley) are anaesthetized and placed in a prone position and the left paraspinal muscles are separated from the spinous processes at the L6-S2 levels. The L6 transverse process is carefully removed to visually identify the L4-L6 spinal nerves. The left L5 spinal nerves are isolated and tightly ligated with 6-0 silk thread. The skin is sutured to close the open tissue and animals are allowed to recover for 1 week before pain assessment. Basal readings for mechanical allodynia and thermal hyperalgesia are performed using a dynamic plantar aesthesiometer and plantar test surface, respectively, before surgery. On Day 7 following surgery, mechanical allodynia and thermal hyperalgesia are assessed and animals are randomized into treatment groups based on these baseline readings. From Day 7 to Day 14, the rats either receive vehicle, tramadol (reference standard) or representative compounds of the invention (*i.e.*, test compounds). Thermal hyperalgesia and mechanical allodynia are tested at 0, 60 and 120 minutes post administration of compounds on Days 7 and 14.

Compounds of the invention may be tested in this assay to determine their ability to modulate neuropathic pain.

### BIOLOGICAL EXAMPLE 14

### Bleomycin Lung Fibrosis

Mice (male, C57BL/6) are randomized into treatment groups and fibrosis is induced by intratracheal administration of bleomycin. Treatment with representative compounds of the invention (i.e., test compounds) or reference standard (pirfenidone) is initiated on Day -1 and administered daily unto Day 7 or Day 21. For groups undergoing bronchoalveolar lavage (BAL), the trachea is cannulated and infused with ice cold Hank's Balanced Salt Solution (HBSS, pH 7.2). The collected lavage fluid is analyzed for cell numbers (total and differential counts) and levels of soluble TGFβ and collagen. For other groups, the lungs are either snap-frozen or formalin-fixed for further analyses.

Compounds of the invention may be tested in this assay to determine their ability to modulate pulmonary inflammation and fibrosis.

### BIOLOGICAL EXAMPLE 15

### Metabolism by Human Liver Microsomes

Representative compounds of the invention (i.e., test compounds) and the positive control, 7-ethoxycoumarin, are prepared as stock solutions at 10mM in DMSO and eventually diluted to 10 µM in the test plate, with an appropriate amount of acetonitrile and tris HCl buffer. Final DMSO and acetonitrile concentrations are 0.01 % and 0.5 % respectively. NADPH is prepared in tris HCl buffer to a stock of 10 mM. A frozen aliquot of live rmicrosomes is retrieved from the freezer (-80 °C) and thawed by placing the tube on wet ice. After thawing, the tubes are gently mixed and the required amount transferred to tris HCl buffer. Test compounds and the positive control are preincubated, separately, for 10 minutes, with liver microsomes (1 mg/mL protein), at 37 °C, in 100 mM Tris HCl at pH 7.5. After preincubation the reaction is started by adding 1 mM NADPH (pre-equilibrated to 30 °C) and the reaction is allowed to proceed for 60 minutes. At 60 minutes, a 50 µL aliquot is removed and quenched with 200 uL of acetonitrile containing a mixture of internal standards (Tolbutamide (500 ng/mL) and Telmistartan (500 ng/mL)) and is vortexed then centrifuged at 4000 rpm for 10 minutes (Eppendorf 5810R). The supernatant is transferred to a 96 well plate for LC-MS/MS analysis.

An LC-MSIMS method is devised for test compounds and the control, using a AB Sciex API 4000 system coupled to a NEXAR^{™} UHPLC (Shimadzu) system. Analytes are separated on a Phenomenex Kinetex C18 column (50 x 2.1 mm, 5 µm) using a gradient that is appropriate for each compound, at a flow rate of 1 ml/minute, utilizing a mobile phase of 0.1 % formic acid in MILLI-Q^{™} water (A) and 0.1 % formic acid in acetonitrile (B). The MS instrument is operated in positive mode (ESI+)/negative (ESI-). The multiple reaction monitoring (MRM) transition for the representative compounds and the control compounds is used for the LC-MSIMS analysis. MRM transitions for control compound 7-ethoxycoumarinm are Quadropole 1: 191.0, Quadropole 3: 163.0, dwell time: 75 msec, using curtain gas settings of 5 V, ion-spray voltage of 5500 V, temperature of 50 °C and gas settings for nebulizer and auxiliary set to 206843 and 275790 Pa (30 and 40 psi), respectively. The interface heater is kept on. Entrance potential and collision cell exit potential are varied to tune for a specific compound.

Using a suitable LC-MSIMS method, the percentage of test compound remaining at 60 minutes (PCR60) is assessed by comparing the average analyte to internal standard area ratio at 60 minutes to the average analyte to internal standard area ratio at 0 time control, as a percentage, from a 5 to 10 µL sample injection.

Compounds of the invention may be tested in this assay to determine their stability against microsomal metabolism.

## Claims

1. A compound of formula (I): 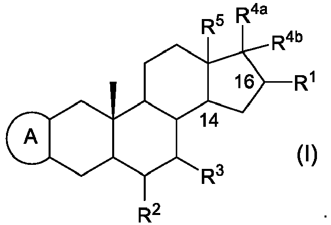 wherein: 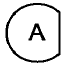
is an optionally substituted fused pyrazolyl;
R¹ is hydrogen or -OR⁶;
R² is -OR⁶ or -N(R⁷)₂;
R³ is -OR⁶ or -N(R⁷)₂;
R^{4a} is hydrogen, alkyl, alkenyl , -R⁸-OR⁶;
R^{4b} is hydrogen, alkyl, -OR⁶ or a direct bond to the carbon at C16;
R⁵ is alkyl or a direct bond to the carbon at C14;
each R⁶ is independently selected from hydrogen or alkyl;
each R⁷ is independently selected from hydrogen or alkyl; and
R⁸ is direct bond or a straight or branched alkylene chain;
or mixtures thereof, or a pharmaceutically acceptable salt or solvate thereof.

2. The compound of Claim 1 wherein:
wherein: 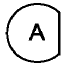
is an optionally substituted fused pyrazolyl;
R¹ is hydrogen or -OR⁶;
R² is -OR⁶;
R³ is -OR⁶;
R^{4a} is hydrogen, alkyl, alkenyl, -R⁸-OR⁶;
R^{4b} is hydrogen, alkyl, -OR⁶ or a direct bond to the carbon at C16;
R⁵ is alkyl or a direct bond to the carbon at C14;
each R⁶ is independently selected from hydrogen or alkyl;
each R⁷ is independently selected from hydrogen or alkyl; and
R⁸ is direct bond or a straight or branched alkylene chain.

3. The compound of Claim 2 wherein: 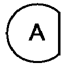
is a fused pyrazolyl optionally substituted by one or more substitutents selected from alkyl, haloalkyl, -C(O)OR⁷, -N(R⁷)₂, -C(O)N(R⁷)₂ or optionally substituted aryl;
R¹ is hydrogen or -OR⁶;
R² is -OR⁶;
R³ is -OR⁶;
R^{4a} is hydrogen, alkyl, alkenyl or -R⁸-OR⁶;
R^{4b} is hydrogen, alkyl, -OR⁶ or a direct bond to the carbon at C16;
R⁵ is alkyl or a direct bond to the carbon at C14;
each R⁶ is independently selected from hydrogen or alkyl;
each R⁷ is independently selected from hydrogen or alkyl; and
R⁸ is direct bond or a straight or branched alkylene chain.

4. The compound of Claim 3 wherein: 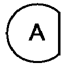
is pyrazolyl optionally substituted by one or more substitutents selected from alkyl, haloalkyl, -C(O)OR⁷, -N(R⁷)₂, -C(O)N(R⁷)₂ or optionally substituted aryl;
R¹ is hydrogen or -OR⁶;
R² is -OR⁶;
R³ is -OR⁶;
R^{4a} is hydrogen, alkyl, alkenyl or -R⁸-OR⁶;
R^{4b} is hydrogen, alkyl, -OR⁶ or a direct bond to the carbon at C16;
R⁵ is alkyl or a direct bond to the carbon at C14;
each R⁶ is independently selected from hydrogen or alkyl;
each R⁷ is independently selected from hydrogen or alkyl; and
R⁸ is direct bond or a straight or branched alkylene chain.

5. The compound of Claim 4 selected from:
(1*S*,3aS,3b*R*,4*R*,5*R*,5a*S*,10a*R*,10b*S*,12a*S*)-1,10a,12a-trimethyl-1,2,3,3a,3b,4,5,5a,6,7,10,10a,10b,11,12,12a-hexadecahydrocyclopenta[5,6]naphtho[1,2-*f*]indazole-1,4,5-triol;
(3a*S*,3b*R*,4*R*,5*R*,5a*S*,10a*R*,10b*S*,12a*R*)-10a,12a-dimethyl-3,3a,3b,4,5,5a,6,7,10,10a,10b,11,12,12a-tetradecahydrocyclopenta[5,6]naphtho[1,2-*f*]indazole-4,5-diol;
(2*R*,3b*R*,4*R*,5*R*,5a*S*,10a*R*,10b*S*)-1,1,10a-trimethyl-1,2,3,3b,4,5,5a,6,7,10,10a,10b,11,12-tetradecahydrocyclopenta[5,6]naphtho[1,2-*f*]indazole-2,4,5-triol;
(1*R*,3a*S*,3b*S*,4*R*,5*R*,5a*S*,10a*R*,10b*S*,12a*R*)-1-((2*S*,5*R*)-5-ethyl-3-hydroxy-6-methylheptan-2-yl)-10a,12a-dimethyl-1,2,3,3a,3b,4,5,5a,6,7,10,10a,10b,11,12,12a-hexadecahydrocyclopenta[5,6]naphtho[1,2-*f*]indazole-4,5-diol;
(1*R*,3a*S*,3b*S*,4*R*,5*R*,5a*S*,10a*R*,12a*R*)-10a,12a-dimethyl-1-((*R*)-6-methylheptan-2-yl)-1,2,3,3a,3b,4,5,5a,6,7,10,10a,10b,11,12,12a-hexadecahydrocyclopenta[5,6]naphtho[1,2-*f*]indazole-4,5-diol; or
(1*R*,3a*S*,3b*S*,4*R*,5*R*,5a*S*,10a*R*,10b*S*,12a*S*)-10a,12a-dimethyl-1-(prop-1-en-2-yl)-1,2,3,3a,3b,4,5,5a,6,7,10,10a,10b,11,12,12a-hexadecahydrocyclopenta[5,6]naphtho[1,2-*f*]indazole-4,5-diol.

6. A composition comprising a compound of Claim 1 or mixtures thereof, or a pharmaceutically acceptable salt or solvate thereof, and a pharmaceutically acceptable excipient.

7. A compound of Claim 1, or mixtures thereof, or a pharmaceutically acceptable salt or solvate thereof, or a composition of Claim 6 for use in a method of treatment of inflammation and/or pain comprising administering an effective amount of said compound or composition to a mammal in need thereof.

## Patentansprüche

1. Verbindung der Formel (I): 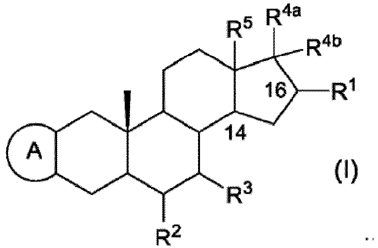 wobei: 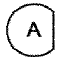
ein optional substituiertes, fusioniertes Pyrazolyl ist;
R¹ Wasserstoff oder -OR⁶ ist;
R² -OR⁶ oder -N(R⁷)₂ ist;
R³ -OR⁶ oder -N(R⁷)₂ ist;
R^{4a} Wasserstoff, Alkyl, Alkenyl, -R⁸-OR⁶ ist;
R^{4b} Wasserstoff, Alkyl, -OR⁶ oder eine direkte Bindung an den Kohlenstoff bei C 16 ist;
R⁵ Alkyl oder eine direkte Bindung an den Kohlenstoff bei C 14 ist;
jedes R⁶ unabhängig voneinander aus Wasserstoff oder Alkyl ausgewählt wird;
jedes R⁷ unabhängig voneinander aus Wasserstoff oder Alkyl ausgewählt wird; und
R⁸ eine direkte Bindung oder eine gerade oder verzweigte Alkylenkette ist;
oder Mischungen davon, oder ein pharmazeutisch akzeptables Salz oder Solvat davon.

2. Verbindung nach Anspruch 1, wobei:
wobei: 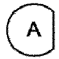
ein optional substituiertes, fusioniertes Pyrazolyl ist;
R¹ Wasserstoff oder -OR⁶ ist;
R² -OR⁶ ist;
R³ -OR⁶ ist;
R^{4a} Wasserstoff, Alkyl, Alkenyl, -R⁸-OR⁶ ist;
R^{4b} Wasserstoff, Alkyl, -OR⁶ oder eine direkte Bindung an den Kohlenstoff bei C 16 ist;
R⁵ Alkyl oder eine direkte Bindung an den Kohlenstoff bei C 14 ist;
jedes R⁶ unabhängig voneinander aus Wasserstoff oder Alkyl ausgewählt wird;
jedes R⁷ unabhängig voneinander aus Wasserstoff oder Alkyl ausgewählt wird; und
R⁸ eine direkte Bindung oder eine gerade oder verzweigte Alkylenkette ist.

3. Verbindung nach Anspruch 2, wobei: 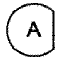
ein kondensiertes Pyrazolyl ist, das gegebenenfalls mit einem oder mehreren Substituenten substituiert ist, und aus Alkyl, Halogenalkyl, -C(O)OR⁷, -N(R⁷)₂, -C(O)N(R⁷)₂ oder gegebenenfalls substituiertem Aryl ausgewählt ist;
R¹ Wasserstoff oder -OR⁶ ist;
R² -OR⁶ ist;
R³ -OR⁶ ist;
R^{4a} Wasserstoff, Alkyl, Alkenyl oder -R⁸-OR⁶ ist;
R^{4b} Wasserstoff, Alkyl, -OR⁶ oder eine direkte Bindung an den Kohlenstoff bei C 16 ist;
R⁵ Alkyl oder eine direkte Bindung an den Kohlenstoff bei C 14 ist;
jedes R⁶ unabhängig voneinander aus Wasserstoff oder Alkyl ausgewählt wird;
jedes R⁷ unabhängig voneinander aus Wasserstoff oder Alkyl ausgewählt wird; und
R⁸ eine direkte Bindung oder eine gerade oder verzweigte Alkylenkette ist.

4. Verbindung nach Anspruch 3, wobei: 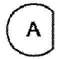
ein Pyrazolyl ist, das gegebenenfalls mit einem oder mehreren Substituenten substituiert ist, und aus Alkyl, Halogenalkyl, -C(O)OR⁷, -N(R⁷)₂, -C(O)N(R⁷)₂ oder gegebenenfalls substituiertem Aryl ausgewählt ist;
R¹ Wasserstoff oder -OR⁶ ist;
R² -OR⁶ ist;
R³ -OR⁶ ist;
R^{4a} Wasserstoff, Alkyl, Alkenyl oder -R⁸-OR⁶ ist;
R^{4b} Wasserstoff, Alkyl, -OR⁶ oder eine direkte Bindung an den Kohlenstoff bei C16 ist;
R⁵ Alkyl oder eine direkte Bindung an den Kohlenstoff bei C14 ist;
jedes R⁶ unabhängig voneinander aus Wasserstoff oder Alkyl ausgewählt wird;
jedes R⁷ unabhängig voneinander aus Wasserstoff oder Alkyl ausgewählt wird; und
R⁸ eine direkte Bindung oder eine gerade oder verzweigte Alkylenkette ist.

5. Verbindung nach Anspruch 4, ausgewählt aus:
(1*S*,3a*S*,3b*R*,4*R*,5*R*,5a*S*,10a*R*,10b*S*,12a*S*)-1,10a, 12a-Trimethyl-1,2,3,3a,3b,4,5,5a,6,7,10,10a, 10b, 11,12,12a-Hexadekahydrocyclopenta[5,6]Naphtho[1,2-*f*]Indazol-1,4,5-triol;
(3aS,3b*R*,4*R*,5*R*,5a*S*, 10aR, 10b*S*, 12a*R*)-10a, 12a-Dimethyl-3,3a,3b,4,5,5a,6,7,10,10a,10b,11,12,12a-Tetradekahydrocyclopenta[5,6]Naphtho[1,2-*f*] Indazol-4,5-diol;
(2*R*,3b*R*,4*R*,5*R*,5a*S*,10a*R*,10b*S*)-1,1,10a-Trimethyl-1,2,3,3b,4,5,5a,6,7,10,10a,10b,11,12-Tetradekahydrocyclopenta[5,6]Naphtho[1,2-*f*]Indazol-2,4,5-triol;
(1*R*,3a*S*,3b*S*,4*R*,5*R*,5a*S*,10a*R*,10b*S*,12a*R*)-1-((2*S*,5*R*)-5-Ethyl-3-Hydroxy-6-Methylheptan-2-yl)-10a, 12a-Dimethyl- 1,2,3,3a,3b,4,5,5a,6,7, 10, 10a, 10b, 11, 12,12a-Hexadekahydrocyclopenta[5,6]Naphtho[1,2-*f*]Indazol-4,5-diol;
(1*R*,3a*S*,3b*S*,4*R*,5*R*,5a*S*,10a*R*,12a*R*)-10a,12a-Dimethyl-1-((*R*)-6-Methylheptan-2-yl)-1,2,3,3a,3b,4,5,5a,6,7, 10, 10a, 10b, 11, 12,12a-Hexadekahydrocyclopenta[5,6]Naphtho[1,2-*f*]Indazol-4,5-diol;
Oder (1*R*,3a*S*,3b*S*,4*R*,5*R*,5a*S*,10a*R*,10b*S*,12a*S*)-10a,12a-Dimethyl-1-(prop-1-en-2-yl)-1,2,3,3a,3b,4,5,5a,6,7,10,10a, 10b, 11,12,12a-Hexadekahydrocyclopenta[5,6]naphtho[1,2-*f*]indazole-4,5-diol.

6. Eine Zusammensetzung, umfassend eine Verbindung nach Anspruch 1 oder Mischungen davon oder ein pharmazeutisch akzeptables Salz oder Solvat davon und einen pharmazeutisch akzeptabler Hilfsstoff.

7. Ein Verbindung nach Anspruch 1 von Mischungen davon oder eines pharmazeutisch akzeptablen Salzes oder Solvats davon oder einer Zusammensetzung nach Anspruch 6 zur Verwendung in einem Verfahren zur Behandlung von Entzündungen und/oder Schmerzen, umfassend die Verabreichung einer wirksamen Menge der Verbindung oder Zusammensetzung an ein Säugetier, das diese benötigt.

## Revendications

1. Un composé de formule (I) : 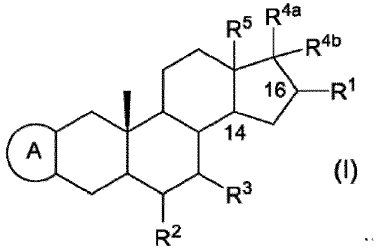 Dans lequel : 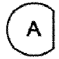
est un pyrazolyle fusionné facultativement substitué ;
R¹ est un hydrogène ou -OR⁶ ;
R² est -OR⁶ ou -N(R⁷)₂ ;
R³ est -OR⁶ ou -N(R⁷)₂ ;
R^{4a} est un hydrogène, alkyle, alkényle, -R⁸-OR⁶ ;
R^{4b} est un hydrogène, alkyle, -OR⁶ ou une liaison directe au carbone en C16 ;
R⁵ est un alkyle ou une liaison directe au carbone en C14 ;
Chaque R⁶ est choisi indépendamment parmi un hydrogène ou un alkyle ;
Chaque R⁷ est sélectionné indépendamment parmi un hydrogène ou un alkyle ; et
R⁸ est une chaîne alkylène en liaison directe ou droite ou ramifiée ;
ou des mélanges, ou un sel ou un solvate pharmaceutiquement acceptable de celui-ci.

2. Le composé selon la revendication 1 :
Dans lequel : 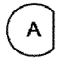
est un pyrazolyle fusionné facultativement substitué ;
R¹ est un hydrogène ou -OR⁶ ;
R² est -OR⁶ ;
R³ est -OR⁶ ;
R^{4a} est un hydrogène, alkyle, alkényle, -R⁸-OR⁶ ;
R^{4b} est un hydrogène, alkyle, -OR⁶ ou une liaison directe au carbone en C16 ;
R⁵ est un alkyle ou une liaison directe au carbone en C14 ;
Chaque R⁶ est choisi indépendamment parmi un hydrogène ou un alkyle ;
Chaque R⁷ est sélectionné indépendamment parmi un hydrogène ou un alkyle ; et
R⁸ est une chaîne alkylène en liaison directe ou droite ou ramifiée.

3. Le composé selon la revendication 2 dans lequel : 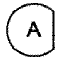
est un pyrazolyle fusionné facultativement substitué par un ou plusieurs substituts sélectionnés parmi un alkyle, haloalkyle, -C(O)OR⁷, -N(R⁷)₂, -C(O)N(R⁷)₂ ou un aryle facultativement substitué ;
R¹ est un hydrogène ou -OR⁶ ;
R² est -OR⁶ ;
R³ est -OR⁶ ;
R^{4a} est un hydrogène, alkyle, alkényle ou -R⁸-OR⁶ ;
R^{4b} est un hydrogène, alkyle, -OR⁶ ou une liaison directe au carbone en C16 ;
R⁵ est un alkyle ou une liaison directe au carbone en C14 ;
Chaque R⁶ est choisi indépendamment parmi un hydrogène ou un alkyle ;
Chaque R⁷ est sélectionné indépendamment parmi un hydrogène ou un alkyle ; et
R⁸ est une chaîne alkylène en liaison directe ou droite ou ramifiée.

4. Le composé selon la revendication 3 dans lequel : 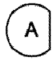
est un pyrazolyle facultativement substitué par un ou plusieurs substituts sélectionnés parmi un alkyle, haloalkyle, -C(O)OR⁷, -N(R⁷)₂, -C(O)N(R⁷)₂ ou un aryle facultativement substitué ;
R¹ est un hydrogène ou -OR⁶ ;
R² est -OR⁶ ;
R³ est -OR⁶ ;
R^{4a} est un hydrogène, alkyle, alkényle ou -R⁸-OR⁶ ;
R^{4b} est un hydrogène, alkyle, -OR⁶ ou une liaison directe au carbone en C16 ;
R⁵ est un alkyle ou une liaison directe au carbone en C14 ;
Chaque R⁶ est choisi indépendamment parmi un hydrogène ou un alkyle ;
Chaque R⁷ est sélectionné indépendamment parmi un hydrogène ou un alkyle ; et
R⁸ est une chaîne alkylène en liaison directe ou droite ou ramifiée.

5. Le composé de la revendication 4 choisi parmi :
(1*S*,3a*S*,3b*R*,4*R*,5*R* 5a*S*10a*R*,10b*S*,12a*S*)-1,10a,12a-triméthyl-1,2,3,3a,3b,4,5,5a,6,7,10,10a, 10b, 11,12,12a-hexadécahydrocyclopenta[5,6]naphtho[1,2-*f*]indazole-1,4,5-triol ;
(3aS,3b*R*,4*R*,5*R*,5a*S*, 10a*R*, 10b*S*, 12a*R*)-10a,12a-diméthyl-3,3a,3b,4,5,5a,6,7,10,10a,10b,11,12,12a-tétradécahydrocyclopenta[5,6]naphtho[1,2-*f*] indazole-4,5-diol ;
(2*R*,3b*R*,4*R*,5*R*,5a*S*,10a*R*,10b*S*)-1,1,10a-triméthyl-1,2,3,3b,4,5,5a,6,7,10,10a,10b,11,12-tétradécahydrocyclopenta[5,6]naphtho[1,2-*f*]indazole-2,4,5-triol ;
(1*R*,3a*S*,3b*S*,4*R*,5*R*,5a*S*,10a*R*,10b*S*,12a*R*)-1-((2*S*,5*R*)-5-éthyl-3-hydroxy-6-méthylheptan-2-yl)-10a,12a-diméthyl-1,2,3,3a,3b,4,5,5a,6,7,10,10a,10b,11,12,12a-hexadécahydrocyclopenta[5,6]naphtho[1,2-*f*]indazole-4,5-diol ;
(1*R*,3a*S*,3b*S*,4*R*,5*R*,5a*S*,10a*R*,12a*R*)-10a,12a-diméthyl-1-((*R* )-6-méthylheptan-2-yl)-1,2,3,3a,3b,4,5,5a,6,7,10,10a, 10b, 11,12,12a-hexadécahydrocyclopenta[5,6]naphtho[1,2-*f*]indazole-4,5-diol ;
ou (1*R*,3a*S*,3b*S*,4*R*,5*R*,5a*S*,10a*R*,10b*S*,12a*S*)-10a,12a-diméthyl-1-(prop-1-en-2-yl)-1,2,3,3a,3b,4,5,5a,6,7,10,10a, 10b, 11,12,12a-hexadécahydrocyclopenta[5,6]naphtho[1,2-*f*]indazole-4,5-diol.

6. Une composition comprenant un composé selon la revendication 1 ou des mélanges, ou un sel ou un solvate pharmaceutiquement acceptable de celui-ci, et un excipient pharmaceutiquement acceptable.

7. Un composé selon la revendication 1, ou des mélanges, ou un sel ou un solvate pharmaceutiquement acceptable de celui-ci, ou une composition selon la revendication 6 pour une utilisation dans un procédé de traitement de l'inflammation et/ou de la douleur comprenant l'administration d'une quantité efficace dudit composé ou de la composition à un mammifère ayant besoin de ce traitement.
